# EUROPÄISCHE PATENTSCHRIFT

(11) **EP 3 461 815 B1**
(45) Veröffentlichungstag und Bekanntmachung des Hinweises auf die Patenterteilung: **03.03.2021**
(21) Anmeldenummer: 18197591.3
(22) Anmeldetag: 28.09.2018
(51) Int. Cl.: C07D 215/10, C07D 311/88, C07D 405/10, C07D 471/04, C07F 7/08, C07F 7/10, C07C 13/00

(54) **NEUE POLY-SULFONIERTE FLUORESZENZ-FARBSTOFFE**
NOVEL POLY SULFONATED FLUORESCENCE DYES
NOUVELLES MATIÈRES COLORANTES FLUORESCENTES POLYSULFONÉES

(30) Priorität: 29.09.2017 EP 17194195
(43) Veröffentlichungstag der Anmeldung: 03.04.2019
(73) Patentinhaber: ATTO-TEC GmbH, 57076 Siegen (DE)
(72) Erfinder: Kemnitzer, Norbert, 51643 Gummersbach (DE); Zilles, Alexander, 57223 Kreuztal (DE); Drexhage, Karl-Heinz, 57076 Siegen (DE); Hamers-Schneider, Monika, 57078 Siegen (DE); Arden-Jacob, Jutta, 90513 Zirndorf (DE)
(74) Vertreter: Dey, Michael

(56) Entgegenhaltungen:
- EP-A1- 2 192 198
- EP-A1- 3 219 712
- WO-A1-2013/152687
- WO-A1-2015/175870
- WO-A1-2016/157937
- WO-A2-2005/003086
- US-A1- 2004 260 093

## Beschreibung

Die Erfindung betrifft Verbindungen der allgemeinen Formeln (I) - (IV), die sich durch eine oder mehrere Sulfonsäuregruppen enthaltende Substituenten B auszeichnen und ihre Verwendung als Markierungsgruppen zum Nachweis von Analyten.

In der chemischen, medizinischen und biologischen Analytik werden organische Farbstoffe in vielfältiger Weise als Markierungs- bzw. Nachweisgruppen verwendet. Insbesondere Fluoreszenzfarbstoffe haben in neuerer Zeit große praktische Bedeutung gewonnen und andere frühere Verfahren, die etwa radioaktive Isotope verwenden, fast vollständig verdrängt. Ein besonderer Vorteil der Fluoreszenzmethoden ist die hohe Nachweisempfindlichkeit: Anhand ihrer Fluoreszenz lassen sich sogar einzelne Moleküle detektieren. Gegenüber radioaktiven Methoden sind die Stabilität der Label sowie die einfacheren Sicherheitsbestimmungen wesentliche Vorteile bei der praktischen Anwendung.

Die meisten dieser analytischen Markierungs- und Nachweisverfahren werden in wässrigem Milieu durchgeführt. Durch die geringe Wasserlöslichkeit vieler als Marker verwendeter organischer Farbstoffe ergeben sich dabei eine Reihe von Problemen: Störende Phänomene sind die unspezifische Bindung des Fluorophors an Gefäßwände oder Substrate sowie die starke Tendenz zur Bildung nicht fluoreszierender Farbstoffaggregate.

Unspezifische Bindungen führen in der Praxis zu einer erhöhten Hintergrundfluoreszenz und damit zu einer stark verringerten Detektionssicherheit. Neben den kovalent an den nachzuweisenden Analyten gebundenen Farbstoffmolekülen (gewünschte Fluoreszenzmarkierung) werden auch solche Fluorophore angeregt, die lediglich durch schwächere Wechselwirkungen an Oberflächen adsorbiert oder mit anderen als den gewünschten Analyten assoziert - also nicht kovalent gebunden - sind. Ist die Affinität zwischen den beteiligten Spezies groß genug, führen die obligatorischen Waschschritte nicht zu einer vollständigen Entfernung der auf diese Weise unspezifisch gebundenen Farbstoffmoleküle. Diese Fluorophore erzeugen dann ein störendes zusätzliches und den eigentlichen Nachweis verfälschendes Fluoreszenzsignal.

Das Auftreten von Farbstoffaggregaten zeigt sich im Absorptionsspektrum durch eine starke Veränderung der langwelligen Absorptionsbande (so genanntes Aggregatspektrum). Dieses Phänomen lässt sich sehr oft in hinreichend konzentrierten wässrigen Farbstofflösungen beobachten, während es in organischen Lösungsmitteln normalerweise nicht auftritt.
Die Ursache der Aggregatbildung sind vor allem hydrophobe Wechselwirkungen zwischen den Farbstoffchromophoren und den umgebenden Wassermolekülen: Es ist energetisch günstiger, wenn sich zwei (oder mehr) Farbstoffmoleküle als Aggregat zusammenlagern. Die damit verbundene Veränderung der elektronischen Energieniveaus kommt im veränderten Absorptionsspektrum zum Ausdruck.
Im Gegensatz zu den monomer vorliegenden Fluoreszenzfarbstoffen sind Farbstoffaggregate aber fast immer nicht fluoreszierend. Ein Einsatz als Fluoreszenzmarker ist unter diesen Umständen nicht mehr effizient möglich, da die Empfindlichkeit und damit die Nachweisgrenze stark absinkt oder die Bildqualität erheblich leidet.

Das beschriebene Phänomen der Aggregation von Farbstoffen in wässriger Lösung tritt auch noch an einer anderen Stelle störend in Erscheinung: Um die Detektionssicherheit zu erhöhen ist es wünschenswert, möglichst viele Fluoreszenzfarbstoffmoleküle zur Markierung an ein einzelnes Biomolekül zu koppeln. Es wird erwartet, dass sich die Fluoreszenzintensität der Einzelmoleküle dabei addiert und so zu einem Signalanstieg führt.
Die durchschnittliche Anzahl an Farbstoffmolekülen pro Biomolekül wird durch das "degree of labeling" (DOL) ausgedrückt. Das Verfahren der DOL-Bestimmung ist dem Fachmann bekannt: Hierzu werden die Absorptionen des Farbstoff-Biomolekül-Konjugats beim Absorptionsmaximum des Farbstoffs, wo das Biomolekül nicht absorbiert, und im UV bei 260 nm (für DNA) bzw. 280 nm (für Proteine) ermittelt. Da auch der Farbstoff im UV absorbiert, muss die dort gemessene (Gesamt-)Absorption noch mit der Absorption des Farbstoffs bei dieser Wellenlänge korrigiert werden, um die tatsächliche Biomolekülabsorption zu erhalten. Dazu werden bei kommerziellen Farbstoffen so genannte CF-Werte (correction factor, engl. Korrekturfaktor) angegeben, die das Verhältnis der Absorptionen des Farbstoffs beim langwelligen Maximum und bei 260 nm bzw. 280 nm beschreiben.
Aus den so ermittelten (Einzel-)Absorptionen von Farbstoff und Biomolekül im Konjugat wird unter Berücksichtigung der beiden Extinktionskoeffizienten der Quotient gebildet, der direkt das molekulare Konzentrationsverhältnis und damit die mittlere Anzahl an Farbstoffmolekülen pro Biomolekül ("degree of labeling", DOL) angibt.
Eine Erhöhung des DOL kann dazu führen, dass sich die kovalent an das Biomolekül gebundenen Farbstoffe in enger räumlicher Nachbarschaft befinden und damit auch hier Aggregationsphänomene beobachtet werden können. Man erhält in solchen Fällen nicht etwa wie erwartet einen immer weiteren Anstieg des Fluoreszenzsignals. Vielmehr wird im Allgemeinen schon nach einem oder wenigen Farbstoffmolekülen pro Biomolekül eine Art Sättigung erreicht, bei der die detektierte Fluoreszenz nicht weiter zunimmt. Bei noch höherem DOL ist dann sogar wieder eine Abnahme der Fluoreszenzintensität zu beobachten.

WO 2016/157937 A1 beschreibt pH-sensitive Fluoreszenzsonden umfassend einen Piperazinrest.

WO 2015/175870 A1 beschreibt Carbopyrononverbindungen.

WO 2005/003086 beschreibt Sulfonamidderivate polyzyklischer Farbstoffe für analytische Anwendungen.

EP 3 219 712 A1 beschreibt Kumarinfarbstoffe und Konjugate davon.

EP 2 192 198 betrifft eine Kombination von Fluoreszenzfarbstoffen zum Nachweis von Nukleinsäuren.

WO 2013/152687 A1 beschreibt ein Verfahren zur Herstellung von Nah-Infrafrot-fluoreszierenden Farbstoffen.

US 2004/0260093 A1 betrifft Markerfarbstoffe auf Basis von Polymethinen.

Eine Aufgabe der vorliegenden Erfindung bestand somit darin, Fluoreszenzfarbstoffe bereitzustellen, die als Markierungsgruppe in Nachweisverfahren von Analyten eingesetzt werden können und die diese Nachteile des Standes der Technik zumindest teilweise vermeiden. Insbesondere war es eine Aufgabe der vorliegenden Erfindung Fluoreszenzfarbstoffe bereitzustellen, die eine möglichst geringe unspezifische Bindung an Gefäßwände oder vom Analyten unterschiedliche Substrate zeigen. Eine weitere Aufgabe bestand darin, Fluoreszenzfarbstoffe bereitzustellen, die eine möglichst geringe oder keine Aggregatbildung in wässriger Umgebung zeigen.

Es ist bekannt, dass hydrophobe organische Verbindungen durch die Substitution mit hydrophilen oder ionischen Gruppen wie z.B. Sulfonsäuregruppen besser in Wasser löslich sind. Dies führt im Allgemeinen zu einer Verringerung der hydrophoben Wechselwirkung der Moleküle untereinander, was bei organischen Farbstoffen - wie oben erläutert - die Aggregationsneigung minimiert, so dass wiederum ein höheres Fluoreszenzsignal erwartet werden kann.

Die Wirksamkeit einer solchen Substitution hinsichtlich der gesuchten Fluoreszenzeigenschaften ist aber von der Art (direkt am Chromophor oder über einen Linker) und dem Ort (Position im Molekül) der Anknüpfung der Sulfonsäuren sowie von deren Gesamtanzahl abhängig.

Dies wurde beispielhaft an Cyanin-Farbstoffen gezeigt von J. Pauli, M. Grabolle, R. Brehm, M. Spieles, F. Hamann, M. Wenzel, I. Hilger, U. Resch-Genger, Suitable Labels for Molecular Imaging - Influence of Dye Structure and Hydrophilicity on the Spectroscopic Properties of IgG Conjugates, Bioconjugate Chem., 2011, 22, 1298 - 1308 und J. Pauli, K. Licha, J. Berkemeyer, M. Grabolle, M. Spieles, N. Wegner, P. Welker, U. Resch-Genger, New Fluoescent Labels with Tunable Hydrophilicity for the Rational Design of Bright Optical Probes for Molecular Imaging, Bioconjugate Chem., 2013, 24, 1174 - 1185 und F. M Hamann, R. Brehm, J. Pauli, M. Grabolle, W. Frank, W. A. Kaiser, D. Fischer, U. Resch-Genger, I. Hilger, Controlled modulation of serum protein binding and biodistribution of asymmetric cyanine dyes by variation of the number of sulfonate groups, Molecular imaging, 2011, 10, 258-269.

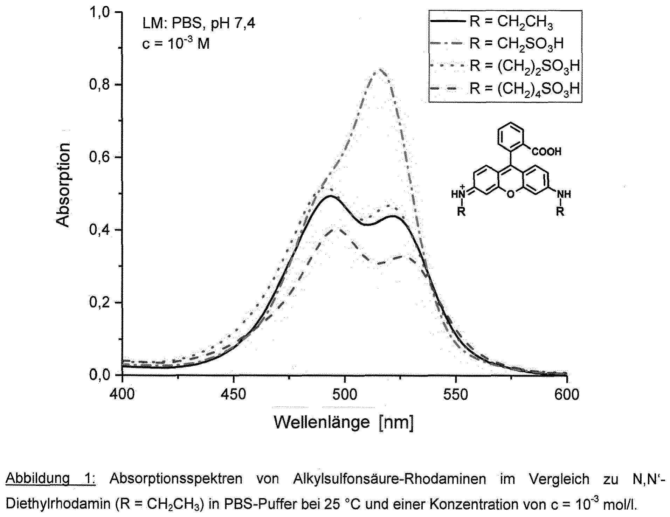

Für Rhodaminfarbstoffe konnten von uns selber ähnliche Beobachtungen gemacht werden:
Befinden sich Sulfonsäuregruppen an Alkylketten der terminalen Stickstoffe der Farbstoffe, so lässt mit zunehmendem Abstand dieser hydrophilen Gruppen vom Chromophor deren aggregationshemmende Wirkung drastisch nach (Abb. 1). So zeigen die Absorptionsspektren gleich konzentrierter wässriger Lösungen (PBS-Puffer: Phosphat-gepufferte Salzlösung) der Farbstoffe mit Ethylgruppen bzw. Sulfoethylgruppen etwa die gleiche Aggregation. Unter identischen Bedingungen erkennt man nur im Fall der Sulfomethylgruppen eine verminderte Aggregation im Absorptionsspektrum. Rhodaminfarbstoffe mit solchen einfachen Sulfoalkylgruppen werden allerdings wegen ihrer besseren Wasserlöslichkeit patentiert, siehe z.B. C. Barnes, N.N. Romanov, Illumina Cambridge Limited, Dye compounds and the use of their labelled conjugates, United States Patent US8178360B2.

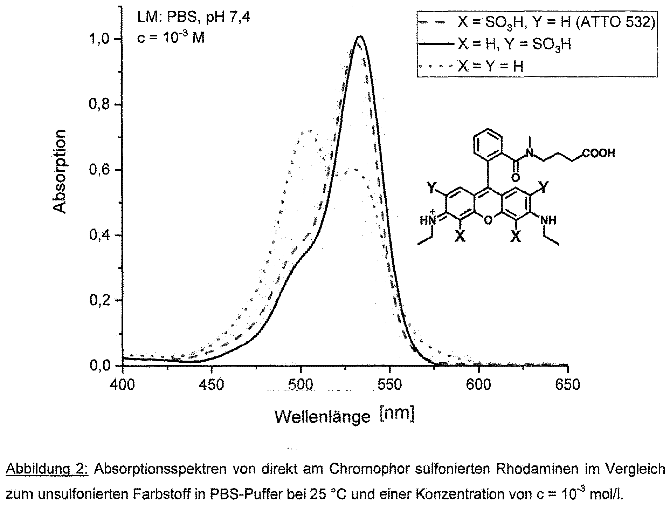

Andererseits bewirkt die direkte Substitution mit Sulfonsäuregruppen am Chromophor neben der Erhöhung der Wasserlöslichkeit auch eine massive Reduktion der Aggregation (Abb. 2). Bei den sulfonierten Farbstoffen erscheint selbst in konzentrierter Lösung noch die schmale Monomerenbande, während der unsubstituierte Farbstoff eine deutliche Dimerenbande zeigt.

In keinem der beiden diskutierten Fälle (Sulfoalkyl-Substitution der terminalen Stickstoffe oder direkte Sulfonsäuregruppen-Substitution am Chromophor) bleibt bei der Kopplung an das von uns wegen seiner weiten Verbreitung bei biochemischen Verfahren, wie etwa Proteinreinigung oder Immunoassays, gewählte Modellprotein Streptavidin - trotz erhöhter Wasserlöslichkeit und evtl. verringerter Aggregation - die hohe Fluoreszenzquantenausbeute der freien Farbstoffe erhalten, siehe Tabelle 1, Beispiel ATTO 532. Bei solchen Farbstoffen wird die Quantenausbeute im Konjugat gegenüber dem freien, ungekoppelten Farbstoff deutlich reduziert.

Ein weiterer Nachteil der bisher beschriebenen Sulfonierung von Rhodaminen ist die begrenzte Verfügbarkeit von direkten Anknüpfungsstellen am Molekül. Auf diese Weise lässt sich also nur eine geringe Anzahl von Sulfonsäuregruppen - gewöhnlich maximal zwei - in den Farbstoff einführen. Siehe hierzu z.B. F. Mao, W.-Y. Leung, Richard P. Haugland, Molecular Probes Inc., Sulfonated Xanthene Derivatives, United States Patent US6130101A oder R. L. Cournoyer, J. W. Foley, Polaroid Corporation, Photographic Products and Processes with a pH sensitive Xanthene Light Screeing Dye, United States Patent US4345017A.

Im Falle der Oxazine lassen sich Sulfonsäuregruppen sogar nur über Alkylketten an der Molekülperipherie einführen: A. Toutchkine, Sigma-Aldrich Co., Oxazine dyes with improved aqueous solubility, United States Patent Application US2012/0010404A1.

Eine weitere Aufgabe der vorliegenden Erfindung bestand also darin, noch mehr Sulfonsäuregruppen in Farbstoffe einzuführen als dies nach dem Stand der Technik möglich ist.

Die bevorzugten Farbstoffe sind Vertreter der nachfolgend zusammengestellten Klassen - aber nicht auf diese beschränkt - mit den allgemeinen Strukturen:

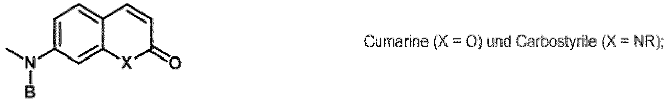

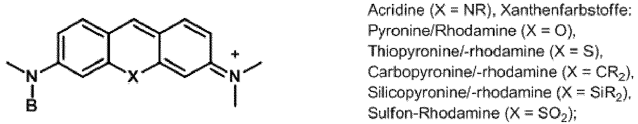

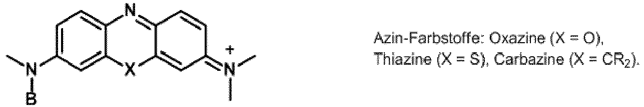

Über den Rest B werden Substituenten eingeführt, die mindestens eine Sulfonsäuregruppe aufweisen.

Diese Aufgabe wurde gelöst durch Verbindungen der allgemeinen Formeln (I) - (IV) und deren Verwendung als Markierungsgruppen in einem Verfahren zum Nachweis eines Analyten 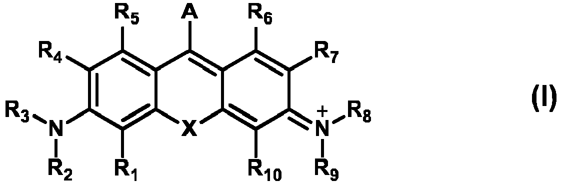 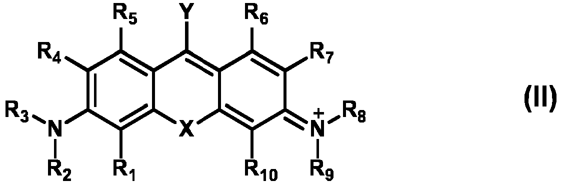 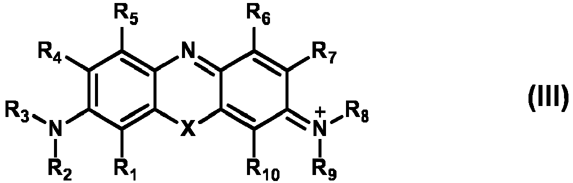 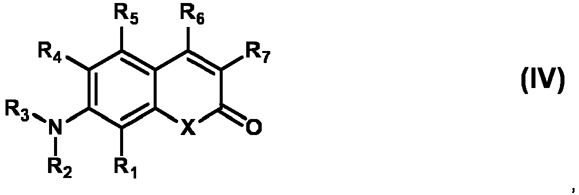
worin R₁, R₄, R₅, R₆, R₇ und R₁₀ unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R₂, R₃, R₈ und R₉ unabhängig voneinander Wasserstoff oder eine Kohlenwasserstoffgruppe mit 1-20 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
wobei jeder der Reste in den Verbindungen der allgemeinen Formeln (I) - (IV) mit einem oder mehreren benachbarten Resten ein Ringsystem bilden kann,
X in der allgemeinen Formel (I) eine Gruppe ausgewählt aus O, S, SO₂, CR₁₁R₁₂ oder SiR₁₃R₁₄ bedeutet,
X in der allgemeinen Formel (II) eine Gruppe ausgewählt aus O, S, CR₁₁R₁₂, SiR₁₃R₁₄ oder NR₁₅ bedeutet,
X in der allgemeinen Formel (III) eine Gruppe ausgewählt aus O, S oder CR₁₁R₁₂ bedeutet,
X in der allgemeinen Formel (IV) O oder NR₁₅ bedeutet
und wobei R₁₁, R₁₂, R₁₃, R₁₄ und R₁₅ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
oder R₁₁ und R₁₂ oder R₁₃ und R₁₄ jeweils zusammen mit dem Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
A in der allgemeinen Formel (I) einen Aryl- oder Heteroaryl-Rest bedeutet, der gegebenenfalls einen oder mehrere Substituenten vorzugsweise ausgewählt aus der Gruppe bestehend aus Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), CONH(Alkyl), CON(Alkyl)₂, CONH(Aryl), CON(Aryl)₂, PO₃H₂, SO₃H und einer Kohlenwasserstoffgruppe umfasst, wobei die Kohlenwasserstoffgruppe ihrerseits gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
Y in der allgemeinen Formel (II) Wasserstoff, CN, COOH, COO(Alkyl), COO(Aryl), NHR₁₆, NR₁₇R₁₈, PO₃H₂, SO₃H oder eine aliphatische Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
und R₁₆, R₁₇, und R₁₈ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
oder R₁₇ und R₁₈ zusammen mit dem Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
mit der Maßgabe, dass mindestens einer der Reste R₂, R₃, R₈ oder R₉ die Struktur B aufweist, wobei es sich bei B um eine der folgenden isomeren Strukturen (H), (I) oder (J) handelt 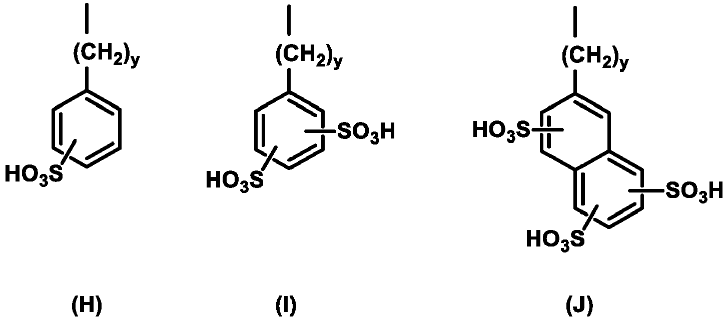
mit y = 0, 1, 2, 3, 4, 5, 6 und wobei nicht näher bezeichnete Positionen in den gezeigten Aryl-Systemen wie R₁ definiert sind, oder
wobei es sich bei B um eine der Strukturen (K) oder (L) mit x = 1, 2, 3, 4, 5, 6 handelt 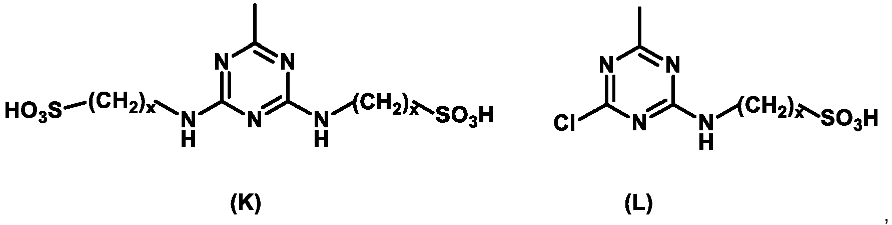 oder wobei es sich bei B um -CH₂SO₃H oder um -CH₂CH₂SO₃H handelt
   wobei in Verbindungen der allgemeinen Formel (I) für X = O der Rest B nicht -CH₂SO₃H oder - CH₂CH₂SO₃H darstellt, wenn nicht gleichzeitig wenigstens einer oder mehrere der Reste R₁, R₄, R₅, R₆, R₇, R₁₀ und/oder A wenigstens eine weitere SO₃H-Gruppe aufweist.

Die für die Struktur B angegebenen Definitionen bedeuten, dass die Struktur B nicht ein Ringsystem mit einem oder mehreren benachbarten Resten bildet.

Der Begriff "Kohlenwasserstoffgruppe", wie er im Rahmen der vorliegenden Anmeldung verwendet wird, umfasst einen gesättigten oder ungesättigten Kohlenwasserstoffrest mit 1-20 Kohlenstoffatomen, bevorzugt 1-12 Kohlenstoffatomen, stärker bevorzugt 1-6 Kohlenstoffatomen, welcher linear, verzweigt oder cyclisch ausgebildet sein kann und eine Bindungsvalenz an einem beliebigen der 1-20 Kohlenstoffatome aufweist. Beispiele für Kohlenwasserstoffgruppen im Sinne der vorliegenden Erfindung umfassen Alkyl, Cycloalkyl, Alkenyl oder Alkinyl und Aryl.
Sofern die Kohlenwasserstoffgruppe ein oder mehrere Heteroatome ausgewählt aus der Gruppe bestehend aus N, O und S umfasst, so kann die Kohlenwasserstoffgruppe zudem als Heteroalkyl, Heterocycloalkyl oder Heteroaryl ausbildet sein.

Der Begriff "aliphatische Kohlenwasserstoffgruppe", wie er im Rahmen der vorliegenden Anmeldung verwendet wird, umfasst die gleiche Bedeutung wie Kohlenwasserstoffgruppe mit Ausnahme von Aryl oder Heteroaryl.

Der Begriff "Alkyl", wie er im Rahmen der vorliegenden Anmeldung verwendet wird, bezeichnet einen gesättigten, linearen oder verzweigten Kohlenwasserstoffrest mit 1-20 Kohlenstoffatomen, welcher eine Bindungsvalenz an einem beliebigen der 1-20 Kohlenstoffatome aufweist. Bevorzugt stellt Alkyl einen Kohlenwasserstoffrest mit 1-12 Kohlenstoffatomen, stärker bevorzugt mit 1-6 Kohlenstoffatomen, dar. Besonders bevorzugte Alkyle umfassen Methyl, Ethyl, n-Propyl, iso-Propyl, n-Butyl, iso-Butyl, sec-Butyl und tert-Butyl.
Sofern der Kohlenwasserstoffrest ein oder mehrere Heteroatome ausgewählt aus der Gruppe bestehend aus N, O und S umfasst, kann der Rest als Heteroalkyl ausgebildet sein und damit u.a. Alkoxygruppen wie Methoxy, Ethoxy etc. umfassen.

Der Begriff "Cycloalkyl", wie er im Rahmen der vorliegenden Anmeldung verwendet wird, bezeichnet einen gesättigten oder ungesättigten, cyclischen Kohlenwasserstoffrest mit 3-20 Kohlenstoffatomen, welcher eine Bindungsvalenz an einem beliebigen der 3-20 Kohlenstoffatome aufweist. Bevorzugt stellt Cycloalkyl einen cyclischen Kohlenwasserstoffrest mit 3-12 Kohlenstoffatomen, stärker bevorzugt mit 3-8 Kohlenstoffatomen, dar. Besonders bevorzugte Cycloalkyle umfassen Cyclopropyl, Cyclobutyl, Cyclopentyl, Cyclohexyl, Cycloheptyl und Cyclooctyl. Außerdem können die cyclischen Kohlenwasserstoffreste verbrückt sein und bicyclische oder polycyclische Verbindungen ausbilden, wie Norbornan, Norbornen und Bicyclo[3.2.2]octyl.
Sofern der cyclische Kohlenwasserstoffrest ein oder mehrere Heteroatome ausgewählt aus der Gruppe bestehend aus N, O und S umfasst, kann der Rest auch als Heterocycloalkyl ausgebildet sein und damit aliphatische Heterocyclen wie Tetrahydropyrrol, Piperidin, Dioxan oder Tetrahydrofuran umfassen.

Der Begriff "Alkenyl", wie er im Rahmen der vorliegenden Anmeldung verwendet wird, bezeichnet einen ungesättigten, linearen oder verzweigten Kohlenwasserstoffrest mit 2-20 Kohlenstoffatomen, welcher eine Bindungsvalenz an einem beliebigen der 2-20 Kohlenstoffatome und mindestens eine Doppelbindung aufweist. Bevorzugt stellt Alkenyl einen Kohlenwasserstoffrest mit 2-12 Kohlenstoffatomen, stärker bevorzugt mit 2-6 Kohlenstoffatomen, dar. Besonders bevorzugte Alkenyle umfassen Ethenyl, Propenyl und Butenyl.

Der Begriff "Alkinyl", wie er im Rahmen der vorliegenden Anmeldung verwendet wird, bezeichnet einen ungesättigten, linearen oder verzweigten Kohlenwasserstoffrest mit 2-20 Kohlenstoffatomen, welcher eine Bindungsvalenz an einem beliebigen der 2-20 Kohlenstoffatome und mindestens eine Dreifachbindung aufweist. Bevorzugt stellt Alkinyl einen Kohlenwasserstoffrest mit 2-12 Kohlenstoffatomen, stärker bevorzugt mit 2-6 Kohlenstoffatomen, dar. Besonders bevorzugte Alkinyle umfassen Ethinyl, Propinyl und Butinyl.

Der Begriff "Aryl", wie er im Rahmen der vorliegenden Anmeldung verwendet wird, bezeichnet ein aromatisches Ringsystem mit 3-20 Ringatomen, stärker bevorzugt mit 6-14 Ringatomen, welches als Ringatome ausschließlich Kohlenstoffatome enthält und eine Bindungsvalenz an einem beliebigen Kohlenstoffatom der 3-20 ringbildenden Atome aufweist. Bevorzugte Aryle umfassen Phenyl, Naphthyl, Anthracenyl und Phenanthrenyl.

Der Begriff "Heteroaryl", wie er im Rahmen der vorliegenden Anmeldung verwendet wird, bezeichnet ein aromatisches Ringsystem mit 3-20 Ringatomen, stärker bevorzugt mit 5-14 Ringatomen, welches als Ringatome neben Kohlenstoffatomen mindestens ein Heteroatom ausgewählt aus der Gruppe bestehend aus N, O und S enthält und eine Bindungsvalenz an einem beliebigen Kohlenstoffatom oder Stickstoffatom der 3-20 ringbildenden Atome aufweist. Bevorzugte Heteroaryle umfassen Furanyl, Imidazolyl, Isothiazolyl, Isoxazolyl, Oxadiazolyl, Oxazolyl, Pyrazolyl, Pyrrolyl, Tetrazolyl, Thiadiazolyl, Thiazolyl, Thienyl, Triazolyl, Pyrazinyl, Pyridazinyl, Pyridyl, Pyrimidinyl und Triazinyl.

Der Begriff "Halogen", wie er im Rahmen der vorliegenden Anmeldung verwendet wird, umfasst Fluor, Chlor, Brom und lod.

Die erfindungsgemäßen Verbindungen weisen wenigstens eine Gruppe B, welche eine Sulfonsäuregruppe (-SO₃H) enthält, gebunden an ein Stickstoffatom des chromophoren Systems, also in Position R₂, R₃, R₈ und/oder R₉ auf. Erfindungsgemäß wurde festgestellt, dass durch Einführen von Sulfonsäuregruppen-enthaltenden Resten an diesen Positionen Fluoreszenzfarbstoffe bereitgestellt werden können, die eine deutlich verringerte und insbesondere keine unspezifische Bindung an Gefäßwände sowie keine Bindung an vom gewünschten Analyten verschiedene Substrate zeigen. Weiterhin bilden die erfindungsgemäßen Verbindungen auch in wässrigen Lösungen keine nichtfluoreszierenden Farbstoffaggregate. Dadurch ist es möglich, eine hohe mittlere Anzahl an Farbstoffmolekülen pro Analytmolekül (DOL) einzusetzen, ohne dass es zu unerwünschten Aggregationsphänomenen und somit zu einer unerwünschten Sättigung kommt. Weiterhin ist durch die Einführung von Sulfonsäuregruppen in den Resten R₂, R₃, R₈ und/oder R₉ das Einbringen von einer großen Anzahl an Sulfonsäuregruppen in die erfindungsgemäßen Verbindungen möglich.

Besonders gute Ergebnisse werden erhalten, wenn die Gruppe B eine aromatische Sulfonsäuregruppe aufweist. Beispiele für B in den erfindungsgemäßen Verbindungen der allgemeinen Formeln (I) - (IV) sind die nachfolgenden Strukturen (H), (I) oder (J), 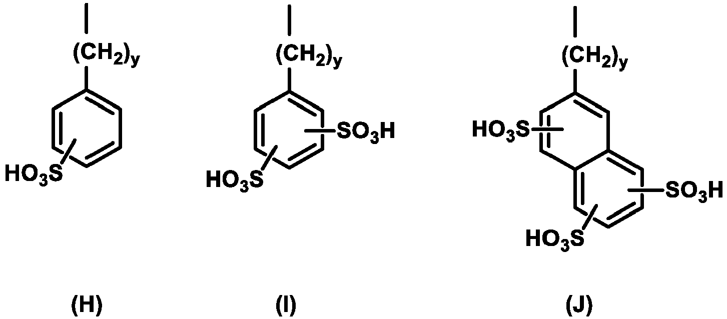 mit y = 0, 1, 2, 3, 4, 5, 6 bevorzugt y = 0,1 oder 2, stärker bevorzugt y = 1 oder 2, noch stärker bevorzugt y = 1 und wobei nicht näher bezeichnete Positionen wie R₁ definiert sind.

Besonders bevorzugt handelt es sich bei der Gruppe B, die einen oder mehrere der Reste R₂, R₃, R₈ und/oder R₉ darstellt, um einen Rest ausgewählt aus

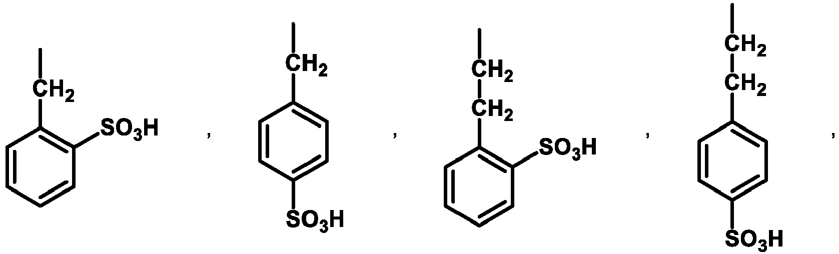

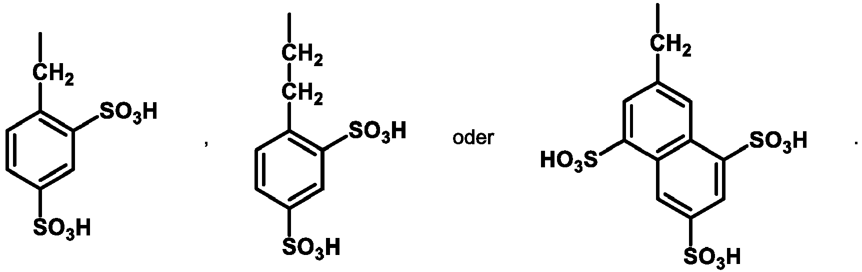

Außerdem kann es sich bei B um eine der vom Cyanurchlorid abgeleitete Strukturen (K) oder (L) mit x = 1, 2, 3, 4, 5, 6 bevorzugt x = 1 oder 2, stärker bevorzugt x = 2 handeln

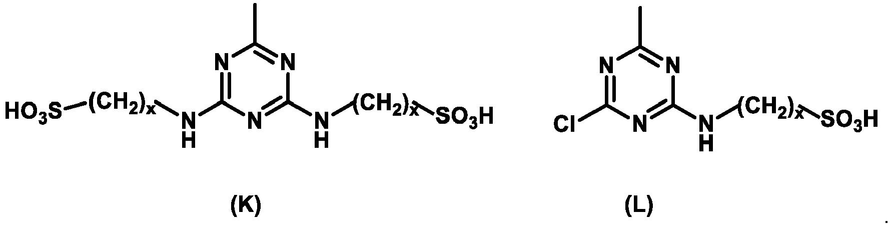

In einer weiteren Ausführungsform handelt es sich bei der Gruppe B um -CH₂SO₃H oder um - CH₂CH₂SO₃H, noch mehr bevorzugt um -CH₂-SO₃H.

Bevorzugte Bedeutungen für R₂, R₃, R₈ und/oder R₉, soweit diese Reste keine Sulfonsäuregruppierung enthalten, sind Wasserstoff, -CH₃, -CH₂CH₃ sowie -CH₂CH₂CH₂COOH.

Die Reste R₁, R₄, R₇ und/oder R₁₀ in den Verbindungen der Formeln (I) - (IV) sind bevorzugt Wasserstoff oder -SO₃H. Durch eine oder mehrere -SO₃H Gruppen an diesen Positionen kann die Gesamtzahl an Sulfonsäuregruppen in den erfindungsgemäßen Verbindungen weiter erhöht werden.

In einer bevorzugten Ausführungsform der erfindungsgemäßen Verbindungen der allgemeinen Formeln (I) - (IV) bilden R₁ und R₂, R₃ und R₄, R₇ und R₈ oder/und R₉ und R₁₀ zusammen mit den Atomen, an welche sie gebunden sind, einen 5- oder 6-gliedrigen Ring, wobei 6-gliedrige Ringe stärker bevorzugt sind. Der mindestens eine 5- oder 6-gliedrige Ring kann dabei eine oder mehrere Doppelbindungen oder/und einen oder mehrere Substituenten, beispielsweise 1, 2, 3 oder 4 Substituenten, umfassen, welche ihrerseits gleich oder verschieden sein können. Die Fluoreszenz derartiger Verbindungen ist besonders effizient und zeigt lediglich eine geringe Abhängigkeit von Temperatur und Umwelteinflüssen. Der mindestens eine 5- oder 6-gliedrige Ring kann einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und eine Kohlenwasserstoffgruppe mit 1-20 C-Atomen, bevorzugt 1-12 C-Atomen, stärker bevorzugt 1-6 C-Atomen umfassen, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst. Besonders bevorzugt werden als Substituenten des mindestens einen 5- oder 6-gliedrigen Ringes Kohlenwasserstoffgruppen mit 1-20 C-Atomen, insbesondere Alkylgruppen mit 1-6 C-Atomen, wobei die Kohlenwasserstoffgruppen im Falle einer Substitution vorzugsweise einen oder mehrere Substituenten ausgewählt aus der Gruppe bestehend aus COOH, COO(Alkyl), COO(Aryl) und SO₃H umfassen.

Besonders bevorzugt sind Verbindungen, die einen oder mehrere Substituenten -CH₂-SO₃H an aus R₁ und R₂, R₃ und R₄, R₇ und R₈ oder/und R₉ und R₁₀ gebildeten Ringen aufweisen.

So lassen sich in diesen Ringen weitere Sulfonsäuregruppen einführen, d.h. bevorzugte Ausführungsformen sind die Strukturen (C), (D), (E), (F) oder (G) 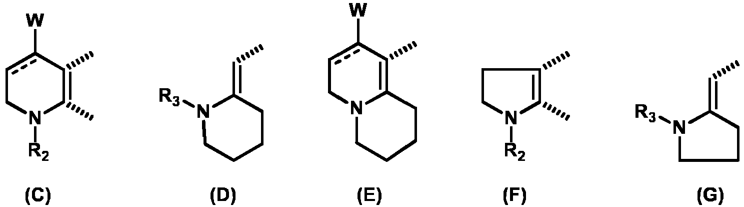 mit W = H, CH₃ oder CH₂SO₃H und wobei nicht näher bezeichnete Positionen in den gezeigten Ringsystemen wie R₁ definiert sind und gestrichelte Linien (- - -) gegebenenfalls vorhandene Doppelbindungen bezeichnen. Die gleichen Strukturen (C), (D), (E), (F) oder (G) können auch unabhängig voneinander zweimal in den Verbindungen der allgemeinen Formeln (I), (II) oder (III) vorhanden sein, wobei dann entsprechend in den angegebenen Strukturen R₉ bzw. R₈ für R₂ bzw. R₃ einzusetzen sind.

Besonders bevorzugte Ausführungsformen umfassen die Strukturen (C) und/oder (E).

Besonders bevorzugt sind Verbindungen der Formeln (I) - (IV) umfassend eine der folgenden Strukturen

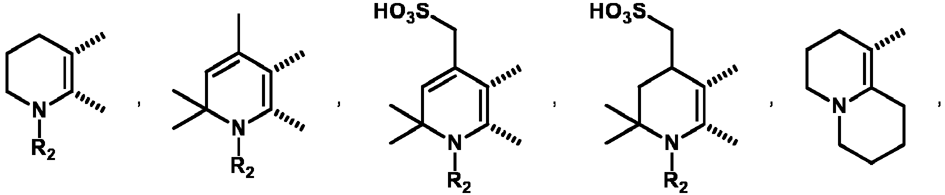

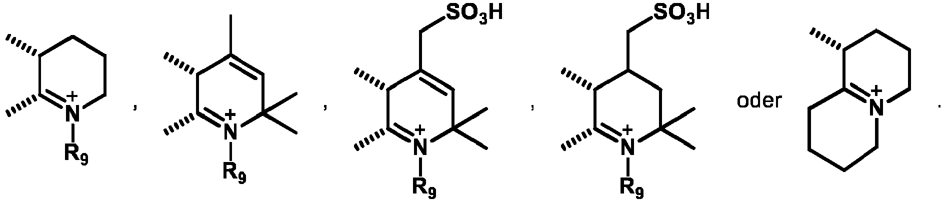

In den erfindungsgemäßen Verbindungen der allgemeinen Formeln (I) und (II) bedeutet X bevorzugt eine Gruppe ausgewählt aus O, CR₁₁R₁₂, SiR₁₃R₁₄ und in der allgemeinen Formel (III) bedeutet X bevorzugt O, CR₁₁R₁₂. Sofern X durch CR₁₁R₁₂ oder SiR₁₃R₁₄ repräsentiert wird, so stellt vorzugsweise mindestens einer der Reste R₁₁, R₁₂, R₁₃, oder R₁₄ eine Kohlenwasserstoffgruppe mit 1-20 C-Atomen, bevorzugt eine Alkylgruppe mit 1-6 C-Atomen, stärker bevorzugt Methyl oder Ethyl, dar.

Die Verbindungen der Formel (I) weisen einen Rest A auf. In bevorzugten Ausführungsformen der Erfindung handelt es sich bei A (Aryl-System, "Rhodamin-artig") in der allgemeinen Formel (I) um eine der Strukturen (M), (N), (O), (P) oder (Q) 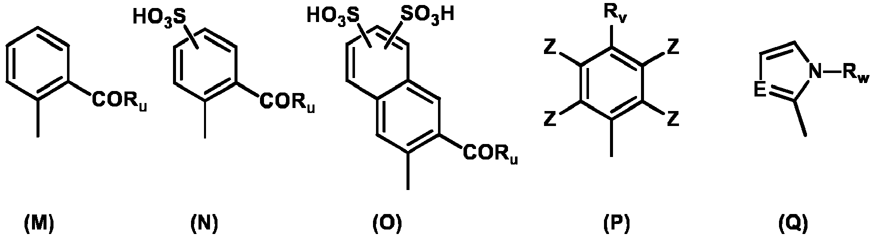 wobei
E eine Gruppe ausgewählt aus O, S, N oder ⁺NR₁₉ darstellt,
Rᵤ = Halogen, OR₂₀, NHR₂₁, NR₂₂R₂₃,
Z ausgewählt aus Wasserstoff oder Halogen ist,
Rᵥ = Z, S(CH₂)ₓCOOH, S(CH₂)ₓSO₃H, NHR₂₄, NR₂₅R₂₆ und
R_{w} = H, Alkyl, (CH₂)ₓCOOH, (CH₂)ₓSO₃H mit x = 1, 2, 3, 4, 5, 6 bedeutet,
R₁₉ und R₂₀ unabhängig voneinander Wasserstoff oder eine Kohlenwasserstoffgruppe mit 1-20 C-Atomen, bevorzugt 1-12 C-Atomen, stärker bevorzugt 1-6 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H enthält, und
R₂₁, R₂₂ und R₂₃ sowie R₂₄, R₂₅ und R₂₆ genau wie R₁₆, R₁₇ und R₁₈ definiert sind und nicht näher bezeichnete Positionen wie R₁ definiert sind.

Bevorzugte Gruppen A sind

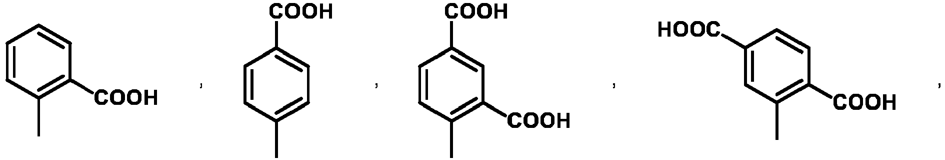

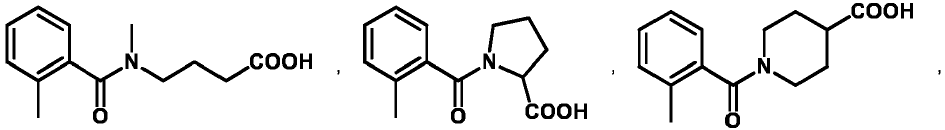

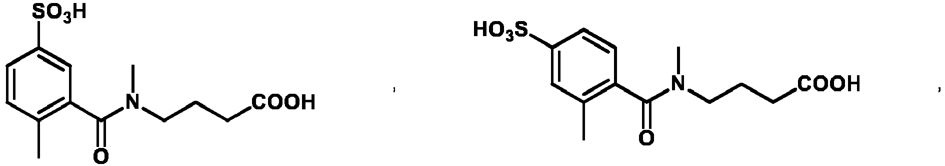

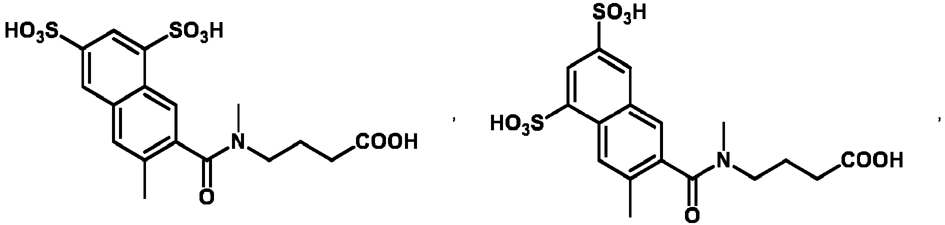

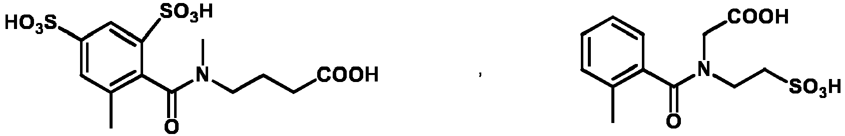

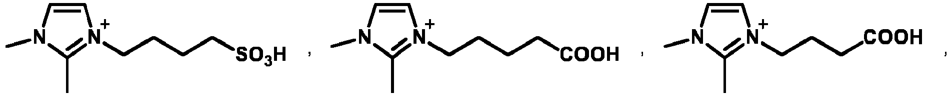

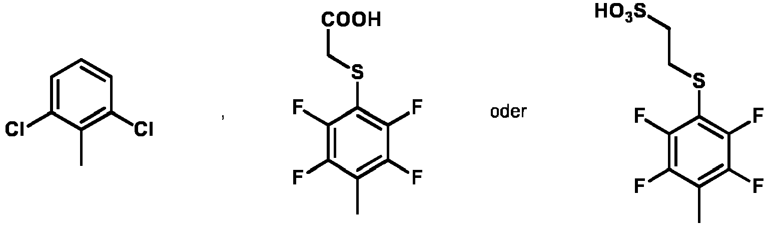

Besonders bevorzugt sind Verbindungen der Formel (I) in denen X = O (Rhodamine), X = S (Thiorhodamine), X = C(CH₃)₂ (Carborhodamine) oder X = Si(CH₃)₂ (Silicorhodamine).

In Verbindungen der Formel (I) mit X = O stellt der Rest B bevorzugt keine Sulfoalkylgruppe dar und insbesondere keinen Rest -CH₂-CH₂-CH₂-SO₃H (Sulfopropylgruppe).

Soweit in Verbindungen der Formel (I) mit X = O der Rest B eine Sulfoalkylgruppe darstellt, ist vorzugsweise wenigstens eine weitere SO₃H-Gruppe im Molekül, insbesondere in den Resten R₁, R₄, R₅, R₆, R₇, R₁₀ oder/und A, enthalten.

Die Verbindungen der Formel (II) weisen eine Gruppierung Y auf. In bevorzugten Ausführungsformen der Erfindung handelt es sich bei Y (aliphatische und sonstige Substituenten, "Pyronin-artig") in der allgemeinen Formel (II) um Wasserstoff, CN, CF₃, NHR₁₆, NR₁₇R₁₈, (CF₂)ₓCOOH mit x = 1, 2, 3, 4, 5, 6, oder die Struktur (T) 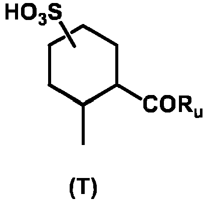
wobei Rᵤ = Halogen, OR₂₀, NHR₂₁, NR₂₂R₂₃ und nicht näher bezeichnete Positionen wie R₁ definiert sind.
Y ist besonders bevorzugt ausgewählt aus Wasserstoff, CN, -NH-(CH₂)₃-COOH, -NH-(CH₂)₅-COOH, -NH-CH(COOH)-(CH₂)₂-SO₃H, -NH-CH₂-CH(SO₃H)₂ oder -NH-CH₂-CH₂-SO₃H.

Besonders bevorzugt sind Verbindungen der Formel (II) in denen X = C(CH₃)₂ (Carbopyronine) oder X = Si(CH₃)₂ (Silicopyronine).

Weiterhin sind Verbindungen der Formel (III) bevorzugt, in denen X = O (Oxazine) oder X = C(CH₃)₂ (Carbazine).

Schließlich sind Verbindungen der Formel (IV) bevorzugt, in denen X = O (Cumarine) oder X = NR (Carbostyrile).

R₂ in Verbindungen der Formel (IV) ist vorzugsweise ein Rest B ausgewählt aus CH₂SO₃H, CH₂CH₂SO₃H,

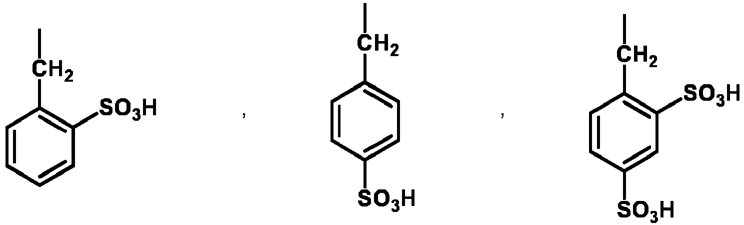

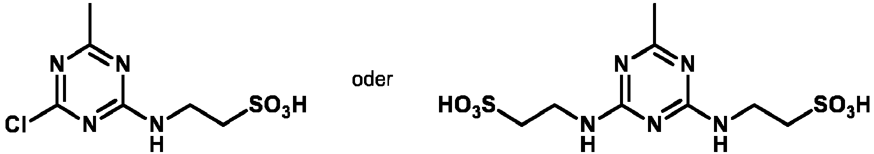

In Verbindungen der Formel (IV) ist weiterhin bevorzugt wenigstens einer der Reste R₁, R₄ und/oder R₇, insbesondere R₁ und/oder R₄ eine Sulfonsäuregruppe (-SO₃H).

In Verbindungen der Formel (IV), in denen X = O stellt der Rest B, für den Fall, dass es sich um eine aliphatische Kohlenwasserstoffgruppe handelt, bevorzugt -CH₂SO₃H oder -CH₂CH₂SO₃H dar.

Erfindungsgemäß werden Verbindungen bereitgestellt, die an wenigstens einem der Stickstoffatome der Verbindungen der Formeln (I) - (IV) eine Sulfonsäuregruppe enthaltende Gruppierung aufweisen. Die Sulfonsäuregruppen können dabei über die Reste R₂, R₃, R₈ oder R₉ bereitgestellt werden, wobei in einer bevorzugten Ausführungsform die Gesamtzahl an Sulfonsäuregruppen in den Resten R₂, R₃, R₈ und R₉ wenigstens 2, insbesondere wenigstens 3, bevorzugt wenigstens 4, noch mehr bevorzugt wenigstens 5 und am meisten bevorzugt wenigstens 6 beträgt. Es wurde festgestellt, dass solche Verbindungen ein hohes DOL ermöglichen, ohne dass Aggregationsphänomene beobachtet werden, die das Fluoreszenzsignal verringern. Weiterhin werden für diese Verbindungen eine hohe Wasserlöslichkeit und eine geringe Aggregationsneigung in wässrigen Medien beobachtet.

Erfindungsgemäß ist es weiterhin möglich und bevorzugt, zusätzlich zu den in den Resten R₂, R₃, R₈ und R₉ bereitgestellten Sulfonsäuregruppierungen weitere Sulfonsäuregruppen einzufügen, bevorzugt in der Form von -SO₃H an den Positionen R₁, R₄, R₇ und/oder R₁₀ sowie bevorzugt als -CH₂-SO₃H als Substituenten an Ringsystemen, gebildet durch R₁ und R₂, R₃ und R₄, R₇ und R₈ oder/und R₉ und R₁₀.

Die Anzahl der über die Reste R₁, R₄, R₇ und/oder R₁₀ eingeführten Sulfonsäuregruppen beträgt vorzugsweise wenigstens 1 und noch mehr bevorzugt wenigstens 2. Die Anzahl der über die ein Ringsystem bildenden Gruppierungen R₁ und R₂, R₃ und R₄, R₇ und R₈ und/oder R₉ und R₁₀ eingeführten Sulfonsäuregruppierungen beträgt vorzugsweise 1 und noch mehr bevorzugt wenigstens 2.

Weiterhin ist es möglich und bevorzugt, weitere Sulfonsäuregruppen über die Gruppierungen A und Y einzuführen. Auf diese Weise können Verbindungen mit einer großen Gesamtzahl an Sulfonsäuregruppen bereitgestellt werden. Bevorzugt weisen die erfindungsgemäßen Verbindungen im gesamten Molekül wenigstens 2, insbesondere wenigstens 3, bevorzugt wenigstens 4, noch mehr bevorzugt wenigstens 5 und am meisten bevorzugt wenigstens 6 Sulfonsäuregruppen auf.

Aus den protonierten, sauren Formen der Verbindungen können ionische/geladene Salze erhalten werden, wenn sie mit Laugen bzw. Basen neutralisiert werden. Diese Salze besitzen z.T. eine veränderte Löslichkeit in polaren, vor allem wässrigen, Medien. Aber ansonsten sind solche Salze äquivalent zu den gezeigten Formeln.
Darüber hinaus können die Verbindungen auch in einer solvatisierten, vor allem hydratisierten, Form vorliegen bzw. ein oder mehrere Kristallwassermoleküle enthalten. Auch kristalline oder amorphe Formen sind äquivalent zu den gezeigten Formeln.
Einige Verbindungen besitzen asymmetrische Kohlenstoffatome als optische Zentren oder Doppelbindungen. Die daraus resultierenden Racemate, Diastereomere, geometrischen Isomere etc. sind alle von der vorliegenden Erfindung umfasst.
Darüber hinaus können die Verbindungen auch unnatürliche Isotopenverhältnisse aufweisen bzw. sogar radioaktiv markiert sein. Diese Isotopenvariationen, egal ob radioaktiv oder nicht, werden ebenfalls von den gezeigten Formeln umfasst.

Die Verbindungen der allgemeinen Formeln (I) - (IV) können als Markierungsgruppen in Verfahren zur qualitativen oder/und quantitativen Bestimmung eines Analyten eingesetzt werden. Diese Bestimmung kann vorzugsweise in wässrigen Flüssigkeiten, z.B. Proben von Körperflüssigkeiten wie etwa Blut, Serum, Plasma, Lymphflüssigkeit, Gallenflüssigkeit, Cerebrospinalflüssigkeit, extrazelluläre Gewebeflüssigkeit, Harn, Speichel und Schweiß, Abwasserproben oder Lebensmitteln, durchgeführt werden. Das Verfahren kann sowohl als Nasstest, z.B. in einer Küvette, oder als Trockentest auf einem entsprechenden Reagenzträger durchgeführt werden. Der Träger kann dabei grundsätzlich aus jedem dem Fachmann geeignet erscheinenden Material bestehen, das von der zu untersuchenden Probe benetzt werden kann. Beispiele für derartige Trägermaterialien umfassen poröses Glas, Kunststoffe, Ionenaustauschharze, Dextrane, Cellulose, Cellulosederivate oder/und hydrophile Polymere, sind jedoch nicht auf diese beschränkt. Die Bestimmung des Analyten kann hierbei mittels einer einzigen Reaktion oder durch eine Sequenz von Reaktionen erfolgen. Die Analyten sind häufig Biomoleküle, vorzugsweise ausgewählt aus Peptiden, Polypeptiden, Proteinen, Nukleotiden, Nukleosiden, Nukleinsäuren, Nukleinsäureanaloga oder/und Haptenen.

Die Verbindungen der allgemeinen Formeln (I) - (IV) können in allen dem Fachmann bekannten chemischen, medizinischen und biologischen Nachweisverfahren, in denen Fluoreszenzfarbstoffe als Markierungsgruppen geeignet sind, verwendet werden. Hierzu werden die Verbindungen im Allgemeinen kovalent an einen für den nachzuweisenden Analyten spezifischen Rezeptor oder den Analyten selber gekoppelt. Hierzu verfügen die erfindungsgemäßen Verbindungen der allgemeinen Formeln (I) - (IV) vorzugsweise über mindestens eine zur kovalenten Kopplung befähigte funktionelle Gruppe, wie beispielsweise OH, SH, NH₂ und/oder COOH.

Ein allgemein bekanntes Verfahren ist die Kopplung des N-Hydroxysuccinimidylesters des Farbstoffs an eine Aminogruppe des Substrats. Der spezifische Rezeptor oder Analyt kann jede geeignete Verbindung oder jedes geeignete Molekül sein, vorzugsweise ist es ein Peptid, ein Polypeptid oder eine Nukleinsäure. Eine Vielzahl gängiger Kopplungsmethoden wird z.B. beschrieben in Greg T. Hermanson, Bioconjugate Techniques, Academic Press, San Diego, CA, 1996.

Neben diesen herkömmlichen Verfahren hat sich in den letzten Jahren die so genannte "Click-Chemie" etabliert. Dabei werden nach verschiedenen Reaktionsprinzipien zwei Moleküle über funktionelle Gruppen bio-orthogonal - d.h. die beiden Reaktionspartner reagieren gezielt auch in Gegenwart vieler anderer Funktionalitäten und ohne die biologischen Vorgänge zu beeinflussen - miteinander verknüpft. Das bekannteste Beispiel ist die Cu(I)-katalysierte 1,3-dipolare Cycloaddition (Huisgen-Cycloaddition) eines Alkins und eines Azids. Auf diesem sehr aktuellen Forschungsgebiet werden stetig neue verbesserte Reaktionsmethoden erforscht. So wurden Cu-freie und damit biokompatiblere Reaktionen entwickelt. Einen Überblick über die Prinzipien findet man z.B. in K. Nwe, M.W. Brechbiel, Growing Applications of "Click Chemistry" for Bioconjugation in Contemporary Biomedical Research, Cancer Biotherapy and Radiopharmaceuticals, 2009, 24 (3), 289-302.

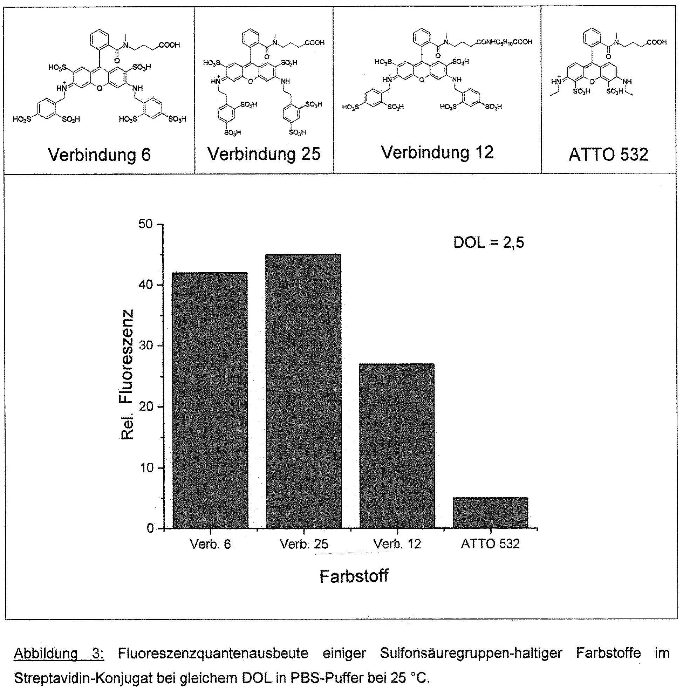

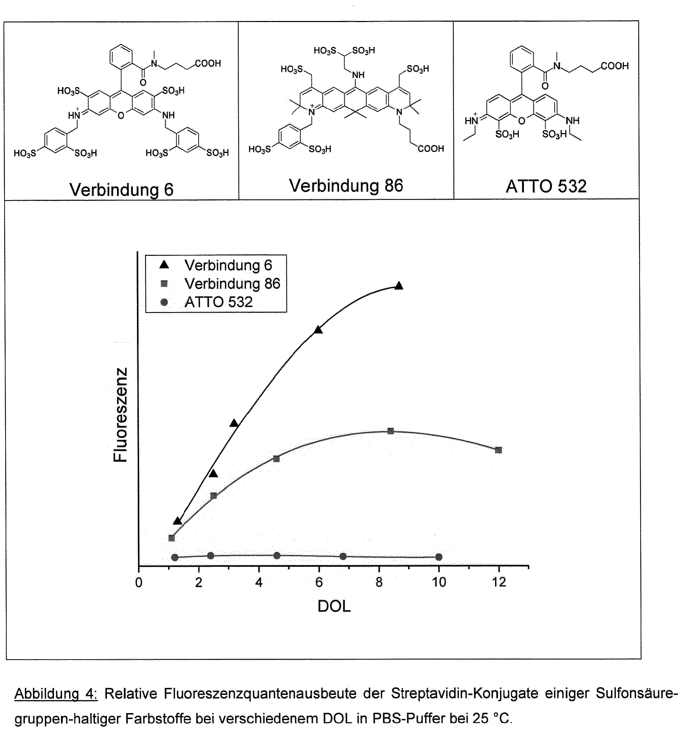

Sowohl die Absorptions- und Fluoreszenzmaxima als auch vor allem die Fluoreszenzquantenausbeute werden durch eine der soeben beschriebenen Kopplungen von erfindungsgemäßen Verbindungen an die oben genannten Träger und Biomoleküle - im Gegensatz zu den bisher oft verwendeten Fluoreszenzsonden - nicht wesentlich verändert.
Beispielhaft werden diese Ergebnisse durch die Kopplung von erfindungsgemäßen Farbstoffen an das Modellprotein Streptavidin erläutert. Die in der Tabelle 1 aufgeführten Verbindungen zählen zur Klasse der Rhodamine mit demselben chromophoren System. Sie weisen in wässriger Lösung bei Raumtemperatur alle eine vergleichbar hohe Fluoreszenzquantenausbeute von etwa 90 % auf. Während aber die Quantenausbeute bei den dem Stand der Technik entsprechenden Farbstoffen nach Kopplung an Streptavidin stark reduziert wird (vgl. Tabelle 1, Beispiel ATTO 532), bleibt die Quantenausbeute bei den erfindungsgemäßen Farbstoffen selbst bei einem DOL von etwa 5 oder 7 überraschenderweise außerordentlich hoch (siehe Tabelle 1, Beispiele für erfindungsgemäße Verbindungen). Damit lässt sich bei gängigen Markierungsverfahren eine viel bessere Detektionssicherheit erreichen. Wie in den Abbildungen 3 und 4 graphisch dargestellt, ist die erhaltene Fluoreszenz im Konjugat gegenüber beispielhaften Farbstoffen des Stands der Technik stark erhöht.

**Tabelle 1: Fluoreszenzquantenausbeute einiger Sulfonsäuregruppen-haltiger Farbstoffe im Streptavidin-Konjugat bei verschiedenen DOL im Vergleich zum ungekoppelten Farbstoff. Eine graphische Darstellung erfolgt in Abbildung 3.**

| **Verbindung** | **DOL** | **Fluoreszenzquantenausbeute des Farbstoffs nach Kopplung an Streptavidin im Vergleich zum ungekoppelten Farbstoff** |
|---|---|---|
| ATTO 532 | 2,5 | 5 % |
| | 4,6 | 3 % |
| 6 | 2,5 | 42 % |
| | 5,5 | 37 % |
| 12 | 3,0 | 27 % |
| 19 | 5,6 | 10 % |
| 25 | 2,8 | 45 % |
| | 6,7 | 35 % |
| 26 | 7,0 | 8 % |
| 27 | 1,5 | 34 % |

Die Fluoreszenzquantenausbeute der erfindungsgemäßen Verbindungen nach Kopplung an Streptavidin im Vergleich zum ungekoppelten Farbstoff beträgt bei einem DOL von 1 vorzugsweise > 20 %, mehr bevorzugt > 30 % und insbesondere > 35 %. Bei einem DOL von 3 beträgt die Fluoreszenzquantenausbeute der erfindungsgemäßen Verbindungen nach Kopplung an Streptavidin im Vergleich zu den ungekoppelten Verbindungen vorzugsweise > 15%, mehr bevorzugt > 20 % und insbesondere > 25%. Bei einem DOL von 5 beträgt die Fluoreszenzquantenausbeute der erfindungsgemäßen Verbindungen nach Kopplung an Streptavidin im Vergleich zu den ungekoppelten Verbindungen vorzugsweise > 10 %, mehr bevorzugt > 15 % und insbesondere > 20 %.

Die Sulfonatgruppe trägt im physiologisch relevanten pH-Bereich eine negative Ladung. Mehrere solcher Gruppen an einem organischen Farbstoff verbessern nicht nur die Wasserlöslichkeit sondern verringern die Aggregationstendenz drastisch. Die erhöhte Anzahl an negativen Ladungen verhindert nämlich eine gegenseitige Anziehung. Vielmehr kommt es zu einer Coulomb-Abstoßung gleicher Ladungen, was außerdem unspezifische Bindungen an negativ geladene "Biopolymere" wie DNA, RNA oder bestimmte Enzyme wirkungsvoll behindert. Die verminderte Aggregation und gegenseitige Abstoßung führt dabei oftmals zu einer erhöhten Fluoreszenz, da die löschenden Wechselwirkungen der beteiligten Chromophore unterbleiben.

Es lassen sich durch die vorliegende Erfindung gezielt anionische Farbstoffmoleküle herstellen, die im Gegensatz zu den positiv geladenen Grundchromophoren eine negative Nettoladung aufweisen.

Ein besonderes Einsatzgebiet von geladenen Farbstoffen ist die elektrophoretische Trennung. Über die Zahl der Sulfonsäuregruppen lassen sich also Farbstoffe mit einer genau definierten Anzahl an negativen Ladungen herstellen. Die Konjugate einer Reihe solcher Farbstoffe können sich dann z.B. jeweils genau um eine Elementarladung unterscheiden, je nachdem, wie viele Sulfonsäuregruppen die entsprechenden Farbstoffgerüste tragen.

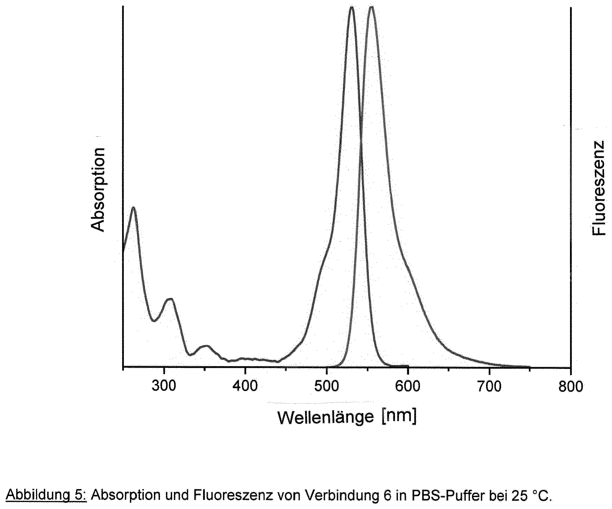

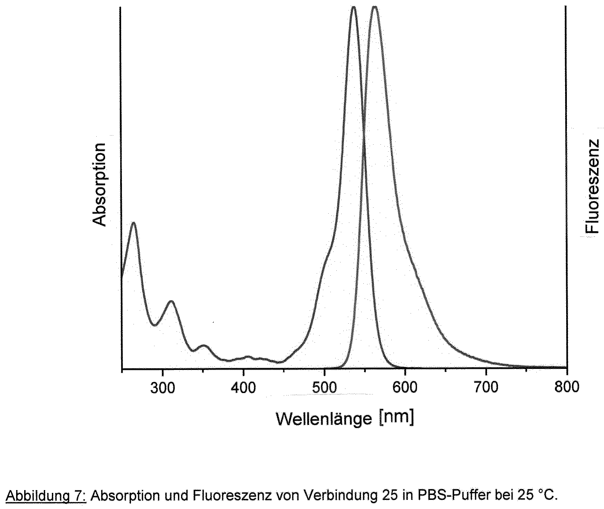

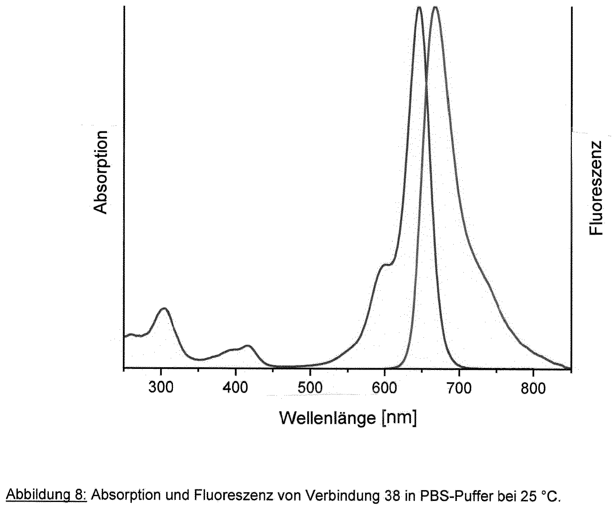

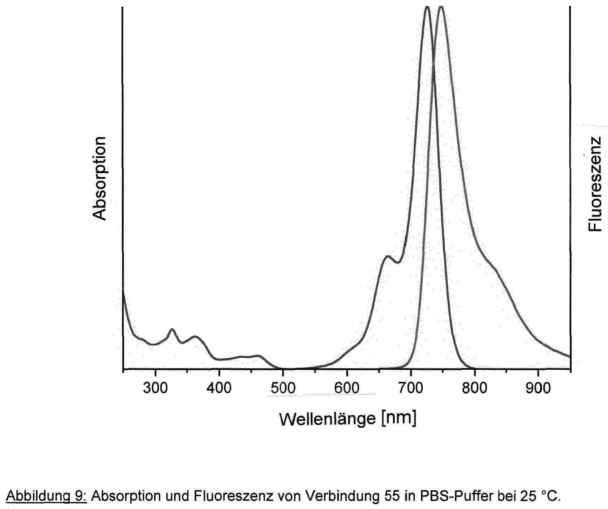

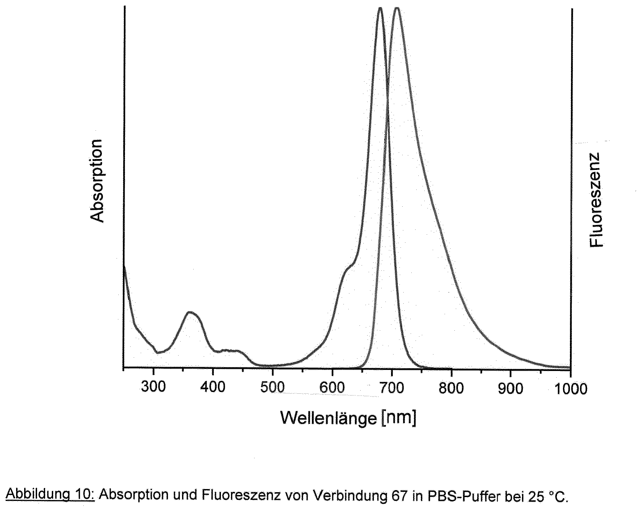

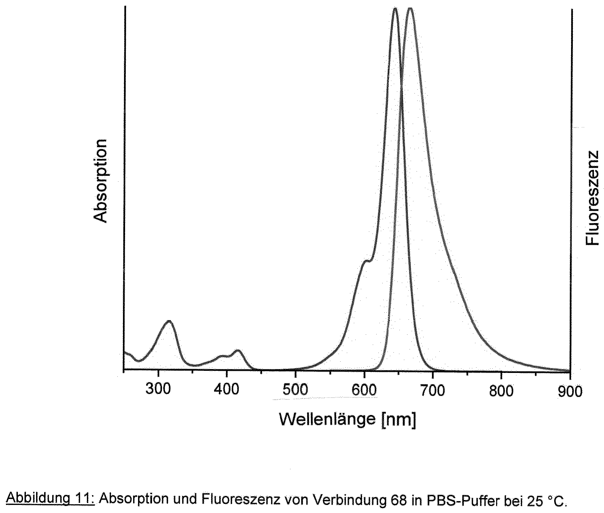

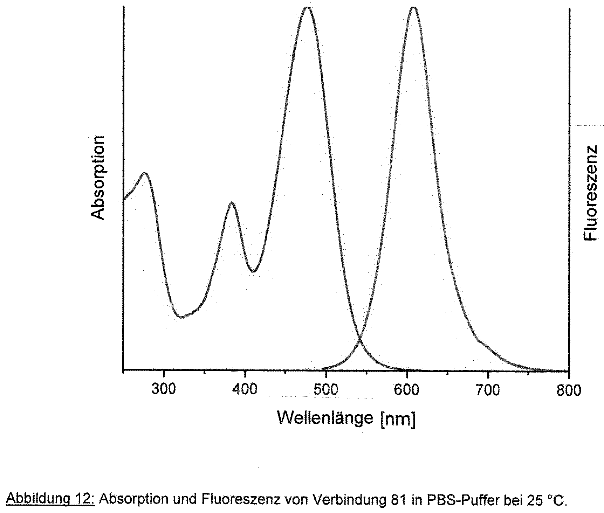

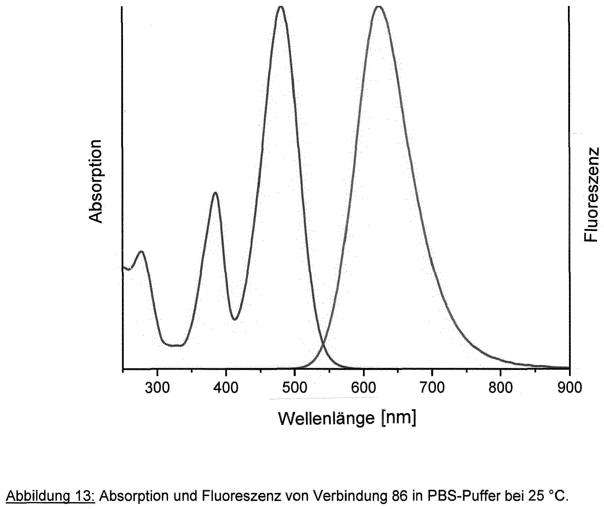

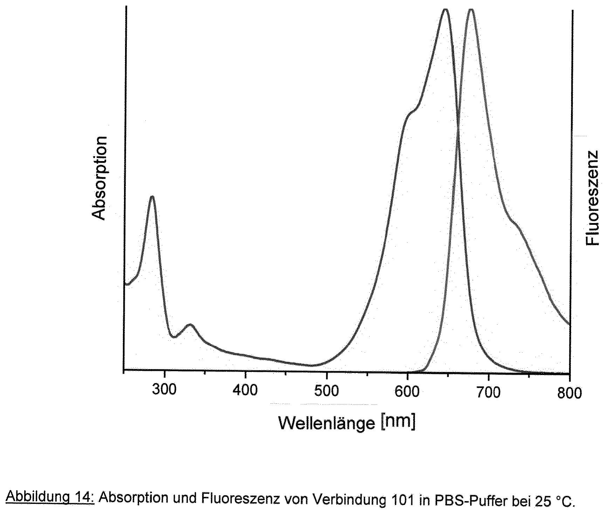

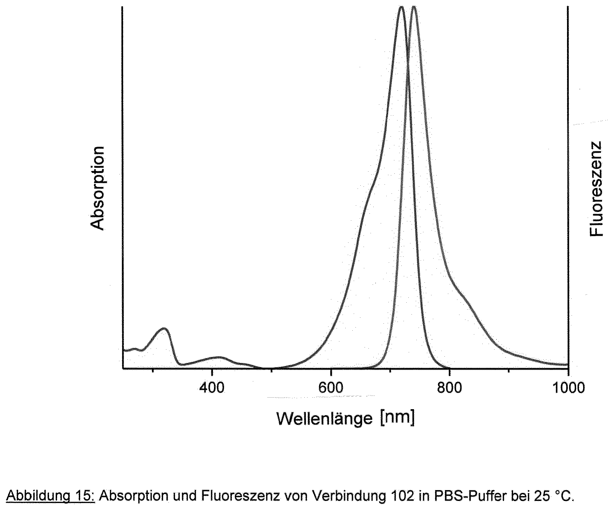

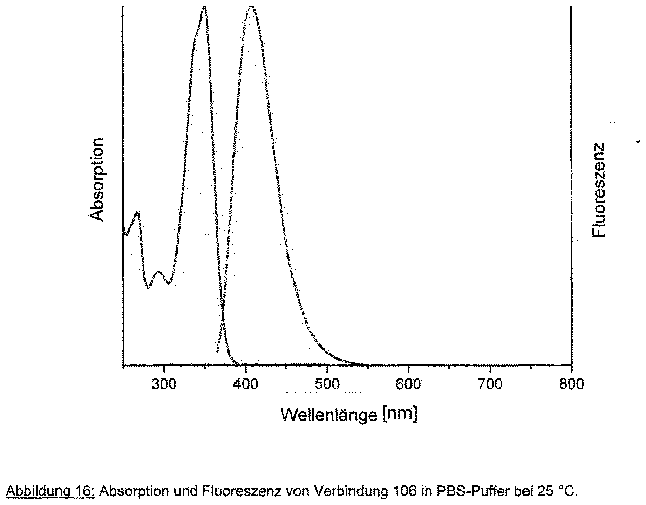

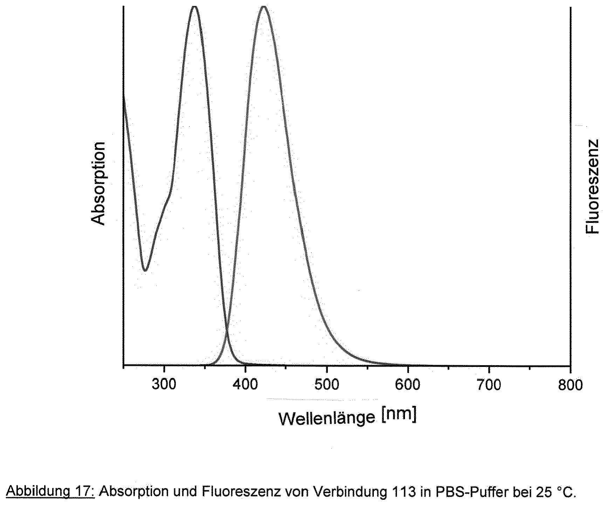

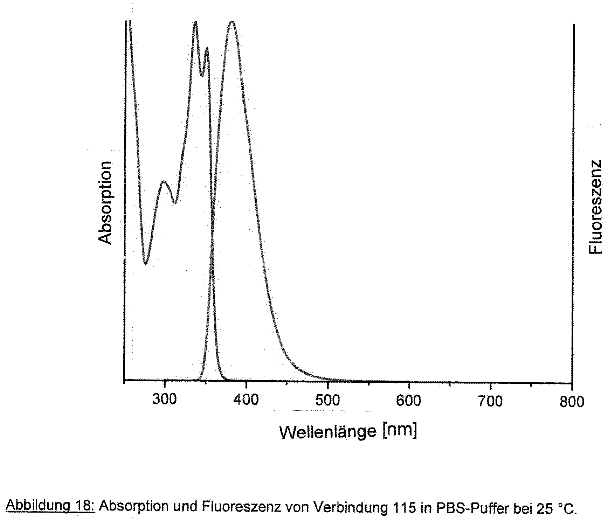

Beispiele für erfindungsgemäße Verbindungen sind in den Tabellen 2 - 6 aufgeführt, dort sind die Strukturen sowie Absorptions- und Fluoreszenzmaxima angegeben. Die Spektren ausgewählter Verbindungen sind in den Abbildungen 5 - 18 dargestellt.

**Tabelle 2: allgemeine Formel (I), Beispiele für X = O (Rhodamine)**

| Lösungsmittel: ^{a} Ethanol, ^{b} PBS pH 7,4, ^{c} Wasser/Acetonitril 1:1 | | | |
|---|---|---|---|
| **Verbindung** | **Struktur** | **λ_{abs} [nm]** | **λ_{fl} [nm]** |
| **1** | 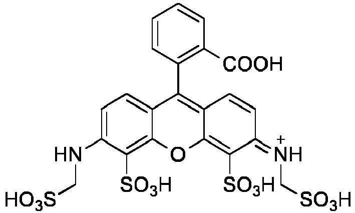 | 533^{c} | 554^{b} |
| **2** | 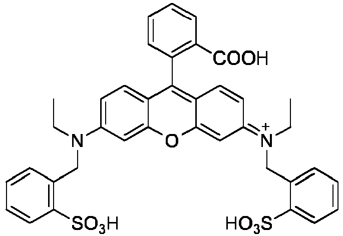 | 554^{c} | 584^{b} |
| **3** | 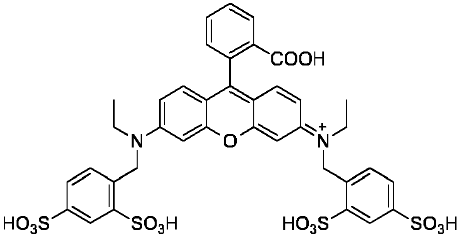 | 550^{c} | 583^{b} |

| **Verbindung** | **Struktur** | **λ_{abs} [nm]** | **λ_{fl} [nm]** |
|---|---|---|---|
| **4** | 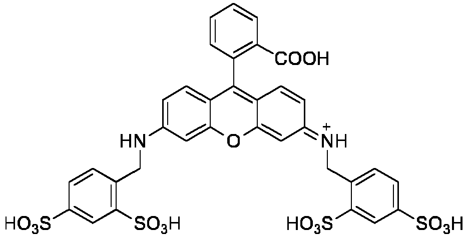 | 536^{c} | 559^{b} |
| **5** | 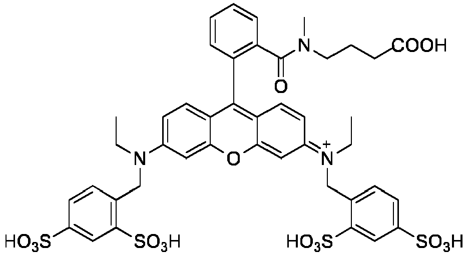 | 560^{c} | 584^{b} |
| **6** | 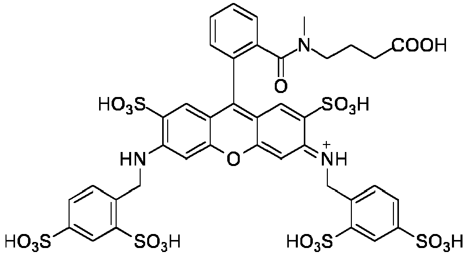 | 534^{c} | 559^{b} |
| **7** | 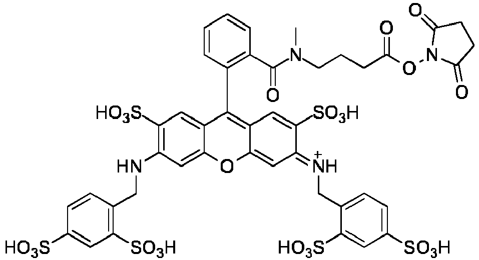 | 534^{c} | 559^{b} |
| **8** | 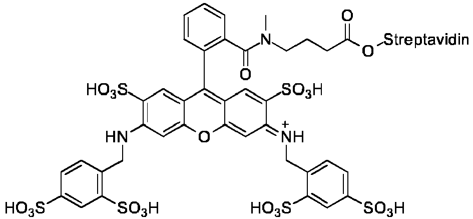 | 533^{c} | 556^{b} |
| **9** | 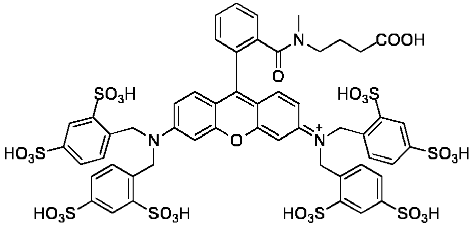 | 550^{c} | 572^{b} |
| **10** | 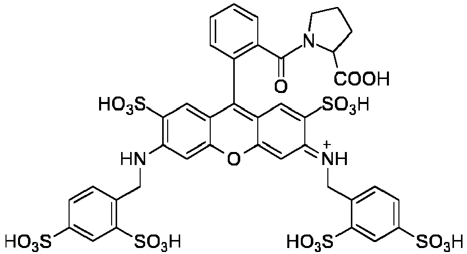 | 534^{c} | 556^{b} |
| **11** | 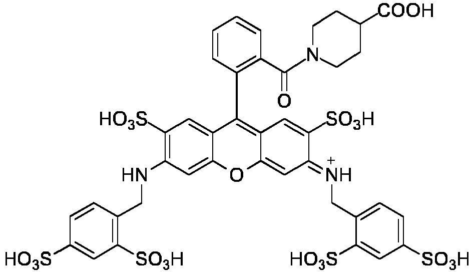 | 529^{c} | 550^{b} |
| **12** | 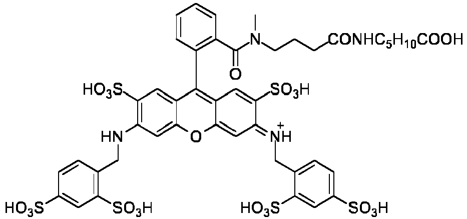 | 536^{c} | 555^{b} |
| **13** | 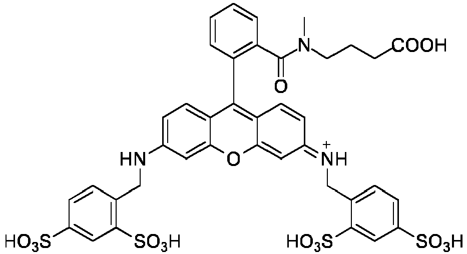 | 534^{c} | 555^{b} |
| **14** | 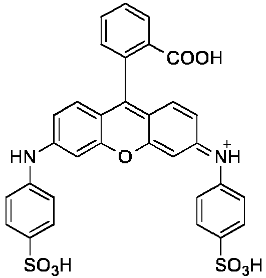 | 560^{b} | --- |
| **15** | 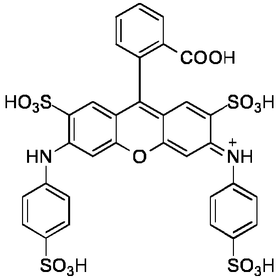 | 558^{c} | --- |
| **16** | 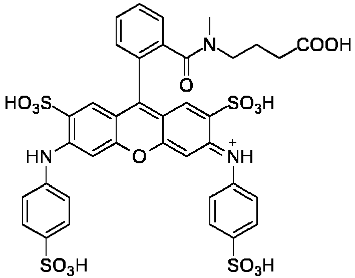 | 566^{c} | --- |
| **17** | 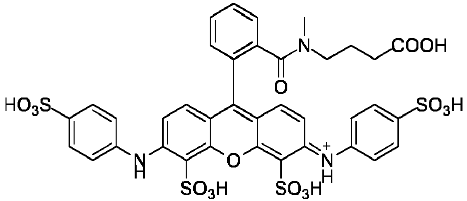 | 555^{c} | - |
| **18** | 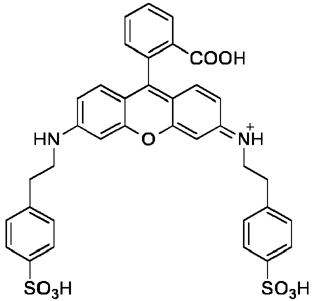 | 530^{c} | 551^{b} |
| **19** | 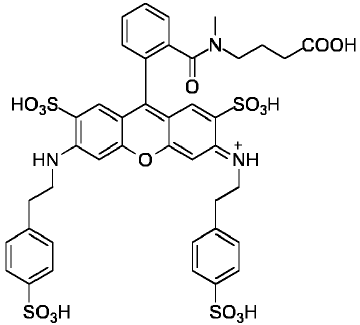 | 537^{c} | 560^{b} |
| **20** | 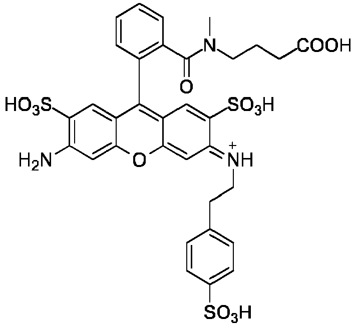 | 528^{c} | 550^{b} |
| **21** | 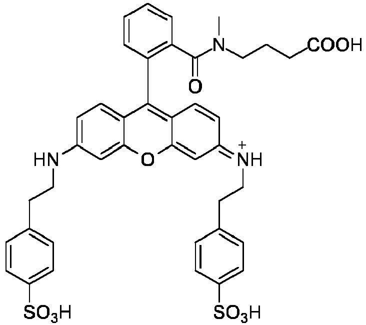 | 538^{c} | 559^{b} |
| **22** | 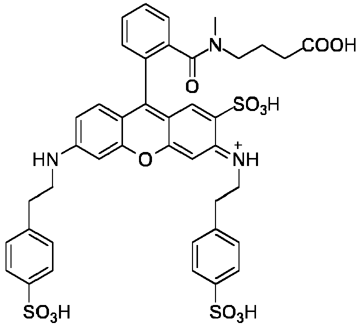 | 536^{c} | 558^{b} |
| **23** | 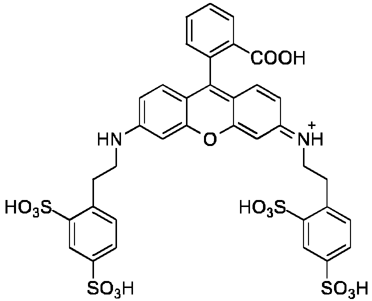 | 532^{c} | 559^{b} |
| **24** | 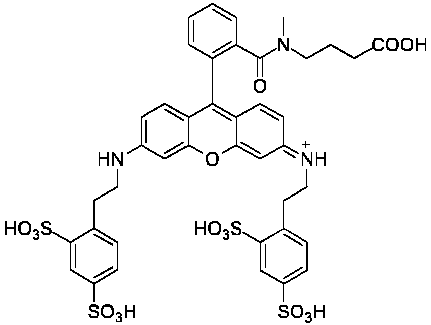 | 539^{c} | 561^{b} |
| **25** | 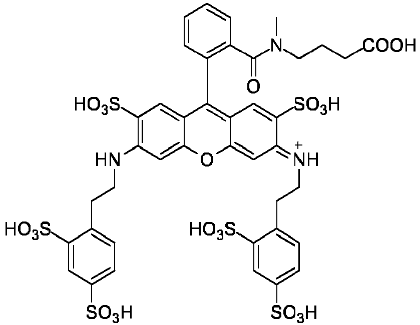 | 539^{b} | 560^{b} |
| **26** | 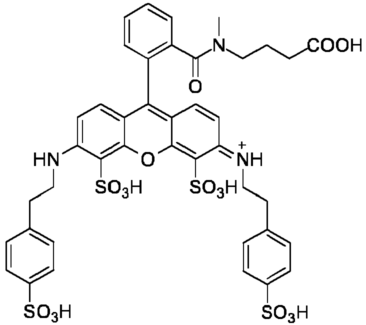 | 538^{c} | 558^{b} |
| **27** | 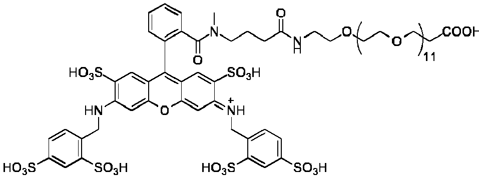 | 539^{c} | 558^{b} |
| **28** | 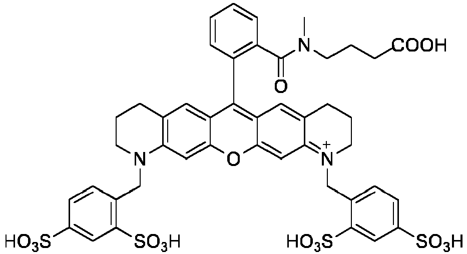 | 567^{c} | |
| **29** | 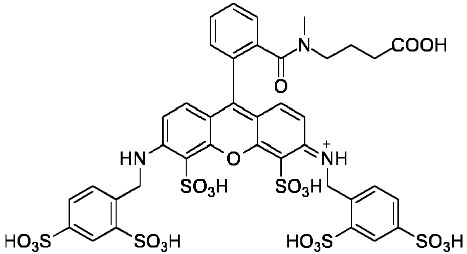 | 531^{c} | |
| **30** | 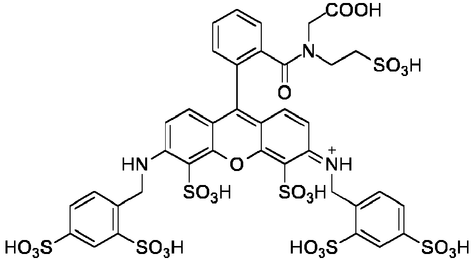 | 536^{c} | |
| **31** | 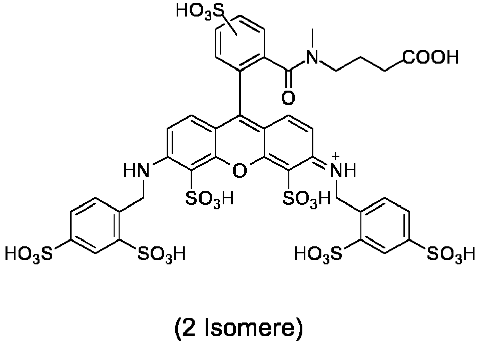 | 532^{c} | |
| **32** | 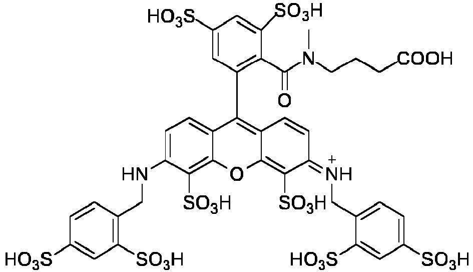 | 530^{c} | |
| **33** | 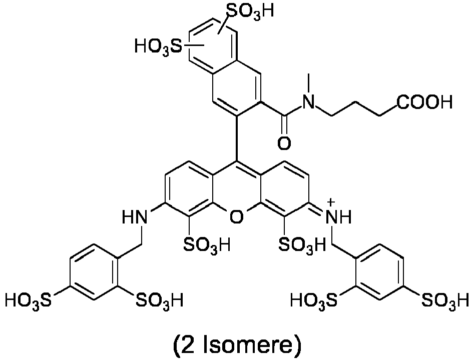 | 539^{c} | |
| **34** | 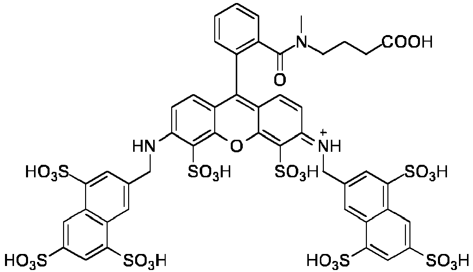 | 530^{b} | |
| **35** | 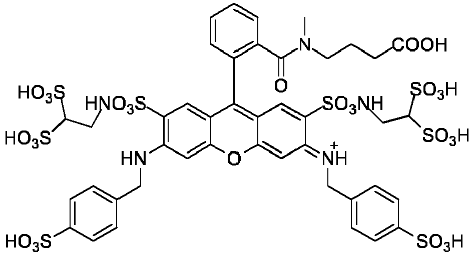 | 540^{b} | 562^{b} |

**Tabelle 3: allgemeine Formel (I), Beispiele für X = C(CH₃)₂ (Carborhodamine), X = Si(CH₃)₂ (Silicorhodamine)**

| Lösungsmittel: ^{a} Ethanol, ^{b} PBS pH 7,4, ^{c} Wasser/Acetonitril 1:1 | | | |
|---|---|---|---|
| **Verbindung** | **Struktur** | **λ_{abs} [nm]** | **λ_{fl} [nm]** |
| **36** | 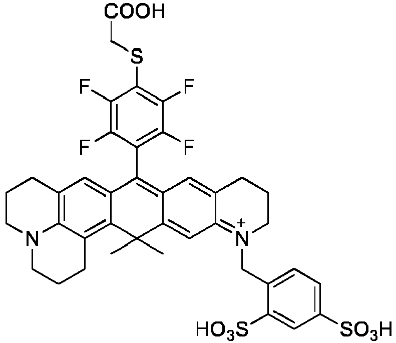 | 656^{b} | 677^{b} |
| **37** | 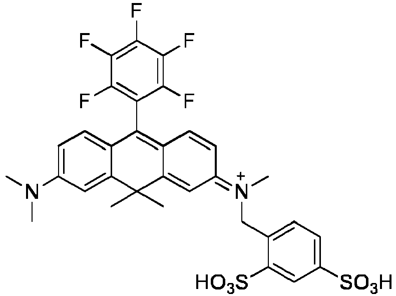 | 642^{b} | 661^{b} |
| **38** | 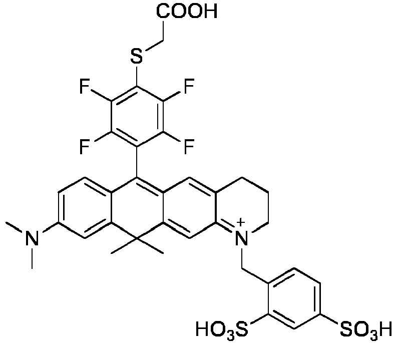 | 646^{b} | 667^{b} |
| **39** | 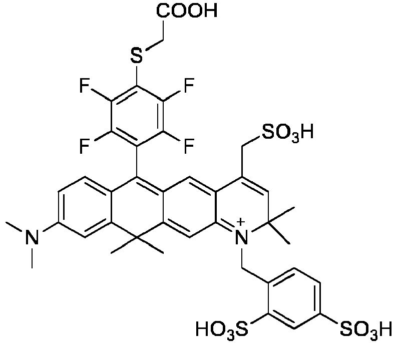 | 673^{b} | 693^{b} |
| **40** | 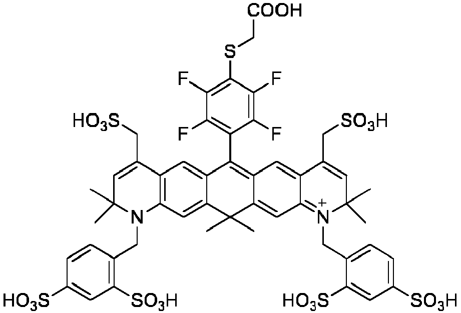 | 699^{b} | 720^{b} |
| **41** | 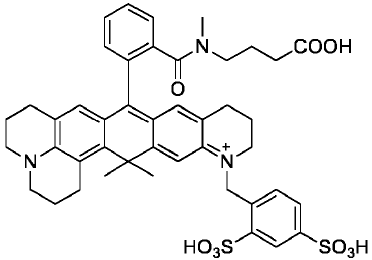 | 642^{a} 640^{b} | 663^{a} 659^{b} |
| **42** | 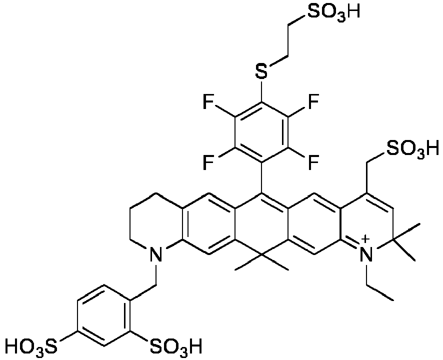 | 682^{b} | 701^{b} |
| **43** | 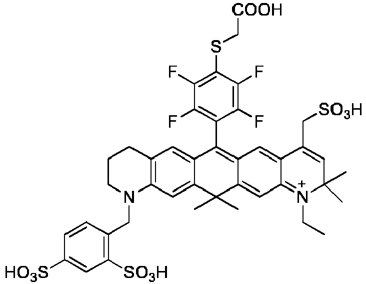 | 684^{b} | 704^{b} |
| **44** | 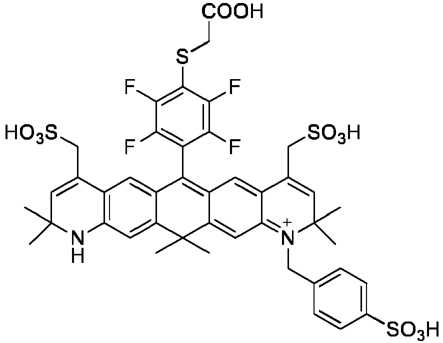 | 687^{b} | 707^{b} |
| **45** | 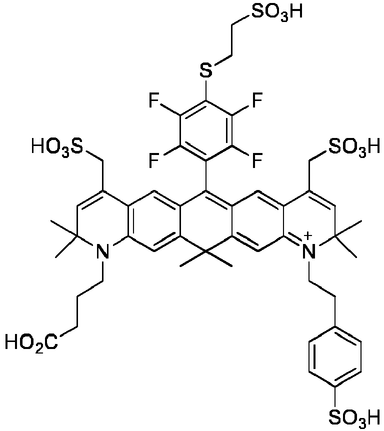 | 698^{b} | 719^{b} |
| **46** | 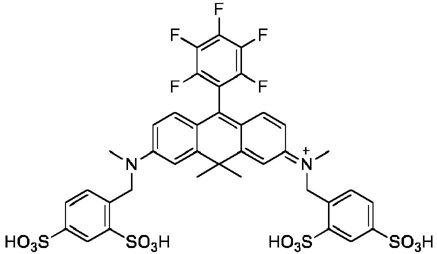 | 646^{b} | 666^{b} |
| **47** | 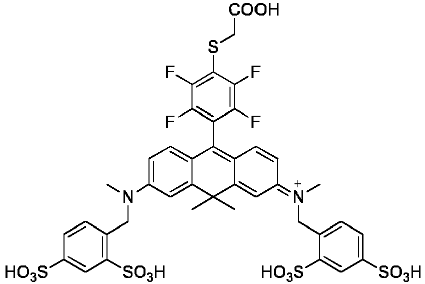 | 645^{b} | 666^{b} |
| **48** | 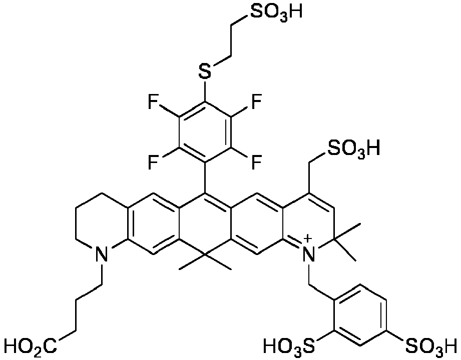 | 682^{b} | 705^{b} |
| **49** | 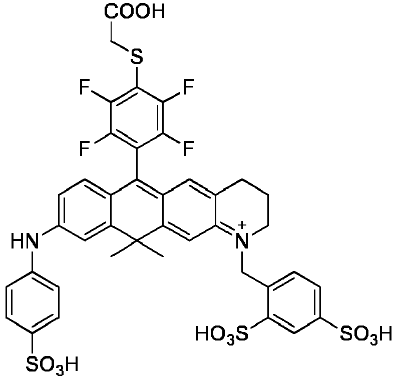 | 648^{b} | --- |
| **50** | 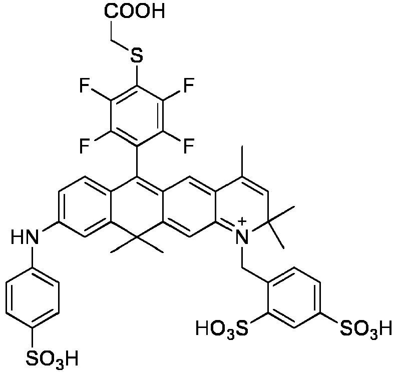 | 669^{b} | --- |
| **51** | 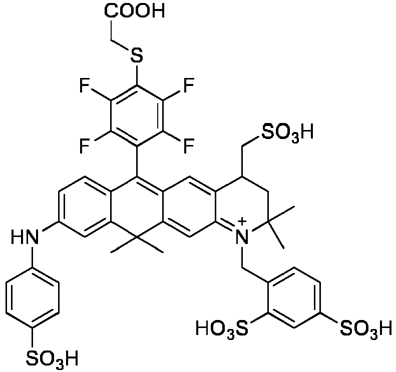 | 650^{b} | --- |
| **52** | 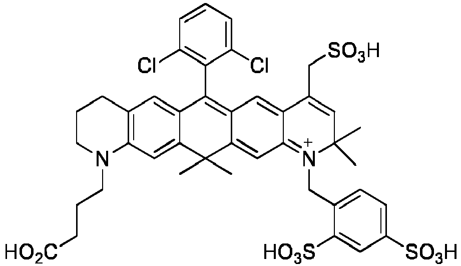 | 673^{b} | 698^{b} |
| **53** | 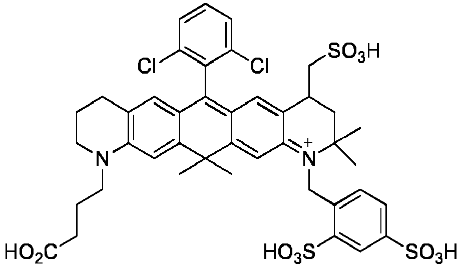 | 654^{b} | 677^{b} |
| **54** | 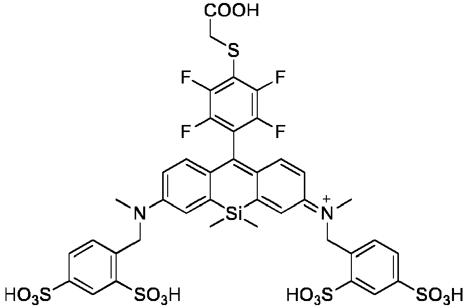 | 685^{b} | 709^{b} |
| **55** | 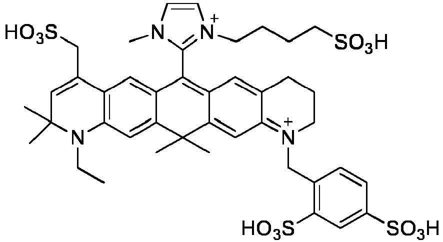 | 725^{b} | 749^{b} |
| **56** | 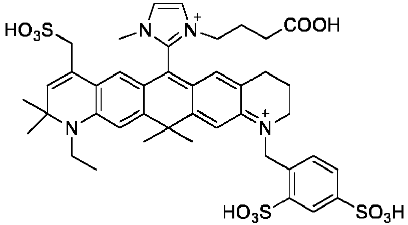 | 724^{b} | 746^{b} |
| **57** | 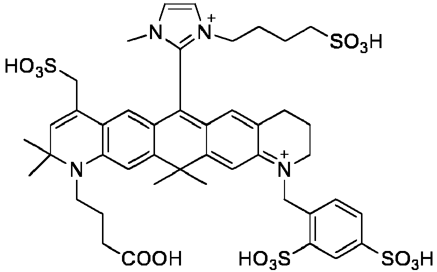 | 725^{b} | 749^{b} |
| **58** | 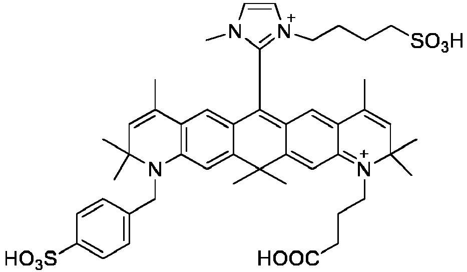 | 742^{b} | 775^{b} |
| **59** | 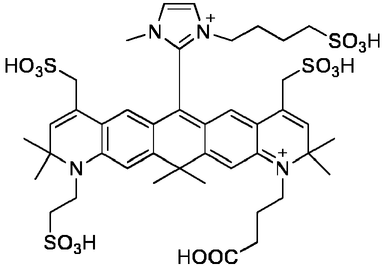 | 739^{b} | 771^{b} |
| **60** | 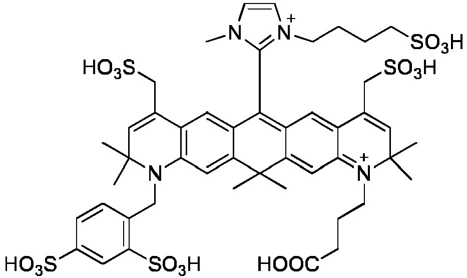 | 742^{b} | 775^{b} |
| **61** | 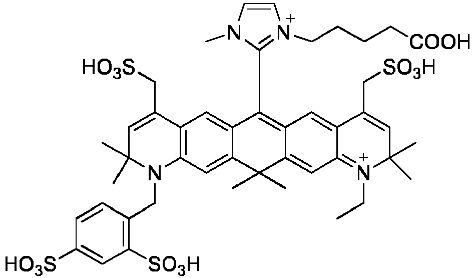 | 743^{b} | 776^{b} |
| **62** | 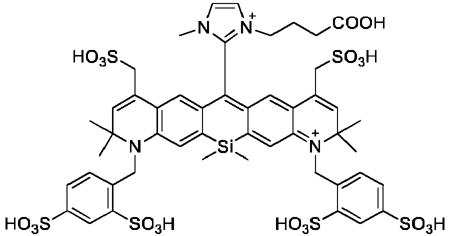 | 791^{b} | 817^{b} |
| **63** | 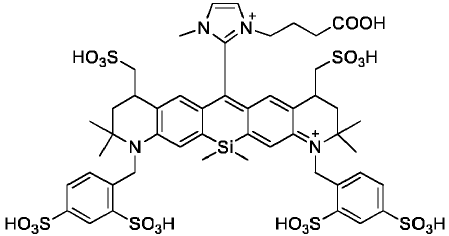 | 752^{b} | 776^{b} |
| **64** | 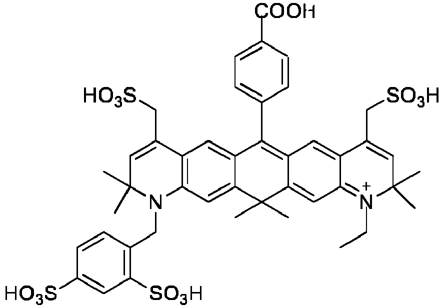 | 667^{b} | 697^{b} |
| **65** | 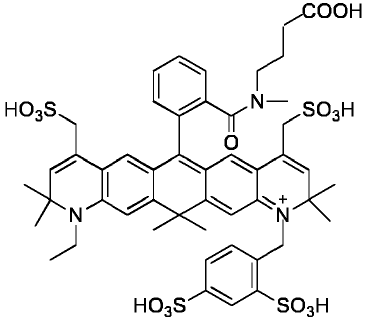 | 679^{b} | 706^{b} |
| **66** | 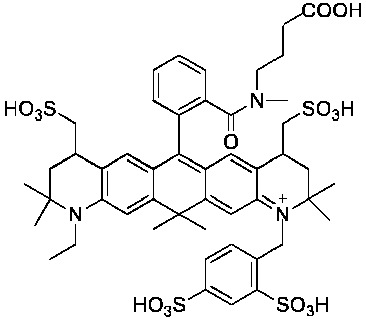 | 645^{b} | 665^{b} |
| **67** | 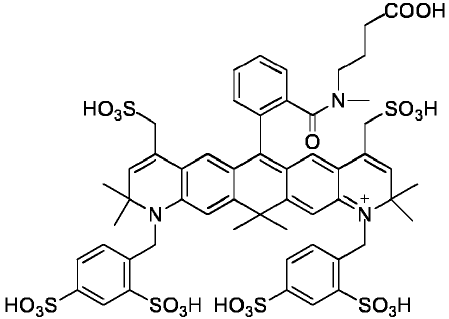 | 679^{b} | 707^{b} |
| **68** | 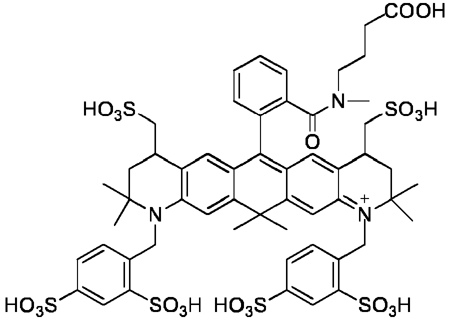 | 645^{b} | 666^{b} |
| **69** | 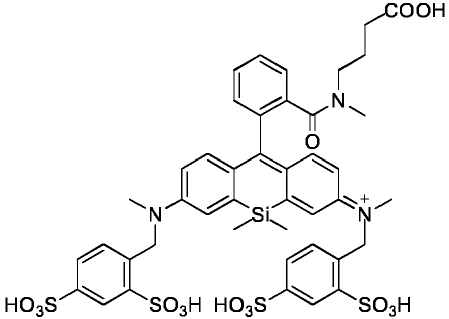 | 658^{b} | 680^{b} |
| **70** | 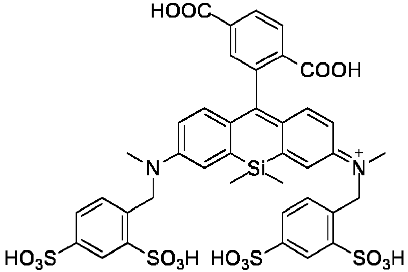 | 649^{b} | 670^{b} |
| **71** | 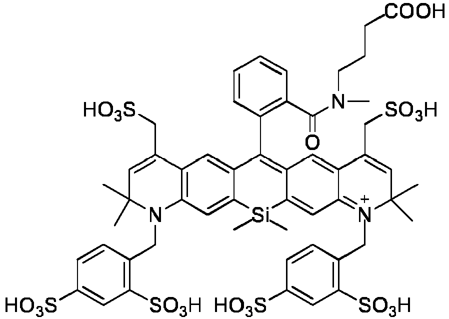 | 722^{b} | 752^{b} |
| **72** | 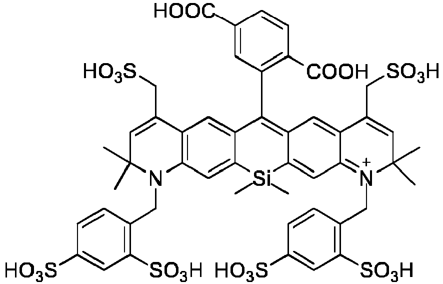 | 711^{b} | 740^{b} |
| **73** | 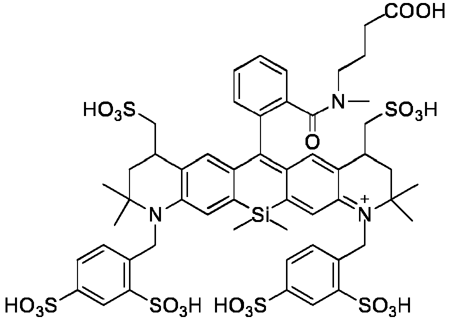 | 686^{b} | 708^{b} |
| **74** | 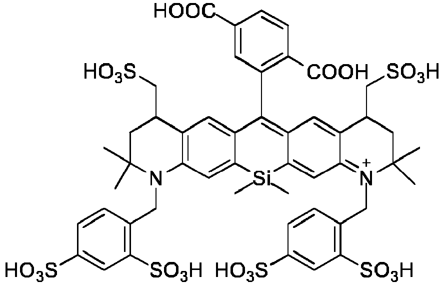 | 675^{b} | 698^{b} |

**Tabelle 4: allgemeine Formel (II), Beispiele für X = C(CH₃)₂ (Carbopyronine), X = Si(CH₃)₂ (Silicopyronine)**

| Lösungsmittel: ^{a} Ethanol, ^{b} PBS pH 7,4, ^{c} Wasser/Acetonitril 1:1 | | | |
|---|---|---|---|
| **Verbindung** | **Struktur** | **λ_{abs} [nm]** | **λ_{fl} [nm]** |
| **75** | 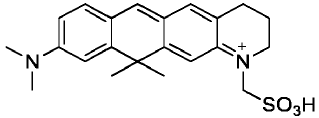 | 597^{a} | 621^{a} |
| **76** | 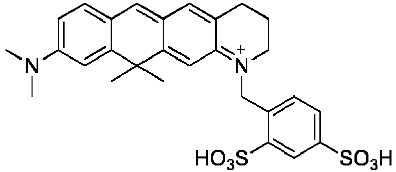 | 596^{b} | 622^{b} |
| **77** | 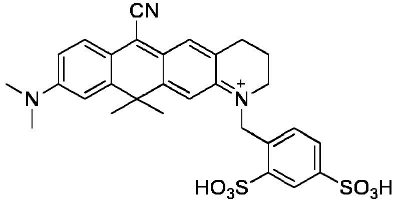 | 730^{b} | 751^{b} |
| **78** | 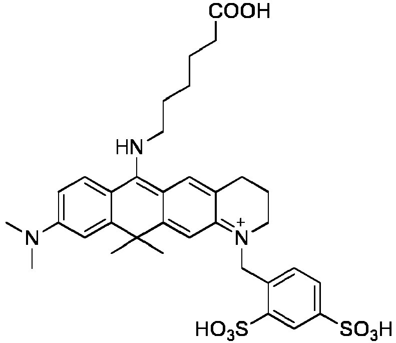 | 468^{a} | 609^{b} |
| | | 463^{b} | |
| **79** | 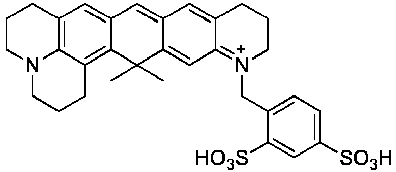 | 627^{b} | 649^{b} |
| **80** | 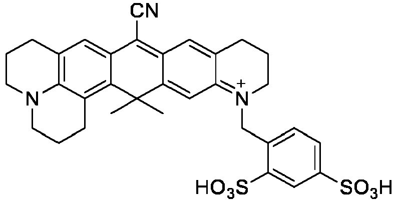 | 739^{b} | 761^{b} |
| **81** | 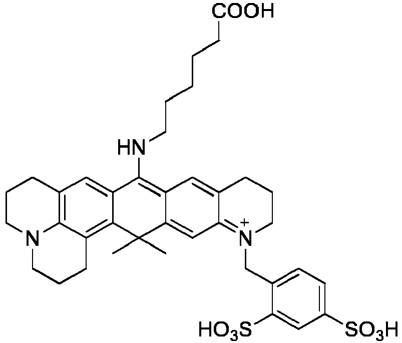 | 476^{b} | 614^{b} |
| **82** | 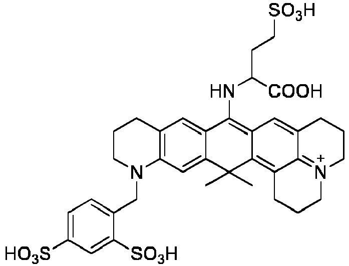 | | |
| **83** | 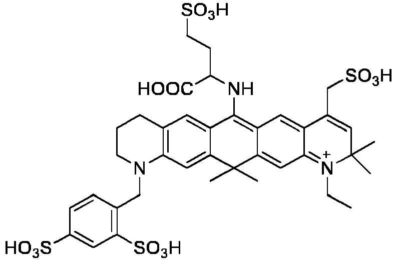 | | |
| **84** | 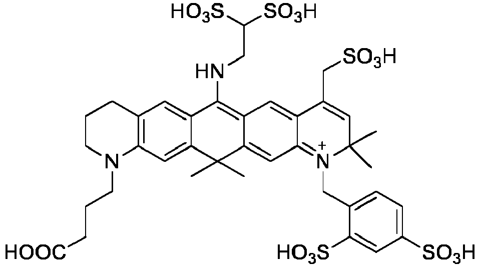 | 490^{b} | 644^{b} |
| **85** | 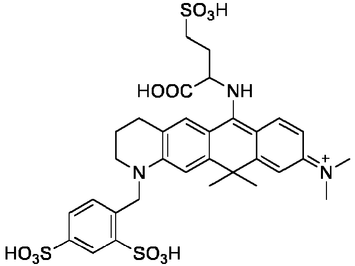 | 496^{b} | 673^{b} |
| **86** | 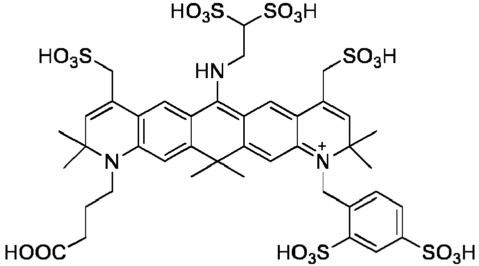 | 502^{b} | 660^{b} |
| **87** | 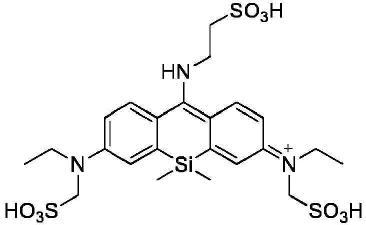 | 472^{b} | 605^{b} |
| **88** | 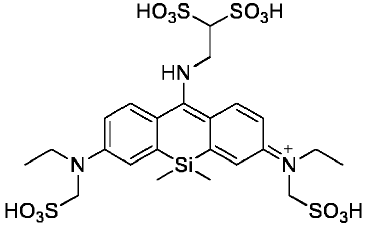 | 471^{b} | 605^{b} |
| **89** | 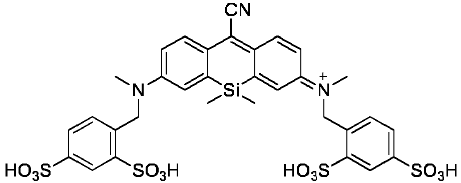 | 746^{b} | 763^{b} |
| **90** | 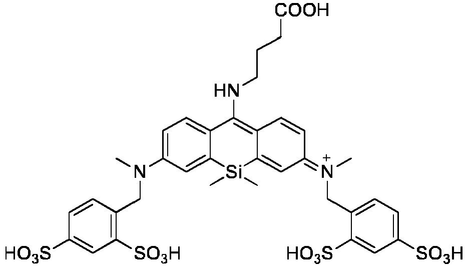 | 483^{b} | 637^{b} |
| **91** | 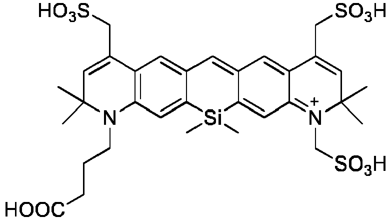 | 715^{b} | 738^{b} |
| **92** | 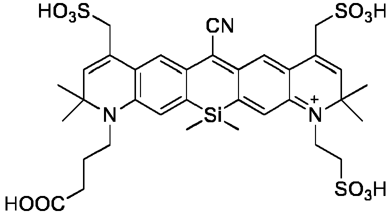 | 817^{b} | 841^{b} |
| **93** | 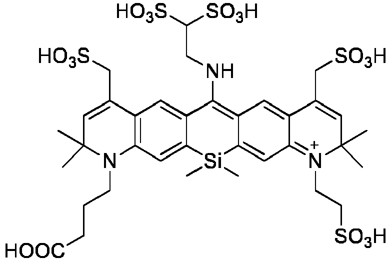 | 516^{b} | 695^{b} |
| **94** | 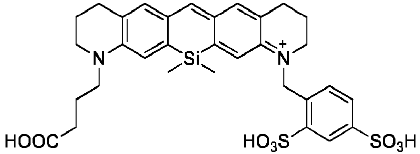 | 670^{b} | 693^{b} |

| **Verbindung** | **Struktur** | **λ_{abs} [nm]** | **λ_{fl} [nm]** |
|---|---|---|---|
| **95** | 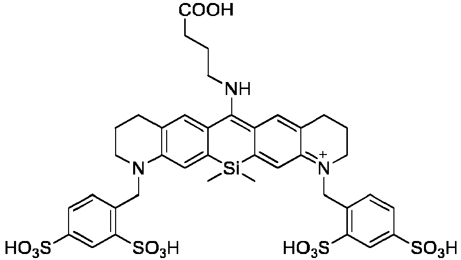 | 489^{b} | 661^{b} |
| **96** | 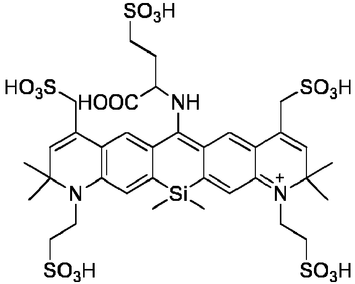 | 518^{b} | 695^{b} |

**Tabelle 5: allgemeine Formel (III), Beispiele für X = O (Oxazine), X = C(CH₃)₂ (Carbazine)**

| Lösungsmittel: ^{a} Ethanol, ^{b} PBS pH 7,4, ^{c} Wasser/Acetonitril 1:1 | | | |
|---|---|---|---|
| **Verbindung** | **Struktur** | **λ_{abs} [nm]** | **λ_{fl} [nm]** |
| **97** | 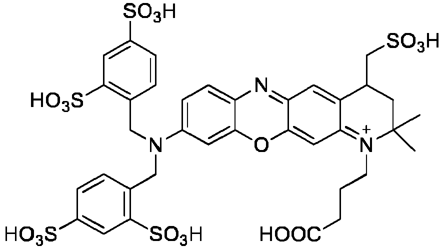 | 649^{b} | 667^{b} |
| **98** | 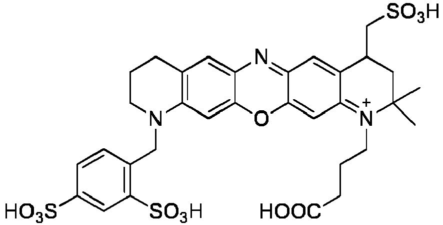 | 664^{b} | 682^{b} |
| **99** | 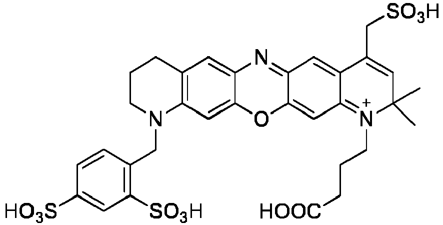 | 682^{b} | 700^{b} |
| **100** | 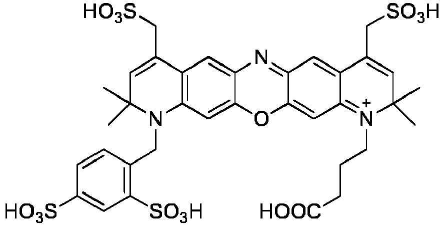 | 701^{b} | 718^{b} |
| **101** | 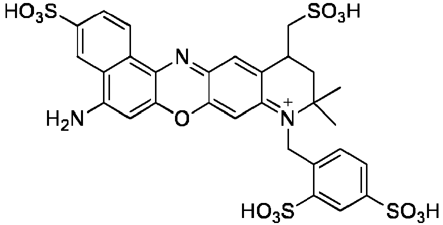 | 647^{b} | 678^{b} |
| **102** | 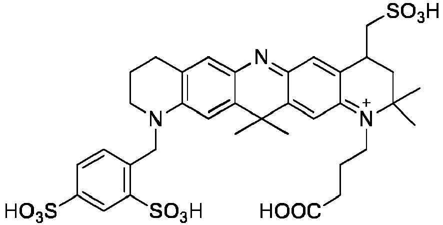 | 721^{b} | 742^{b} |
| **103** | 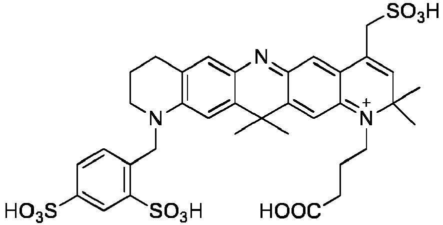 | 740^{b} | 761^{b} |
| **104** | 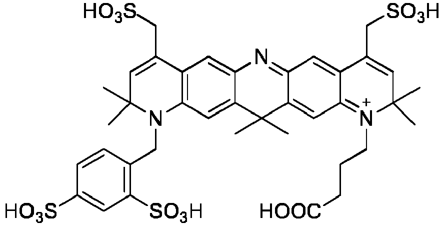 | 758^{b} | 778^{b} |

**Tabelle 6: allgemeine Formel (IV), Beispiele für X = O (Cumarine), X = NR (Carbostyrile)**

| Lösungsmittel: ^{a} Ethanol, ^{b} PBS pH 7,4, ^{c} Wasser/Acetonitril 1:1 + 0,1 Vol.-% Trifluoressigsäure | | | |
|---|---|---|---|
| **Verbindung** | **Struktur** | **λ_{abs} [nm]** | **λ_{fl} [nm]** |
| **105** | 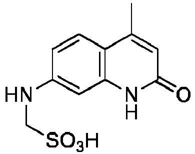 | 355^{a} 349^{b} | 406^{a} 413^{b} |
| **106** | 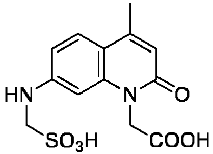 | 350^{b} | 410^{b} |
| **107** | 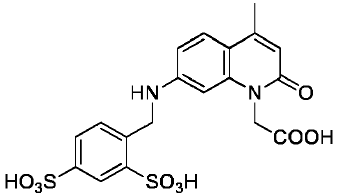 | 351^{b} | 413^{b} |
| **108** | 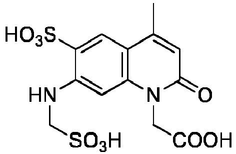 | 348^{b} | 411^{b} |
| **109** | 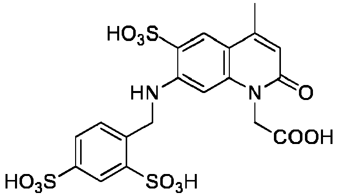 | 349^{b} | 418^{b} |
| **110** | 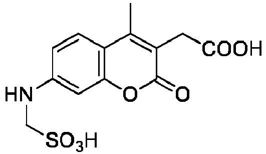 | 354^{b} | 353^{b} |
| **111** | 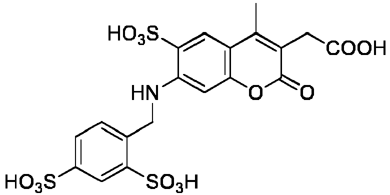 | 348^{b} | 449^{b} |
| **112** | 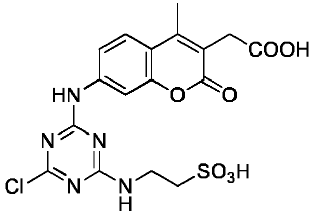 | 338^{b} | 416^{b} |
| **113** | 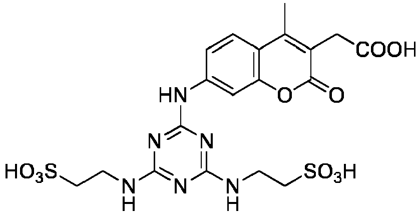 | 336^{b} | 423^{b} |
| **114** | 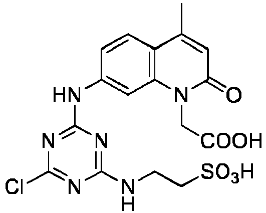 | 338^{b} | 385^{b} |
| **115** | 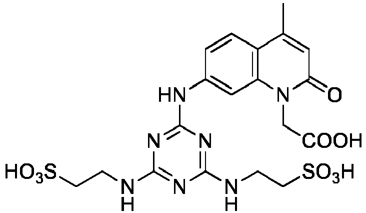 | 335^{b} | 383^{b} |

In einer besonders bevorzugten Ausführungsform weisen Verbindungen der Formel (I) an den Positionen R₄ und/oder R₇ einen Rest -SO₃H auf.
Eine besondere Eigenschaft der Rhodamine, die - wie in den Verbindungen 6, 10, 11, 12, 19, 20, 25 und 27 gezeigt - mit Sulfonsäuregruppen an den Positionen R₄ und R₇ direkt am Chromophor substituiert sind, ist die besonders leichte Lakton- oder Pseudobasenbildung in wässriger schwach alkalischer Lösung. Normalerweise werden solche Laktone nur in aprotischen Lösungsmitteln wie Aceton oder Chloroform genügend stabilisiert. In protischen Lösungsmitteln wird die Carboxylgruppe deprotoniert und es bildet sich ein geringfügig kurzwelliger absorbierendes und fluoreszierendes Zwitterion. Bei den beispielhaften Verbindungen entstehen jedoch aufgrund des starken Elektronenzugs der genannten Substituenten durch innere Esterbildung und der damit verbundenen Unterbrechung des konjugierten Systems farblose Verbindungen. Wenn die Carboxylgruppe nicht frei sondern als Ester oder tertiäres Amid vorliegt, können durch den Angriff von Nucleophilen (Hydroxid-Ionen etc.) am durch die elektronenziehenden Substituenten stärker positivierten zentralen Kohlenstoffatom so genannte Pseudobasen entstehen, die wegen der Unterbrechung des konjugierten Systems ebenfalls farblos sind. Beide Reaktionen sind reversibel: durch die Veränderung des pH-Werts, der Dielektrizitätskonstante ("Polarität") und/oder der Fähigkeit zur Wasserstoff-Brückenbindung ("Protizität") kann wieder der Farbstoff zurückgebildet werden. Daher können solche Verbindungen prinzipiell als Sonden für Eigenschaften ihrer direkten Umgebung verwendet werden: Je nach Milieu liegen die Moleküle in der farblosen oder farbigen Form vor. Damit verbunden ist auch eine Art der Fluorogenität, denn nur in der Farbstoffform erscheint die langwellige Fluoreszenz.

Die Fluorogenität von Verbindungen gewinnt zunehmend an Bedeutung für moderne bildgebende Verfahren wie das Fluoreszenzimaging von lebenden Zellen oder als molekulare Schalter in Mikroskopie- und Nanoskopieverfahren, z.B. STED, PALM, (d)STORM etc. Es werden hierfür sogar neue Silico-Rhodamine entwickelt, siehe z.B. G. Lukinavicius, K. Umezawa et al. (2013), A nearinfrared fluorophore for live-cell super-resolution microscopy for cellular proteins, Nature Chemistry 5, 132-139. Aber auch herkömmliche Chromophore werden für diese Art der Anwendung weiter modifiziert, siehe z.B. J. B. Grimm, A. J. Sung et al. (2013), Carbofluoresceins and Carborhodamines as Scaffolds for High-Contrast Fluorogenic Probes, ACS Chem. Biol. 8, 1303-1310.

Die genannte Untergruppe der hier vorgestellten, neuen und erfindungsgemäßen Farbstoffe lässt sich daher prinzipiell auch in solchen Verfahren verwenden und sollte bedeutende Fortschritte ermöglichen.

Eine spezielle Untergruppe der erfindungsgemäßen Verbindungen sind Fluoreszenzquencher. Diese Farbstoffe fluoreszieren selber nicht, können aber als Akzeptoren für die Emission fluoreszierender Verbindungen wirken. Ein weit verbreitetes Einsatzgebiet solcher Quencher ist etwa die Messung der Geometrie (Abstände, Größe und Anordnung von Molekülgruppen) oder der Reaktivität (Enzymaktivität, Reaktionsgeschwindigkeiten etc.) mithilfe der Förster-Energieübertragung (FRET). Die Fluoreszenz des Donors kann nur dann in Erscheinung treten, wenn der Abstand zwischen Donor und Akzeptor einen kritischen Wert überschreitet. Unterhalb dieser Distanz wird die Fluoreszenz des Donors durch den - selber nicht fluoreszierenden - Akzeptor (= Quencher) gelöscht. Einen Überblick über bioanalytische Anwendungen der FRET-Technik liefert z.B. A. Roda, M. Guardigli, E. Micheline, M. Mirasoli, Nanobioanalytical luminescence: Förster-type energy transfer methods, Anal. Bioanal. Chem. 393, 2009, 109-123.

Beispiele für erfindungsgemäße Quencher sind die Verbindungen 14, 15, 16 und 17. Es handelt sich bei diesen Rhodaminen um eine Weiterentwicklung der aufgrund ihrer magentafarbenen Lösungen als "Violamine" bezeichneten Farbstoffe, vgl. z.B. H.E. Fierz-David, Künstliche Organische Farbstoffe, Springer-Verlag, Berlin, 1926, S. 276ff. Aber auch bei anderen Farbstoffklassen lässt sich auf diese Weise eine Modifizierung der Struktur vornehmen. Erfindungsgemäße Beispiele sind hier die Verbindungen 49, 50 und 51 aus der Klasse der Carbopyronine.

Erfindungsgemäße Fluorezenzquencher weisen insbesondere eine Gruppe B mit der Struktur 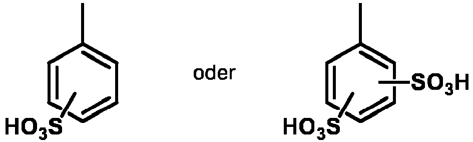 auf und insbesondere wenigstens eine Gruppe B, ausgewählt aus

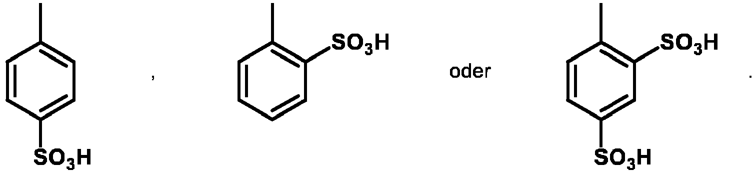

Der aromatische Ring der Gruppe B ist bei Verbindungen mit Fluoreszenzquencher-Eigenschaften somit direkt an das Stickstoffatom gebunden.

Ein wichtiger Vorzug der vorliegenden Erfindung besteht darin, dass die neuen Verbindungen der allgemeinen Formeln (I) - (IV), die insbesondere zur Verwendung als Markierungsgruppe in Nachweisverfahren von Analyten geeignet sind, mit einfachen und kostengünstigen Verfahren hergestellt werden können und problemlos handhabbar sind.
Ein wesentlicher Vorteil der Erfindung besteht also in der kommerziellen Verfügbarkeit bzw. der kostengünstigen Herstellung von Ausgangsfarbstoffen nach dem Fachmann bekannten Methoden. Viele bekannte, gut fluoreszierende und photostabile Farbstoffe aus den Klassen der Cumarine, Xanthene, Carbopyronine, Oxazine etc. können nämlich in einfachen chemischen Reaktionen zu erfindungsgemäßen Verbindungen umgesetzt werden. Damit ist einerseits das komplette sichtbare Spektrum abgedeckt, da der Grundchromophor in weiten Grenzen frei gewählt werden kann. Durch eine fast beliebige Substituentenvariation können andererseits die Lage der Absorptions- und Fluoreszenzmaxima, Fluoreszenzquantenausbeute und Abklingzeit stark variiert und somit wie gewünscht ausgewählt werden. So können Interferenzen mit Störsubstanzen in Proben vermindert oder sogar ganz vermieden werden.

Die Herstellung der Verbindungen der Formeln (I) - (IV) erfolgt nach den in den Beispielen genauer beschriebenen Verfahren.

Vor allem im Falle der Rhodamine ist die Herstellung besonders einfach und kostengünstig: Kommerziell erhältliche oder nach bekannten Verfahren leicht herzustellende Rhodamine werden in die Leukoform überführt. Die Leukoform wird mit einem mehrfach sulfonierten Aryl-alkylhalogenid an den Aminogruppen alkyliert und anschließend zum erfindungsgemäßen Farbstoff oxidiert (siehe Beispiel: Verbindung 6).
Andererseits können erfindungsgemäße Rhodamine aus käuflichem 3',6'-Dichlorfluoran z.B. durch Umsetzung mit einem mehrfach sulfonierten Benzylamin leicht in bekannter Weise hergestellt werden (siehe Beispiel: Verbindung 18).
In diesen beiden Verfahren wird die Gruppierung B nachträglich in den Farbstoff eingeführt. Diese Methode ist nicht auf Rhodamine beschränkt, sondern kann auch bei anderen Vertretern der allgemeinen Formeln (I) - (IV) angewendet werden.

Es ist aber auch möglich die erfindungsgemäßen Verbindungen der Formeln (I) - (IV) durch Synthese des Ringsystems unter Verwendung von Bausteinen, die bereits die Gruppierung B enthalten herzustellen. Eine solche Vorgehensweise ist für die Verbindungen 102 und 103 nachfolgend beispielhaft dargestellt.

**Beispiele** für die Darstellung von Vorstufen.

### 2-(Brommethyl)-benzolsulfonsäure

### 1. Stufe: 2-(Hydroxymethyl)-benzolsulfonsäure

25 g (120 mmol) 2-Formyl-benzolsulfonsäure Natriumsalz (M = 208.17 g/mol) werden in 400 ml Methanol suspendiert, portionsweise mit 3.63 g (96 mmol) Natriumborhydrid versetzt und bei Raumtemperatur verrührt. Die Reaktion wird dünnschichtchromatographisch verfolgt (Wasser/Acetonitril 1:9). Nach ca. 90 Minuten wird abgesaugt und das Filtrat zur Trockne eingeengt. Für eine vollständige Trocknung wird der Rückstand mit Dichlormethan behandelt und erneut eingeengt. Dieser Vorgang wird solange wiederholt, bis ein fast farbloses Pulver resultiert. Ausbeute: 100 %;
NMR in D₂O: 7.8 ppm (1H, DvD); 7.5 ppm (1 H, DvD); 7.4 ppm (1H, DvT); 7.3 ppm (1H, DvT); 4.9 ppm (2 H, S)

### 2. Stufe: 2-(Brommethyl)-benzolsulfonsäure Natriumsalz (M = 273,08 g/mol)

21 g (100 mmol) 2-(Hydroxymethyl)-benzolsulfonsäure Natriumsalz (M = 210.19 g/mol) werden mit 9.6 g Natriumbromid und 240 ml 48% Bromwasserstoffsäure auf dem Ölbad bei ca. 80 °C für 10 Stunden verrührt. Die Reaktion wird dünnschichtchromatographisch verfolgt (siehe oben).

Nach dem Abkühlen wird zur Trockne eingeengt und der hellgelbe Rückstand für 30 Minuten in 400 ml Aceton zum Rückfluss erhitzt. Nach dem Abkühlen wird abgesaugt und der Rückstand mit Aceton gewaschen.

Das Filtrat wird eingeengt und der resultierende zähe Brei mit 300 ml Diethylether verrührt und abgesaugt. Es wird solange mit Diethylether gewaschen, bis ein hellbeiger Feststoff resultiert, der an der Luft getrocknet wird. Ausbeute: 64 %;
NMR in D₂O: 7.9 ppm (1H, DvD); 7.6 ppm (1 H, DvD); 7.5 ppm (1H, DvT); 7.4 ppm (1H, DvT); 5.0 ppm (2 H, S)

### 4-(Brommethyl)-benzol-1,3-disulfonsäure

*Herstellung gemäß* DE60105258T2*.*

### 1. Stufe: 4-(Hydroxymethyl)-benzol-1,3-disulfonsäure

15 g (48 mmol) 4-Formyl-benzol-1,3-disulfonsäure Dinatriumsalz (M = 310.21 g/mol als Hydrat) werden in 400 ml Methanol suspendiert, portionsweise mit 1.5 g (40 mmol) Natriumborhydrid versetzt und bei Raumtemperatur ca. 90 Minuten verrührt. Die Reaktion wird dünnschichtchromatographisch verfolgt (Wasser/Acetonitril 1:9). Die Reaktionsmischung wird zur Trockne eingeengt. Es resultiert ein farbloses Pulver. Ausbeute: 100 %;
NMR in D₂O: 8.1 ppm (1H, D); 7.9 ppm (1 H, DvD);7.7 ppm (1 H, D); 4.9 ppm (2 H, S)

### 2. Stufe: 4-(Brommethyl)-benzol-1,3-disulfonsäure

12 g (38 mmol) 4-(Hydroxymethyl)-benzol-1,3-disulfonsäure Dinatriumsalz (M = 312.25 g/mol) werden mit 4.5 g Lithiumbromid und 100 ml 48% Bromwasserstoffsäure auf dem Ölbad bei 80 °C für 10 Stunden verrührt. Die Reaktion wird dünnschichtchromatographisch verfolgt (siehe oben).

Nach dem Abkühlen wird zur Trockne eingeengt und der hellgelbe Rückstand für 30 Minuten in 400 ml Aceton zum Rückfluss erhitzt. Nach dem Abkühlen wird abgesaugt und der Feststoff mit Aceton gewaschen. Der Feststoff wird im Exsikkator getrocknet. Ausbeute: 82 %;
NMR in D₂O: 8.2 ppm (1H, D); 7.9 ppm (1 H, DvD);7.7 ppm (1 H, D); 4.95 ppm (2 H, S)

### 4-(Bromethyl)-benzol-1,3-disulfonsäure

*Hergestellt gemäß* Makarova et al., Polyelectrolytes from vinylaromatic monomers which contain sulfo groups, Chimiceskie reaktivy /preparaty 33 (1971), 22-29.

50 ml 2-Bromethylbenzol werden unter Eiskühlung zu 50 ml konzentrierter Schwefelsäure getropft. Anschließend tropft man 150 ml gekühltes 65 % Oleum hinzu, so dass die Innentemperatur unter 50 °C bleibt. Danach wird für zwei Stunden auf 80°C erhitzt.

Die etwas abgekühlte Reaktionsmischung wird auf 500 ml Eis gegossen und mit festem Bariumcarbonat auf pH 9 eingestellt. Es wird vom entstandenen Bariumsulfat abgesaugt und dieses einmal mit kaltem Wasser gewaschen. Das Filtrat wird eingeengt, wobei ein gelblicher Substanzbrei erhalten wird. Dieser wird mit Methanol verrührt, abgesaugt und getrocknet.

### 4-(Bromethyl)-benzolsulfonsäure

*Hergestellt gemäß* DE3023112A1 *aus 2-Bromethylbenzol in 30* % *Oleum und Chlorsulfonsäure. Das entstandene Sulfonylchorid wird in Wasser*/*Acetonitril hydrolysiert.*

42 g 30 % Oleum werden in einem Rundkolben vorgelegt und im Eisbad gekühlt. 29 g 2-Bromethylbenzol werden in 13 g Acetonitril gelöst und so zum Oleum getropft, dass die Temperatur unterhalb 25 °C bleibt. Anschließend wird im Ölbad auf 40 - 60°C erwärmt und bei dieser Temperatur 46 g Chlorsulfonsäure zugetropft. Es wird danach noch eine Stunde auf 100 - 120°C erhitzt.

Die Reaktionsmischung wird auf 500 ml Eis gegossen, der entstandene Niederschlag abgesaugt, dreimal mit eiskaltem Wasser gewaschen und getrocknet. Man erhält 36 g Rohprodukt.

Zur Reinigung wird aus 400 ml Heptan umkristallisiert. Dabei wird vom braunen öligen Bodensatz abdekantiert. Aus dem Überstand fallen im Eisbad farblose Kristalle des gewünschten Produkts aus. Man erhält nach dem Trocknen 20 g.

Zur Hydrolyse des Sulfochlorids wird dieses in einer Mischung aus Wasser und Acetonitril solange zum Rückfluss erhitzt, bis die Reaktion vollständig abgelaufen ist (DC-Kontrolle). Die stark saure Lösung wird mit Natronlauge auf pH 6.5 eingestellt und nach dem Einengen verbleiben 22.7 g 4-(Bromethyl)-benzolsulfonsäure-Natriumsalz (vermischt mit entstehendem Natriumchlorid).

Diese Substanz wird sowohl als Sulfonsäure bzw. Natriumsulfonat kommerziell angeboten.

### 4-(Aminoethyl)-benzolsulfonsäure

50 ml konzentrierte Schwefelsäure werden im Eisbad auf 0 °C gekühlt. Dazu tropft man 50 ml 2-Phenethylamin, so dass die Innentemperatur nicht über 40°C ansteigt. Danach werden 150 ml 30 % Oleum zugetropft, so dass die Temperatur nicht über 70°C ansteigt. Nach dem Ende der Zugabe wird für 30 Minuten auf 80 °C erhitzt. Die Reaktionsmischung wird danach in 1.5 I einer Mischung aus Diethylether und THF (1:1) getropft, wobei das Produkt als farblose Substanz ausfällt. Es wird abgesaugt und getrocknet. Man erhält 71 g.

### Beispiele für die Darstellung von erfindungsgemäßen Verbindungen.

### Verbindung 6: Alkylierung eines Rhodamins mit 4-(Brommethyl)-benzol-1,3-disulfonsäure.

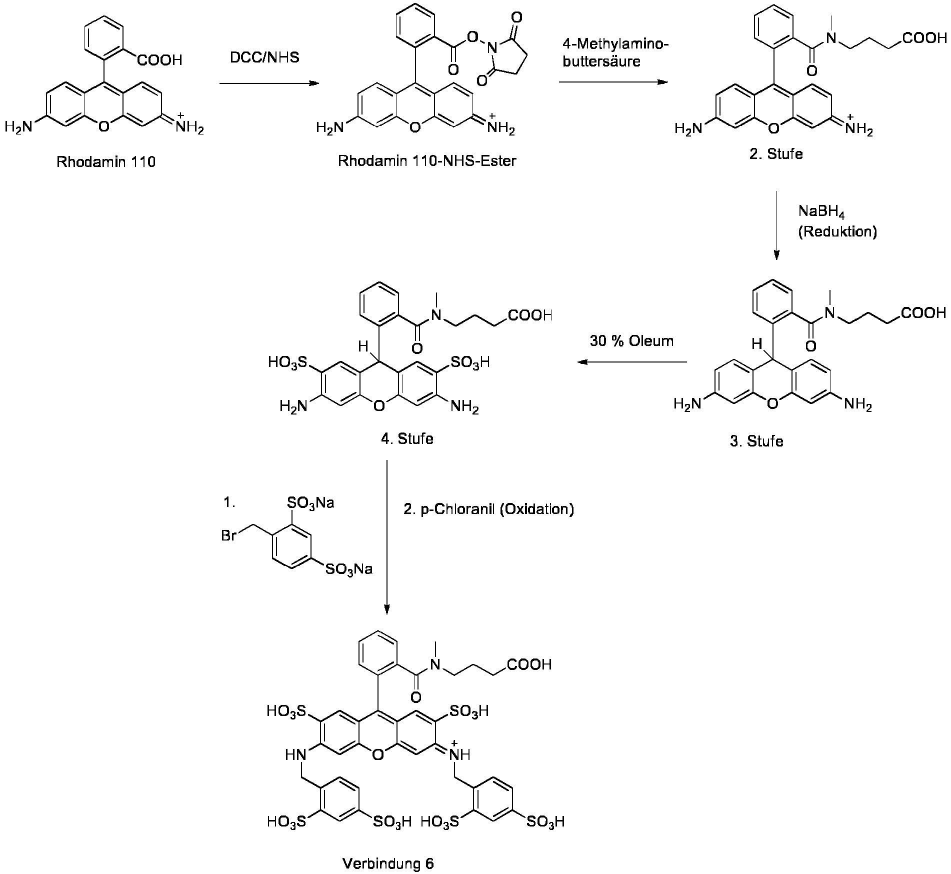

1. und 2. Stufe: Rhodamin 110-Chlorid wird aktiviert und mit 4-Methylaminobuttersäure umgesetzt (siehe EP1576059B1).
3. Stufe: Der Farbstoff der 2. Stufe wird wie bei B.M. Kinsey, A.I. Kassis et al, Synthesis and Biological Studies of lodinated (127/125I) Derivatives of Rhodamine 123, J. Med. Chem. 30, 1987, 1757-1761 beschrieben reduziert.
4. Stufe: Der erhaltene Feststoff der 3. Stufe wird in 30 % Oleum nach der bekannten Vorschrift (siehe US4345017A) sulfoniert.
5. Stufe, Verbindung 6: Das isolierte Sulfonierungsprodukt wird mit 700 mg 4-(Brommethyl)-benzol-1,3-disulfonsäure Dinatriumsalz, 160 mg Kaliumiodid und 130 mg Kaliumcarbonat in ca. 25 ml Dimethylformamid (DMF) suspendiert und für 30 - 60 Minuten bei 100 °C auf dem Ölbad verrührt. Die trübe Lösung färbt sich rötlich.

Die Reaktionsmischung wird in 500 ml Aceton eingerührt, der sich bildende Niederschlag filtriert und zweimal mit Aceton gewaschen. Der Filterrückstand wird in 200 ml Wasser aufgelöst, mit 100 ml Aceton und 200 mg p-Chloranil versetzt und bei Raumtemperatur für anderthalb Stunden verrührt, wobei der Oxidationsvorgang dünnschichtchromatographisch verfolgt wird. Die Reinigung erfolgt anschließend chromatographisch.

### Verbindung 7 (Verbindung 6-NHS-Ester)

100 mg Verbindung 6 werden in 10 ml DMF gelöst, mit 35 mg TSTU und 100 µl Hünigbase versetzt und bei Raumtemperatur für 6 Stunden verrührt. Der NHS-Ester wird durch Eintropfen der Reaktionsmischung in 100 ml Diethylether gefällt. Der ausgefallene Niederschlag wird abgesaugt, mit Diethylether gewaschen und im Vakuumexsikkator über Phosphorpentoxid getrocknet.

### Verbindung 8 (Verbindung 6-Streptavidin-Konjugat)

2 mg Streptavidin (IBA, Gehalt ca. 75 %) werden in einem Glasvial mit 1 ml PBS-Puffer (pH 7.4) und 50 µl einer 0.2 M Natriumhydrogencarbonatlösung, die mit Natronlauge auf pH 9 eingestellt wurde, unter vorsichtigem Schwenken aufgelöst. Die klare Lösung hat einen pH-Wert von 8.3.

Es werden 40.5 µl einer Lösung von 170 µg Verbindung 7 in 50 µl trockenem DMSO hinzugefügt; dies entspricht einem Verhältnis Farbstoff:Streptavidin von 4:1. Die Reaktionslösung wird zwei Stunden bei Raumtemperatur und anschließend über Nacht im Kühlschrank bei 4 °C aufbewahrt.

Die Reinigung erfolgt in der üblichen Weise über Sephadex G25 superfein (Säulenlänge 40 cm, Durchmesser 2,5 cm) mit PBS-Puffer als Eluent. Die erste farbige Zone enthält das Steptavidin-Konjugat. Aus dem Absorptionsspektrum der Konjugat-Fraktion lässt sich nach dem bekannten Verfahren das DOL berechnen.

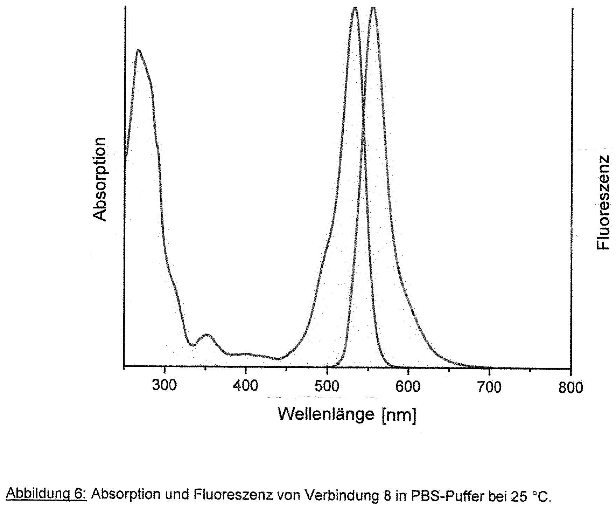

Absorptions- und Fluoreszenzspektum des Streptavidin-Konjugats sind in Abb. 6 dargestellt. Im Vergleich zu Abb. 5 (Verbindung 6) erkennt man im Absorptionsspektrum die im Konjugat durch das Streptavidin bei 280 nm hergerufene Absorption.

### Verbindung 18: Herstellung eines Farbstoff aus Dichlorfluoran.

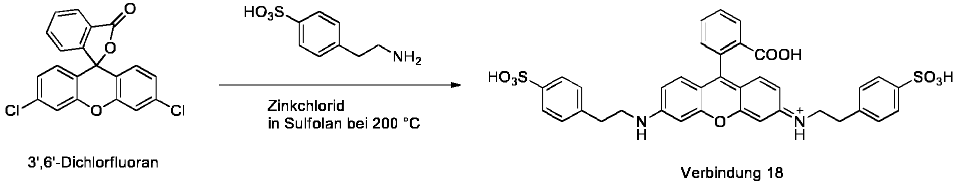

10 g 3',6'-Dichlorfluoran werden mit 15 g Zinkchlorid und 15 g 4-(Aminoethyl)-benzolsulfonsäure in 30 ml Sulfolan für 4 Stunden auf 200 °C erhitzt. Aus dem farblosen Reaktionsbrei entsteht dabei ein rotes Öl.

Nach dem Abkühlen wird mit einer Mischung aus Ethanol und Wasser (1:1) verrührt und abgesaugt. Das Filtrat (250 ml) wird in 1200 ml Aceton und 200 ml THF eingerührt, wobei sich das Produkt in roten Flocken abscheidet. Es wird abgesaugt, mehrmals mit Aceton gewaschen und im Vakuumexsikkator getrocknet. Die Reinigung erfolgt chromatographisch. Ausbeute: 40 %

### Verbindung 38: Herstellung eines arylsubstituierten Carbopyronins.

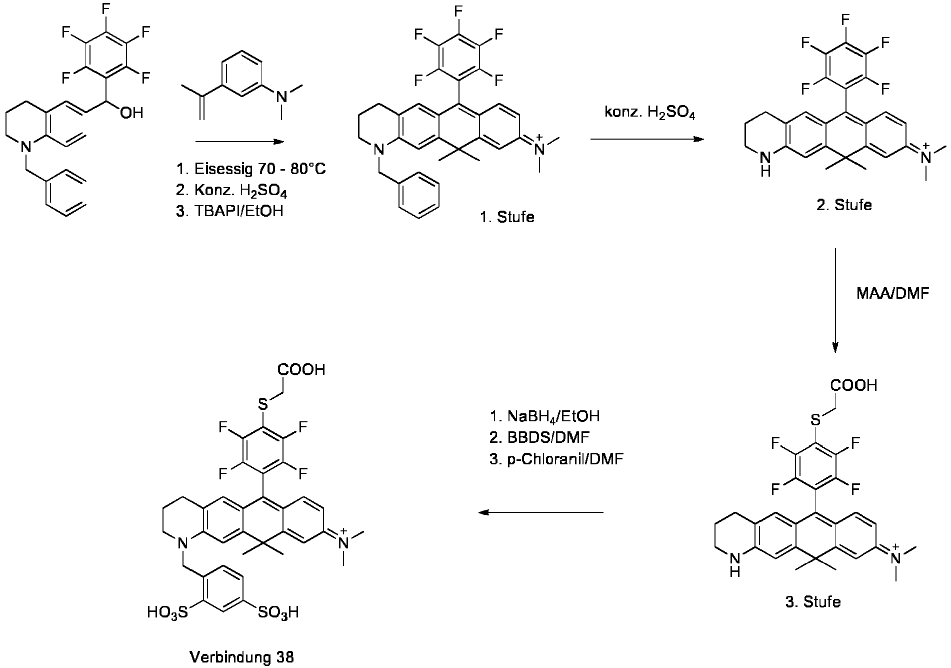

### 1. Stufe:

Der Farbstoff wird nach dem Verfahren zur Darstellung von Carbopyroninen (siehe EP1173519B) aus (1-Benzyl-1,2,3,4-tetrahydrochinolin-yl)(perfluorophenyl)methanol und N,N-Dimethyl-3-(prop-1-en-2-yl)anilin hergestellt.

### 2. Stufe:

2,6 g des Farbstoffs (1. Stufe) werden in 30 ml konz. Schwefelsäure gelöst und bei Raumtemperatur verrührt. Die Reaktion wird mittels HPLC verfolgt und ist nach 5 h beendet. Man tropft in 250 ml Ethanol, versetzt mit 350 ml Eiswasser und 100 ml 5 %-iger Natriumperchloratlösung. Der ausgefallene Niederschlag wird abgesaugt und im Vakuumexsikkator getrocknet. Der Farbstoff wird chromatographisch gereinigt. Ausbeute: 89 %; HPLC-MS: M⁺ = 471.19

### 3. Stufe:

2 g des Farbstoffs (2. Stufe) werden in 50 ml trockenem DMF gelöst und mit 7 eq. Mercaptoessigsäure (MAA) versetzt. Man erwärmt auf 85°C. Die Reaktion wird mittels HPLC verfolgt und ist nach 90 min beendet. Die Reaktionsmischung wird abgekühlt und in 450 ml 5 %-ige Natriumperchloratlösung getropft. Der ausgefallene Farbstoff wird abgesaugt, im Vakuum getrocknet und noch chromatographisch gereinigt. Ausbeute: 73 %; HPLC-MS: M⁺ = 543.17

### 4. Stufe, Verbindung 38:

500 mg des Farbstoffs (3. Stufe) werden in 30 ml trockenem Acetonitril gelöst und portionsweise unter Schutzgas mit 5 x 2 ml einer bei 40°C gesättigten Natriumborhydridlösung versetzt und 30 min verrührt. Die Reaktionslösung mit dem reduzierten Farbstoff wird bis zur Trockene eingeengt, der Rückstand in 30 ml trockenem DMF aufgenommen, mit 5 eq. 4-(Brommethyl)-benzol-1,3-disulfonsäure (BBDS) versetzt und auf 90°C erwärmt. Die Alkylierung wird mittels HPLC verfolgt und ist nach 1 h beendet. Die Reaktionslösung wird auf 50°C abgekühlt und mit 2,5 eq. p-Chloranil versetzt. Die Oxidation zum Farbstoff ist nach 30 min abgeschlossen. Die Reaktionslösung wird in 400 ml Diethylether getropft, der ausgefallene Farbstoff abgesaugt und das Rohprodukt chromatographisch gereinigt. Ausbeute: 79 %; HPLC-MS: M⁺ = 793.13

### Verbindung 55: Herstellung eines arylsubstituierten Carbopyronins.

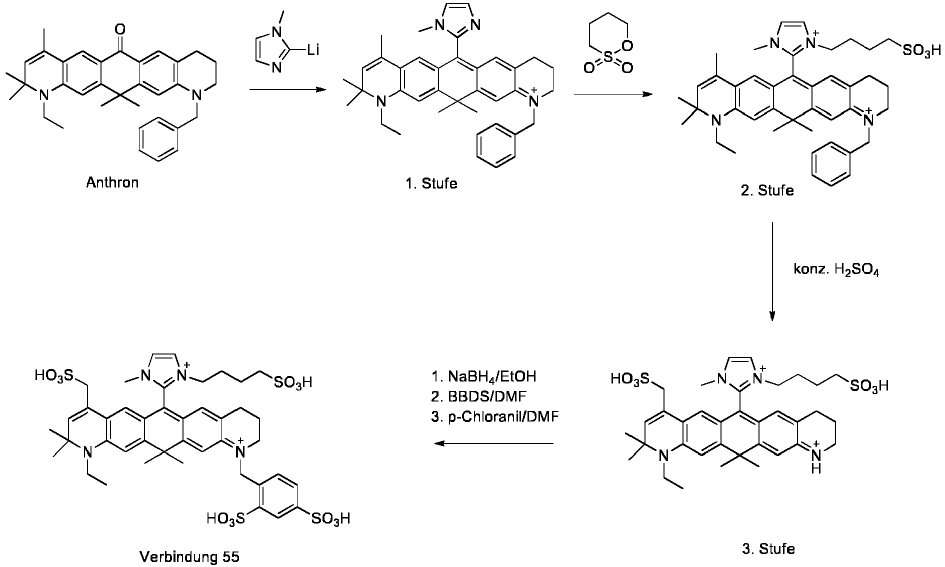

### 1. Stufe:

0,25 g 1-Methyl-1H-imidazol werden in 20 ml trockenem Tetrahydrofuran vorgelegt, unter Schutzgas bei -78°C tropfenweise mit 1 eq. n-Butyllithum versetzt und nach beendeter Zugabe 30 min verrührt. 150 mg des Anthrons (herstellt gemäß DE102010042634A1) werden in 10 ml trockenem Tetrahydrofuran gelöst und tropfenweise bei -78°C zur Reaktionsmischung gegeben. Man lässt die Reaktionslösung langsam auf Raumtemperatur erwärmen und rührt weitere 3 h bevor sie unter Rühren in 100 ml Ethanol + 2 ml Trifluoressigsäure eingebracht wird. Die Lösung wird zur Trockene eingeengt und der Farbstoff chromatographisch gereinigt. Ausbeute: 83 %; HPLC-MS: M⁺ = 555.35

### 2. Stufe:

100 mg des Farbstoffs (1. Stufe) werden in 4 ml Butansulton suspendiert und auf 100°C erhitzt. Die Reaktion wird mittels UV-VIS-Spektroskopie verfolgt und ist nach 2 h beendet. Die Reaktionsmischung wird in 50 ml Wasser:Acetonitril 800:200 gegeben und 30 min verrührt. Die Farbstofflösung wird chromatographisch gereinigt. Ausbeute: 85 %; HPLC-MS: M⁺ = 692.37

### 3. Stufe:

Die Darstellung erfolgt analog zur Synthese der 2. Stufe von Verbindung 38 mit einer Ausbeute von 74 %; HPLC-MS: M⁺ = 682.28

### 4. Stufe, Verbindung 55:

Die Darstellung erfolgt analog zur Synthese der Verbindung 38 mit einer Ausbeute von 79 %; HPLC-MS: M⁺ = 931.24

### Verbindungen 79, 80 und 81: Herstellung eines Carbopyronins.

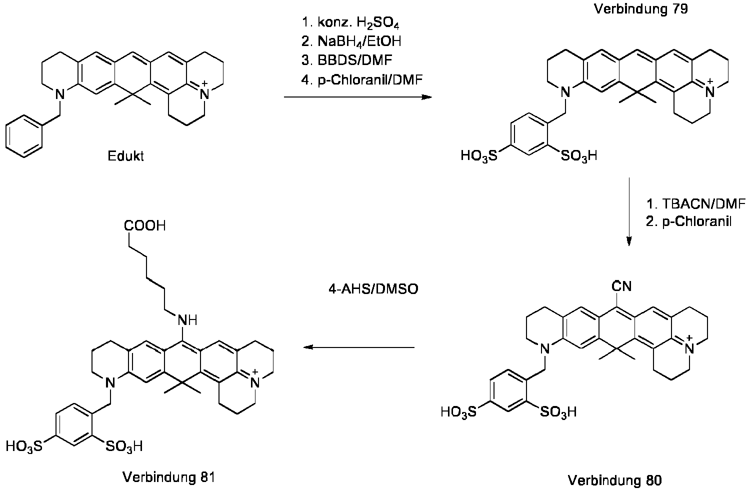

### 1. Stufe, Verbindung 79:

1 g Edukt (hergestellt gemäß DE102010042634A1) wird in 15 ml konz. Schwefelsäure gelöst und bei Raumtemperatur verrührt. Die Reaktion wird mittels HPLC verfolgt und ist nach 5 Stunden beendet. Die Reaktionslösung wird auf 400 ml Eiswasser gegeben, mit 200 ml Dichlormethan versetzt und mit Natronlauge auf pH 5 eingestellt. Man trennt die organische Phase ab und extrahiert mit Dichlormethan. Die vereinigten organischen Phasen werden über wasserfreiem Natriumsulfat getrocknet, filtriert und zur Trockene eingeengt. Der Rückstand wird in 100 ml Ethanol suspendiert und tropfenweise mit einer Lösung von 0,17 g Natriumborhydrid in 5 ml Ethanol versetzt. Die Reaktionsmischung wird 1 h bei Raumtemperatur gerührt. Der Farbstoff wird reduziert und geht vollständig in Lösung. Es wird zur Trockene eingeengt und der Rückstand unter Schutzgas in 20 ml DMF suspendiert. Man versetzt mit 2 eq. 4-(Brommethyl)benzol-1,3-disulfonsäure (BBDS) und erhitzt auf 100°C. Die Reaktion wird dünnschichtchromatographisch verfolgt und ist nach 40 min beendet. Es wird in 150 ml Ethanol getropft, mit 0,54 g p-Chloranil versetzt und auf 60°C erwärmt. Die Oxidation ist nach 20 Minuten abgeschlossen. Man engt ein und reinigt chromatographisch. Ausbeute: 63 %; HPLC-MS: M⁺ = 607.2

### 2. Stufe, Verbindung 80:

500 mg der Verbindung 79 werden wird in 40 ml DMF suspendiert und unter Schutzgas mit 1,5 eq. Tetrabutylammoniumcyanid versetzt. Es wird 1-2 Stunden bei Raumtemperatur gerührt und anschließend mit p-Chloranil oxidiert. Man tropft unter kräftigem Rühren in 300 ml Diethylether und dekantiert vom ausgeölten Farbstoff ab. Der Rückstand wird in 15 ml Wasser/Acetonitril 9:1 aufgenommen und chromatographisch gereinigt. Ausbeute: 60 %; HPLC-MS: M⁺ =632.2

### 3. Stufe, Verbindung 81:

200 mg der Verbindung 80 werden in 6 ml DMSO/Wasser 1:1 gelöst und mit 6 eq. 4-Aminohexansäure (4-AHS), gelöst in 2 ml DMSO/Wasser 7:3, versetzt. Es werden 6 eq. Diisopropylethylamin zugegeben, die Reaktionsmischung bei Raumtemperatur verrührt und die Reaktion mittels HPLC verfolgt. Es wird mit 35 ml Wasser verdünnt und chromatographisch gereinigt. Ausbeute: 67 %; HPLC-MS: M⁺ = 708.24

### Verbindungen 89 und 90: Herstellung eines Silicopyronins.

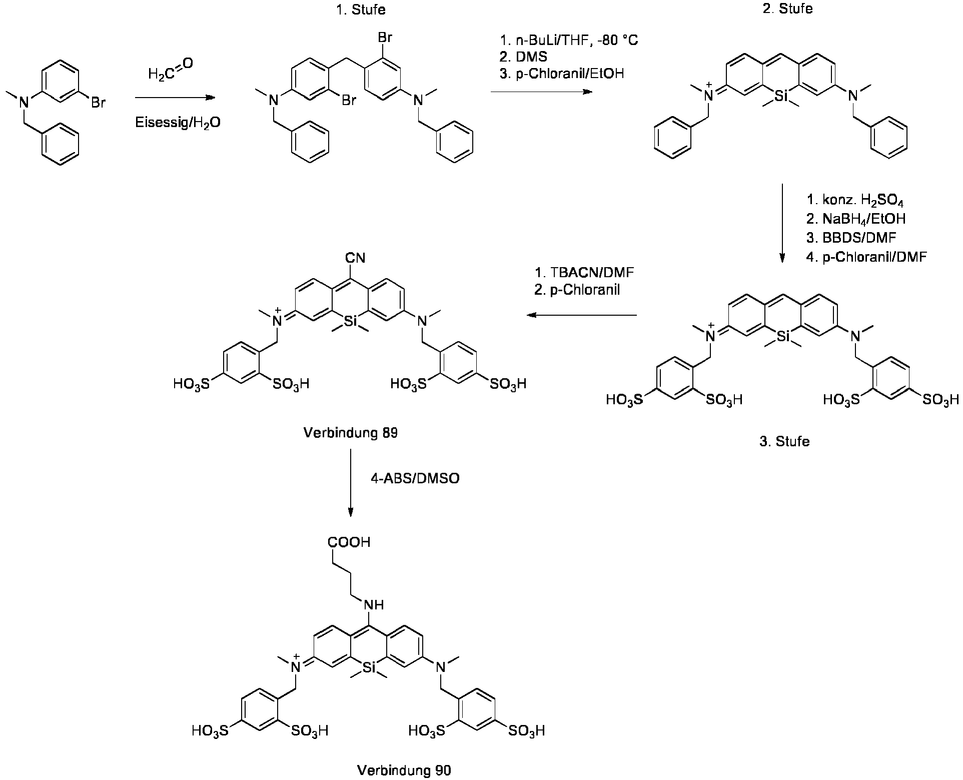

### 1. Stufe:

7,1 g 3-Brom-N-benzyl-N-methylanilin werden in 40 ml Eisessig gelöst, mit 2,53 g 37 %-iger Formalinlösung versetzt und 3-4 Stunden auf 80°C erwärmt. Es wird auf 250 ml Eiswasser gegossen und mit 100 ml Dichlormethan versetzt. Man neutralisiert mit Natronlauge und extrahiert mit Dichlormethan. Die vereinigten organischen Phasen werden über wasserfreiem Natriumsulfat getrocknet, das Lösungsmittel entfernt und der Rückstand chromatographisch gereinigt. Ausbeute: 61 %; HPLC-MS: MH⁺ = 563.07

### 2. Stufe:

3 g 4,4'-Methylen-bis-(N-benzyl-3-brom-N-methylanilin) werden in 120 ml trockenem THF und bei -80°C mit n-Butyllithium versetzt. Nach beendeter Zugabe wird 1 Stunde bei -80°C gerührt. Zur Reaktionsmischung werden 1,38 ml Dichlordimethylsilan zugetropft. Anschließend lässt man langsam auf Raumtemperatur erwärmen und rührt weitere 2 ht. Man gießt in 500 ml Ethanol, versetzt mit 1,57 g p-Chloranil und erhitzt 30 min zum Rückfluss. Die tief blau gefärbte Reaktionsmischung wird im Vakuum bis zur Trockene eingeengt. Der Rückstand wird chromatographisch gereinigt. Ausbeute: 68 %; HPLC-MS: M⁺ = 461.2

### 3. Stufe:

1 g des Farbstoffs (Stufe 2) wird in 12 ml konz. Schwefelsäure gelöst und 5 h bei Raumtemperatur gerührt. Man tropft in Wasser/Acetonitril 1:1, neutralisiert mit wässrigem Natriumhydrogencarbonat und extrahiert mit Dichlormethan. Die vereinigten organischen Phasen werden über wasserfreiem Natriumsulfat getrocknet und am Rotationsverdampfer bis zur Trockene eingeengt. Der Rückstand wird in 40 ml Ethanol aufgenommen und bei Raumtemperatur mit 0,5 eq. Natriumborhydrid versetzt. Es wird erneut bis zur Trockene einrotiert. Der Rückstand wird in Dimethylformamid aufgenommen und unter Schutzgas mit 4 eq. 4-(Brommethyl)-benzol-1,3-disulfonsäure versetzt und auf 100°C erwärmt. Es wird durch Zugabe von p-Chloranil oxidiert und chromatographisch gereinigt. Ausbeute: 73 %; HPLC-MS: M⁺ = 781.07

### 4. Stufe, Verbindung 89:

500 mg des Farbstoffs (Stufe 3) werden in 40 ml DMF suspendiert und unter Schutzgas mit 1,5 eq. Tetrabutylammoniumcyanid (TBACN) versetzt. Es wird 1-2 Stunden bei Raumtemperatur gerührt und anschließend mit p-Chloranil oxidiert. Man tropft unter kräftigem Rühren in 300 ml Diethylether und dekantiert vom ausgeölten Farbstoff ab. Der Rückstand wird in 15 ml Wasser/Acetonitril 9:1 aufgenommen und chromatographisch gereinigt. Ausbeute: 56 %; HPLC-MS: M⁺ = 806.06

### 5. Stufe, Verbindung 90:

300 mg des Farbstoffs (Stufe 4) werden in 6 ml DMSO/Wasser 1:1 gelöst und mit 10 eq. 4-Aminobuttersäure (4-ABS) gelöst in 2 ml DMSO/Wasser 1:1 versetzt. Die Reaktionsmischung wird bei Raumtemperatur verrührt und die Reaktion mittels HPLC verfolgt. Die Reaktionsmischung wird mit 35 ml Wasser verdünnt und chromatographisch gereinigt. Ausbeute: 67 %; HPLC-MS: M⁺ = 882.12

### Verbindungen 102 und 103: Herstellung eines Carbazins.

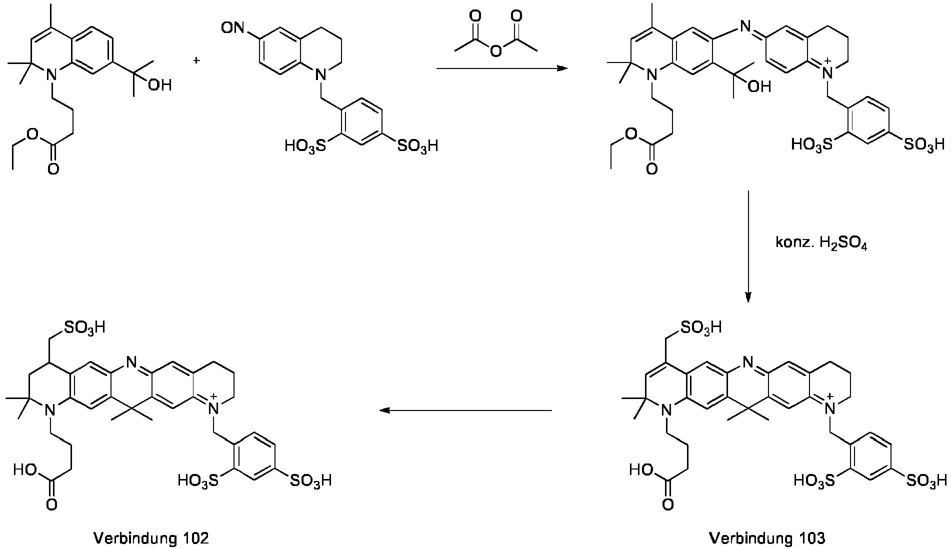

Die beiden Edukte der eigentlichen Farbstoffsynthese werden gemäß dem Fachmann bekannten Verfahren hergestellt:
4-(7-(2-Hydroxypropyl)-2,2,4-trimethyl-1,2-dihydrochinolinyl)-buttersäureethylester wird aus käuflichem 3-Aminobenzoesäureester in drei Stufen erhalten. Zuerst wird gemäß A. Rosowsky, E.J. Modest, 2,2,4-Trimethyl-1,2-dihydroquinolines. Preparation and Nuclear Magnetic Resonance Studies, J. Org. Chem. 30, 1965, 1832-1837 das 2,2,4-Trimethyl-1,2-dihydrochinolin-Gerüst aufgebaut. Es schließt sich eine Grignard-Reaktion mit Methylmagnesiumiodid an, um den tertiären Alkohol zu erhalten. Anschließend wird die Aminogruppe mit 4-Iodbuttersäureethylester alkyliert.

4-(6-Nitroso-1,2,3,4-tetrahydrochinolinyl)-benzyl-1,3-disulfonsäure erhält man aus käuflichem 1,2,3,4-Tetrahydrochinolin in zwei Stufen. Das Ausgangsmaterial wird mit 4-(Brommethyl)-benzol-1,3-disulfonsäure zunächst alkyliert. Das Alkylierungsprodukt wird dann mit Natriumnitrit in salzsaurer Lösung nitrosiert.

Die Synthese zu Verbindung 103 läuft wie dargestellt in zwei Stufen ab. Die Verknüpfung der beiden Edukte gelingt in Essigsäureanhydrid wie bei J. Griffiths, R. Cox, Light absorption and stability properties of some near-IR indamine dyes related to Bindschedler's green, Dyes and Pigments 42, 1999, 29-34 beschrieben. Anschließend erfolgt der vollständige Ringschluss durch Behandeln der verbrückten Zwischenstufe mit konz. Schwefelsäure. In diesem Schritt finden außerdem die Verseifung des Ethylesters sowie die bekannte Sulfonierung der allylischen Methylgruppe am Dihydrochinolinring statt. Der Farbstoff wird durch Eingießen der schwefelsauren Reaktionslösung in eine Mischung aus Ether/Dioxan isoliert und anschließend chromatographisch gereinigt. Durch anschließende katalytische Hydrierung des Dihydrochinolin-Rings mit Wasserstoff und Pd auf Aktivkohle im Autoklaven lässt sich die Verbindung 102 quantitativ erhalten. Gesamtausbeute: 30 %

### Verbindung 106: Herstellung eines sulfonierten Carbostyrils.

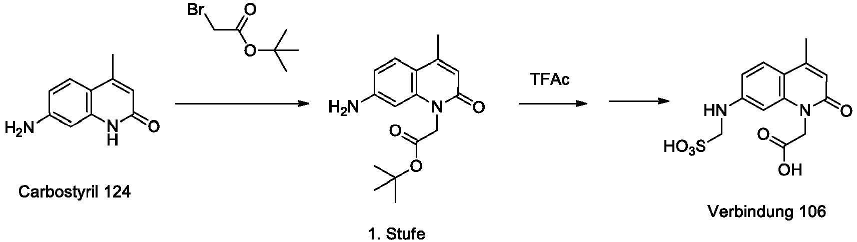

### 1. Stufe:

2 g Carbostyril 124 (7-Amino-4-methylquinolin-2(1H)-one, CAS: 19840-99-4, Fluorochem) werden in 20 ml Dimethylformamid (DMF) suspendiert und unter Schutzgas mit 480 mg einer 60 %-igen Suspension von Natriumhydrid in Paraffinöl versetzt. Die Reaktionsmischung wird 60 min verrührt. Es werden 1,8 ml Bromessigsäure-tert-butylester zugetropft und weitere 24 h gerührt. Man tropft auf 200 ml Wasser. Der sich bildende Niederschlag wird abgesaugt, getrocknet und chromatographiert.

### Verbindung 106:

Der Feststoff der 1. Stufe wird in 60 ml Dichlormethan/Trifluoressigsäure 1:1 gelöst und 18 h verrührt. Das Lösungsmittel wird im Vakuum entfernt und der Rückstand mit 10 ml Acetonitril versetzt. Das Produkt kristallisiert aus. Man saugt ab, trocknet im Vakuum und suspendiert den Feststoff in 30 ml Wasser. Durch Zugabe von 10 %-iger Natronlauge wird er vollständig in Lösung gebracht (pH 10). Es werden 2 g Natriumpyrosulfit in 20 ml Wasser vorgelegt, mit 5 ml 37 %-ige Formaldehydlösung versetzt und zum Rückfluss erhitzt. Die Lösung des Carbostyrils wird zugetropft und nach beendeter Zugabe noch 2 h am Rückfluss erhitzt. Die Reaktionsmischung wird abgekühlt und chromatographisch gereinigt. Gesamtausbeute: 43 %; HPLC-MS: MH⁺ = 327.07

### Verbindungen 112 und 113: Einführung von Alkylsulfonsäuren mittels eines 1,3,5-Triazins.

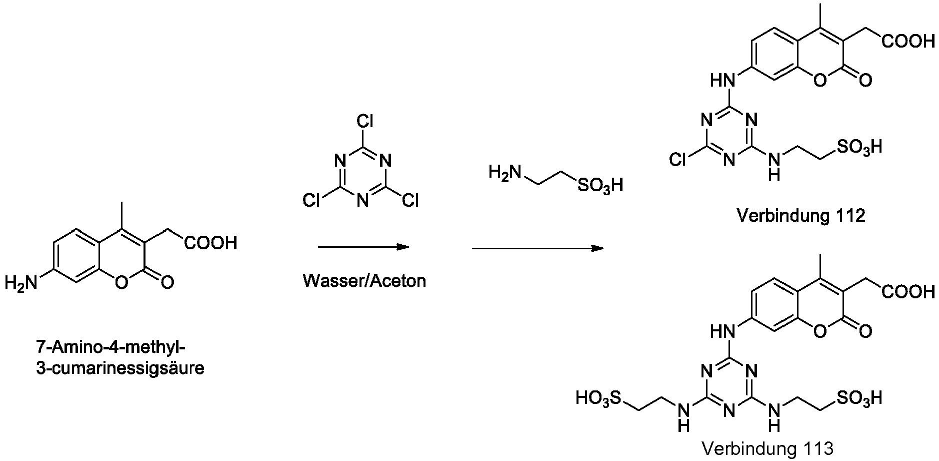

### Verbindung 112:

100 mg 7-Amino-4-methyl-3-cumarinessigsäure (Sigma, CAS: 106562-32-7) werden in Wasser suspendiert und durch Zugabe von Natriumcarbonatlösung vollständig gelöst (pH 8). 79 mg Cyanurchlorid werden in 3 ml Aceton gelöst und in 25 ml Eiswasser getropft. Zur erhaltenen Suspension wird die Lösung des Cumarins bei 0°C langsam zugetropft. Der pH-Wert wird durch Zugabe von 5 %-iger Natronlauge bei 8 - 9 gehalten. Nach beendeter Zugabe wird noch 10 min bei 0°C gerührt. Die Reaktionsmischung wird auf Raumtemperatur erwärmt und tropfenweise mit einer Lösung von 55 mg Taurin in 4 ml Wasser versetzt. Während der Zugabe wird die Temperatur langsam auf 40 - 45°C erhöht und der pH-Wert durch Zugabe von 5 %-iger Natronlauge bei 8 - 9 gehalten. Die Reaktion wird mittels HPLC verfolgt und ist nach 60 min beendet. Die Reaktionsmischung wird chromatographisch gereinigt. Ausbeute: 82 %; HPLC-MS: MH⁺ = 470.06

### Verbindung 113:

100 mg 7-Amino-4-methyl-3-cumarinessigsäure werden in Wasser suspendiert und durch Zugabe von Natriumcarbonatlösung vollständig gelöst (pH 8). 79 mg Cyanurchlorid werden in 3 ml Aceton gelöst und in 25 ml Eiswasser getropft. Zur erhaltenen Suspension wird die Lösung des Cumarins bei 0 °C langsam zugetropft. Der pH-Wert wird durch Zugabe von 5 %-iger Natronlauge bei 8 - 9 gehalten. Nach beendeter Zugabe wird noch 10 min bei 0°C gerührt. Die Reaktionsmischung wird auf Raumtemperatur erwärmt und tropfenweise mit einer Lösung von 55 mg Taurin in 4 ml Wasser versetzt. Während der Zugabe wird die Temperatur langsam auf 40 - 45°C erhöht und der pH-Wert durch Zugabe von 5 %-iger Natronlauge bei 8 - 9 gehalten. Die Reaktionsmischung wird 30 min bei 45°C gehalten. Es werden weitere 70 mg Taurin in Wasser gelöst und zugesetzt. Die Temperatur der Reaktionsmischung wird für 1 h auf 85 - 90°C erhöht. Die Reaktionsmischung wird chromatographisch gereinigt. Ausbeute: 78 %; HPLC-MS: MH⁺ = 559.09

## Patentansprüche

1. Verbindungen der allgemeinen Formeln (I) - (IV) 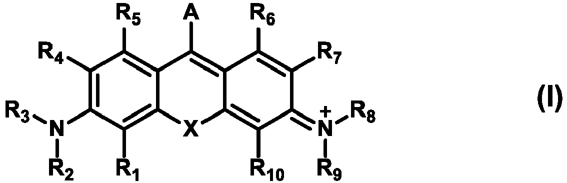 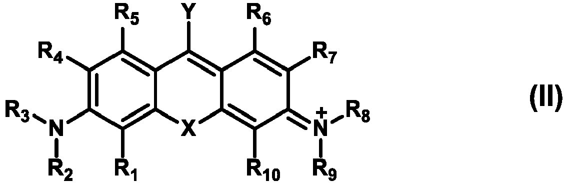 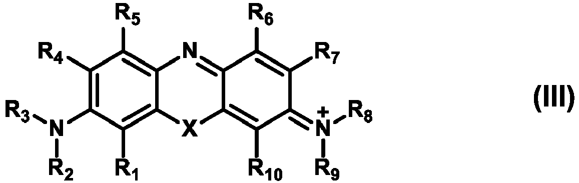 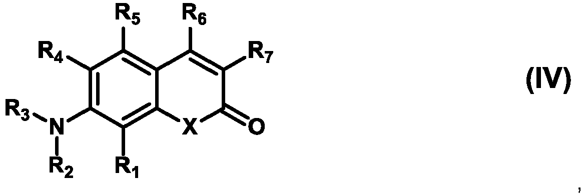
worin R₁, R₄, R₅, R₆, R₇ und R₁₀ unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R₂, R₃, R₈ und R₉ unabhängig voneinander Wasserstoff oder eine Kohlenwasserstoffgruppe mit 1-20 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
wobei jeder der Reste in den Verbindungen der allgemeinen Formeln (I) - (IV) mit einem oder mehreren benachbarten Resten ein Ringsystem bilden kann,
X in der allgemeinen Formel (I) eine Gruppe ausgewählt aus O, S, SO₂, CR₁₁R₁₂ oder SiR₁₃R₁₄ bedeutet,
X in der allgemeinen Formel (II) eine Gruppe ausgewählt aus O, S, CR₁₁R₁₂, SiR₁₃R₁₄ oder NR₁₅ bedeutet,
X in der allgemeinen Formel (III) eine Gruppe ausgewählt aus O, S oder CR₁₁R₁₂ bedeutet,
X in der allgemeinen Formel (IV) O oder NR₁₅ bedeutet
und wobei R₁₁, R₁₂, R₁₃, R₁₄ und R₁₅ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
oder R₁₁ und R₁₂ oder R₁₃ und R₁₄ jeweils zusammen mit dem Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
A in der allgemeinen Formel (I) einen Aryl- oder Heteroaryl-Rest bedeutet, der gegebenenfalls einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), CONH(Alkyl), CON(Alkyl)₂, CONH(Aryl), CON(Aryl)₂, PO₃H₂, SO₃H und einer Kohlenwasserstoffgruppe umfasst, wobei die Kohlenwasserstoffgruppe ihrerseits gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
Y in der allgemeinen Formel (II) Wasserstoff, CN, COOH, COO(Alkyl), COO(Aryl), NHR₁₆, NR₁₇R₁₈, PO₃H₂, SO₃H oder eine aliphatische Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
und R₁₆, R₁₇, und R₁₈ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
oder R₁₇ und R₁₈ zusammen mit dem Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst
mit der Maßgabe, dass mindestens einer der Reste R₂, R₃, R₈ oder R₉ die Struktur B aufweist, wobei es sich bei B um eine der folgenden isomeren Strukturen (H), (I) oder (J) handelt 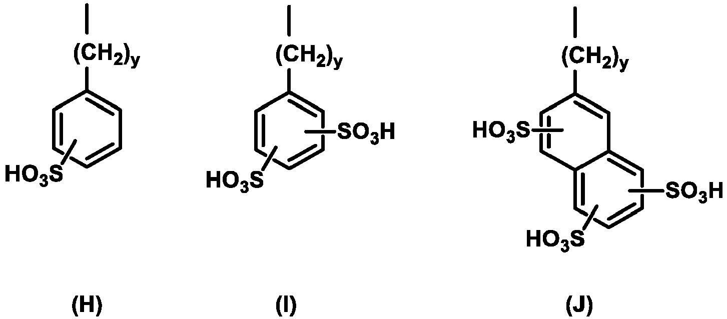
mit y = 0, 1, 2, 3, 4, 5, 6 und wobei nicht näher bezeichnete Positionen in den gezeigten Aryl-Systemen wie R₁ definiert sind, oder
wobei es sich bei B um eine der Strukturen (K) oder (L) mit x = 1, 2, 3, 4, 5, 6 handelt 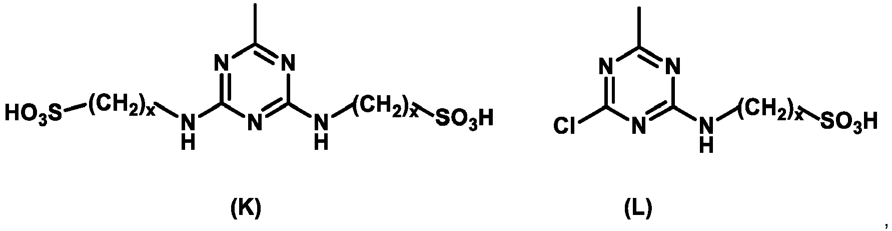 oder wobei es sich bei B um -CH₂SO₃H oder um -CH₂CH₂SO₃H handelt
wobei die Struktur B nicht ein Ringsystem mit einem oder mehreren benachbarten Resten bildet,
wobei in Verbindungen der allgemeinen Formel (I) für X = O der Rest B nicht -CH₂SO₃H oder - CH₂CH₂SO₃H darstellt, wenn nicht gleichzeitig wenigstens einer oder mehrere der Reste R₁, R₄, R₅, R₆, R₇, R₁₀ und/oder A wenigstens eine weitere SO₃H-Gruppe aufweist.

2. Verbindungen der allgemeinen Formeln (I) - (IV) nach Anspruch 1, **dadurch gekennzeichnet, dass** R₁ und R₂, R₃ und R₄, R₇ und R₈ oder/und R₉ und R₁₀ zusammen mit den Atomen, an welche sie gebunden sind, einen 5- oder 6-gliedrigen Ring bilden, wobei der Ring eine oder mehrere Doppelbindungen oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst.

3. Verbindungen der allgemeinen Formeln (I) - (IV) nach Anspruch 2, **dadurch gekennzeichnet, dass** mindestens ein 5- oder 6-gliedriger Ring mit einer oder mehreren Kohlenwasserstoffgruppen mit 1-20 C-Atomen substituiert ist, wobei die Kohlenwasserstoffgruppe(n) gegebenenfalls einen oder mehrere Substituenten ausgewählt aus der Gruppe bestehend aus COOH, COO(Alkyl), COO(Aryl) und SO₃H umfasst/umfassen.

4. Verbindungen der allgemeinen Formeln (I) - (IV) nach Anspruch 3, **dadurch gekennzeichnet, dass** es sich bei den Ringen um eine der folgenden Strukturen (C), (D), (E), (F) oder (G) handelt 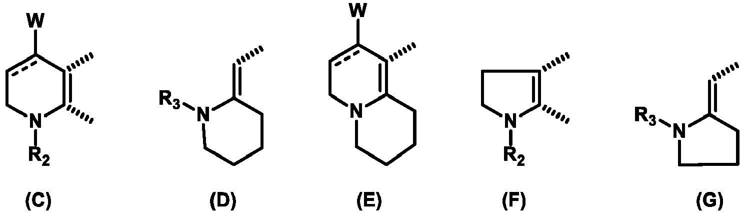 mit W = H, CH₃ oder CH₂SO₃H und wobei nicht näher bezeichnete Positionen in den gezeigten Ringsystemen wie R₁ definiert sind und gestrichelte Linien (- - -) gegebenenfalls vorhandene Doppelbindungen bezeichnen.

5. Verbindungen der allgemeinen Formeln (I) - (IV) nach einem der Ansprüche 1 - 4, **dadurch gekennzeichnet, dass** es sich bei der Gruppe B um einen Rest ausgewählt aus 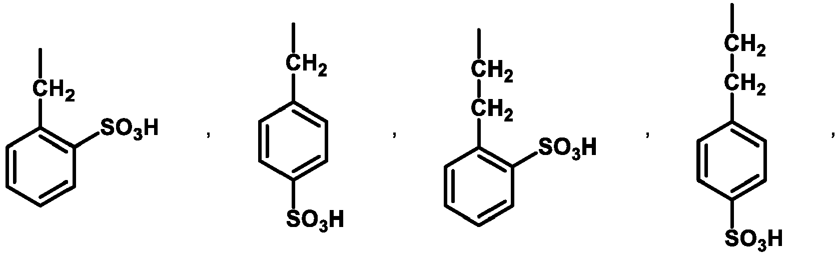 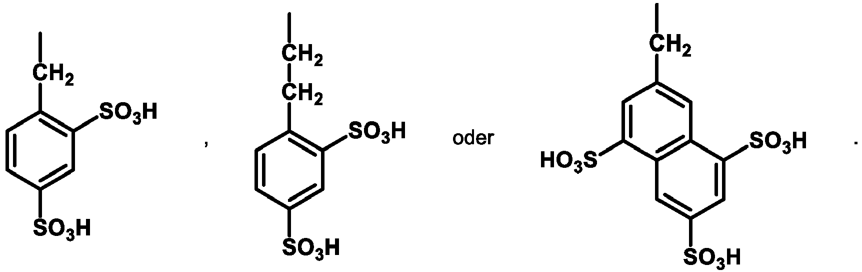 handelt.

6. Verbindungen der allgemeinen Formeln (I) - (IV) nach einem der Ansprüche 1 - 5, wobei X = O.

7. Verbindungen der allgemeinen Formeln (I) - (III) nach einem der Ansprüche 1 - 5, wobei X = CR₁₁R₁₂, wobei R₁₁ und R₁₂ unabhängig voneinander Wasserstoff oder eine Kohlenwasserstoffgruppe mit 1-20 C-Atomen, insbesondere eine Alkylgruppe mit 1-6 C-Atomen, bedeuten.

8. Verbindungen der allgemeinen Formeln (I) und (II) nach einem der Ansprüche 1 - 5, wobei X = SiR₁₃R₁₄, wobei R₁₃ und R₁₄ unabhängig voneinander Wasserstoff oder eine Kohlenwasserstoffgruppe mit 1-20 C-Atomen, insbesondere eine Alkylgruppe mit 1-6 C-Atomen, bedeuten.

9. Verbindungen der allgemeinen Formel (I) nach einem der Ansprüche 1 - 8, **dadurch gekennzeichnet, dass** es sich bei A um eine der folgenden teilweise isomeren Strukturen (M), (N), (O), (P), (Q) handelt 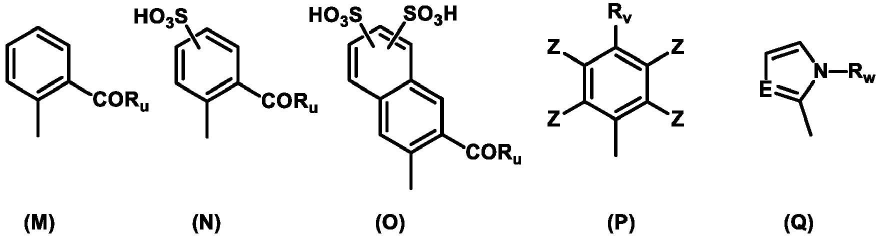
wobei E eine Gruppe ausgewählt aus O, S, N oder ⁺NR₁₉ darstellt,
Rᵤ = Halogen, OR₂₀, NHR₂₁, NR₂₂R₂₃,
Z ausgewählt aus Wasserstoff oder Halogen ist,
Rᵥ = Z, S(CH₂)XCOOH, S(CH₂)XSO₃H, NHR₂₄, NR₂₅R₂₆ und
R_{w} = H, Alkyl, (CH₂)XCOOH, (CH₂)XSO₃H mit x = 1, 2, 3, 4, 5, 6 bedeutet,
R₁₉ und R₂₀ unabhängig voneinander Wasserstoff oder eine Kohlenwasserstoffgruppe mit 1-20 C-Atomen bedeuten, wobei die Kohlenwasserstoffgruppe gegebenenfalls ein oder mehrere Heteroatome ausgewählt aus der Gruppe bestehend aus N, O und S oder/und einen oder mehrere Substituenten vorzugsweise ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H enthält, und R₂₁, R₂₂ und R₂₃ sowie R₂₄, R₂₅ und R₂₆ genau wie R₁₆, R₁₇ und R₁₈ definiert sind und nicht näher bezeichnete Positionen wie R₁ definiert sind.

10. Verbindungen der allgemeinen Formel (II) nach einem der Ansprüche 1 - 8, **dadurch gekennzeichnet, dass** es sich bei Y um Wasserstoff, CN, CF₃, NHR₁₆, NR₁₇R₁₈, (CF₂)ₓCOOH mit x = 1, 2, 3, 4, 5, 6, oder die isomere Struktur (T) handelt 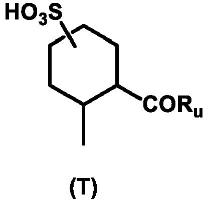 wobei Rᵤ = Halogen, OR₂₀, NHR₂₁, NR₂₂R₂₃ und nicht näher bezeichnete Positionen wie R₁ definiert sind.

11. Verwendung einer Verbindung der allgemeinen Formeln (I) - (IV) gemäß einem der Ansprüche 1 bis 10 zur qualitativen oder/und quantitativen Bestimmung eines Analyten in einer Probe.

12. Verwendung nach Anspruch 11, wobei die Verbindung der allgemeinen Formeln (I) - (IV) an den nachzuweisenden Analyten oder/und an einen Bestandteil eines Nachweisreagenzes oder/und einen Träger gekoppelt wird.

13. Verwendung nach Anspruch 12, wobei die Kopplung über eine kovalente Bindung erfolgt.

14. Konjugat aus einer Verbindung der allgemeinen Formeln (I) - (IV) nach einem der Ansprüche 1 bis 10 und einem Bindepartner.

15. Konjugat nach Anspruch 14, wobei der Bindepartner ausgewählt ist aus Lipiden, Peptiden, Polypeptiden, Nukleinsäuren, Nukleosiden, Nukleotiden, Nukleinsäure-Analoga und Haptenen.

## Claims

1. Compounds of the general formulae (I) - (IV) 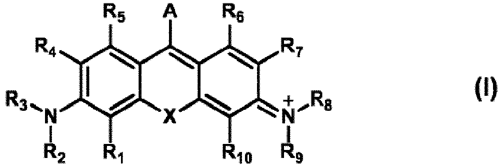 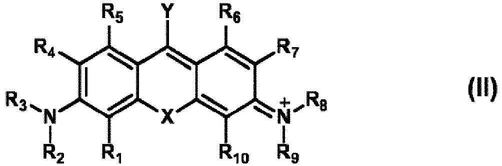 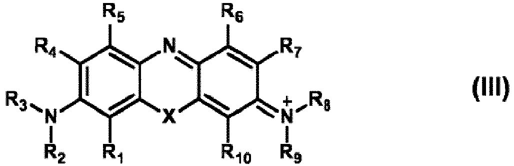 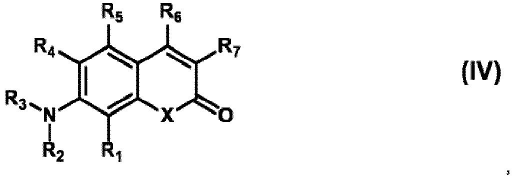 in which R₁, R₄, R₅, R₆, R₇, and R₁₀ independently of one another mean hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H or a hydrocarbon group having 1-20 C atoms, wherein the hydrocarbon group optionally comprises one or more heteroatoms selected from the group consisting of N, O, and S or/and one or more substituents preferably selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
R₂, R₃, R₈, and R₉ independently of one another mean hydrogen or a hydrocarbon group having 1-20 C atoms, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
wherein each of the moieties in the compounds of the general formulae (I) - (IV) can form a ring system with one or more adjacent moieties,
X in the general formula (I) means a group selected from O, S, SO₂, CR₁₁R₁₂ or SiR₁₃R₁₄,
X in the general formula (II) means a group selected from O, S, CR₁₁R₁₂, SiR₁₃R₁₄ or NR₁₅,
X in the general formula (III) means a group selected from O, S or CR₁₁R₁₂,
X in the general formula (IV) means O or NR₁₅,
and wherein R₁₁, R₁₂, R₁₃, R₁₄ and R₁₅ independently of one another mean hydrogen, CN, COOH, COO(alkyl), COO(aryl), PO₃H₂, SO₃H or a hydrocarbon group having 1-20 C atoms, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
or R₁₁ and R₁₂ or R₁₃ and R₁₄ in each case together with the atom to which they are attached, form a 3- to 7-membered ring, wherein the ring can comprise one or more double bonds or/and one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H and a hydrocarbon group having 1-20 C atoms or/and can be fused with one or more 3- to 7-membered rings, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, and SO₃H,
A in the general formula (I) means an aryl or heteroaryl moiety, which optionally comprises one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), CONH(alkyl), CON(alkyl)₂, CONH(aryl), CON(aryl)₂, PO₃H₂, SO₃H and a hydrocarbon group, wherein the hydrocarbon group itself optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
Y in the general formula (II) means hydrogen, CN, COOH, COO(alkyl), COO(aryl), NHR₁₆, NR₁₇R₁₈, PO₃H₂, SO₃H or an aliphatic hydrocarbon group having 1-20 C atoms, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
and R₁₆, R₁₇, and R₁₈ independently of one another mean hydrogen, CN, COOH, COO(alkyl), COO(aryl), PO₃H₂, SO₃H or a hydrocarbon group having 1-20 C atoms, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
or R₁₇ and R₁₈ together with the atom to which they are attached, form a 3- to 7-membered ring, wherein the ring can comprise one or more double bonds or/and one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H and a hydrocarbon group having 1-20 C atoms or/and can be fused with one or more 3- to 7-membered rings, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
provided that at least one of the moieties R₂, R₃, R₈ or R₉ has the structure B, wherein B has one of the following isomeric structures (H), (I) or (J) 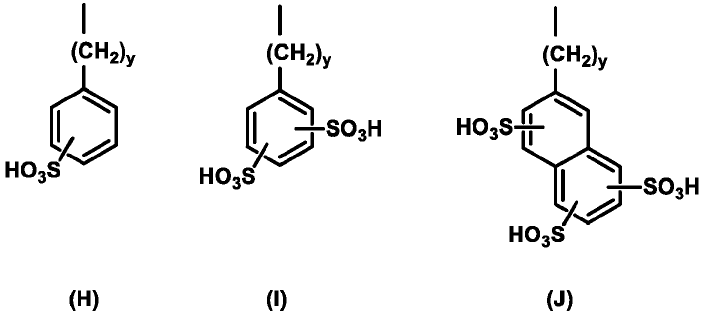 with y = 0, 1, 2, 3, 4, 5, 6 and wherein positions not further specified in the shown aryl systems are defined as R₁ or
wherein B has one of the structures (K) or (L) with x = 1, 2, 3, 4, 5, 6 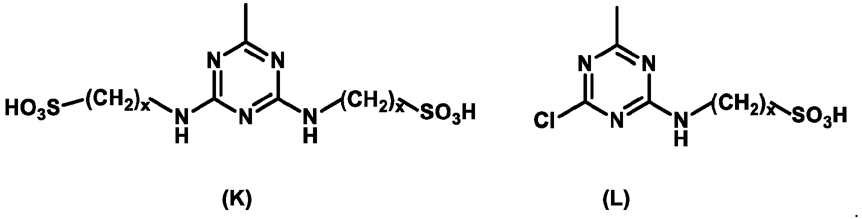 or wherein B represents -CH₂SO₃H or -CH₂CH₂SO₃H,
wherein structure B is not integrated within a ring system with one or more adjacent moieties, wherein in compounds of the general formula (I) for X = O the moiety B is not -CH₂SO₃H or - CH₂CH₂SO₃H, , unless at the same time at least one or more of the moieties R₁, R₄, R₅, R₆, R₇, R₁₀ and/or A comprises at least one further SO₃H group.

2. Compounds of the general formulae (I) - (IV) according to claim 1, wherein R₁ and R₂, R₃ and R₄, R₇ and R₈ or/and R₉ and R₁₀ together with the atoms to which they are attached, form a 5- or 6-membered ring, wherein the ring can comprise one or more double bonds or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H and a hydrocarbon group having 1-20 C atoms, wherein the hydrocarbon group optionally comprises one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, and SO₃H.

3. The compounds of the general formulae (I) - (IV) according to claim 2, wherein at least one 5- or 6-membered ring is substituted with one or more hydrocarbon groups having 1-20 C atoms, wherein the hydrocarbon group(s) optionally comprise(s) one or more substituents selected from the group consisting of COOH, COO(alkyl), COO(aryl) and SO₃H.

4. The compounds of the general formulae (I) - (IV) according to claim 3, wherein the rings have one of the following structures (C), (D), (E), (F) or (G) 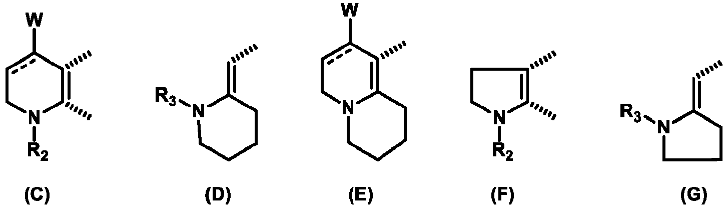 with W = H, CH₃ or CH₂SO₃H and wherein positions not further specified are defined in the shown ring systems as R₁ and the broken lines (- - -) designate optionally present double bonds.

5. The compounds of the general formulae (I) - (IV) according to any one of claims 1-4, wherein group B represents a residue selected from

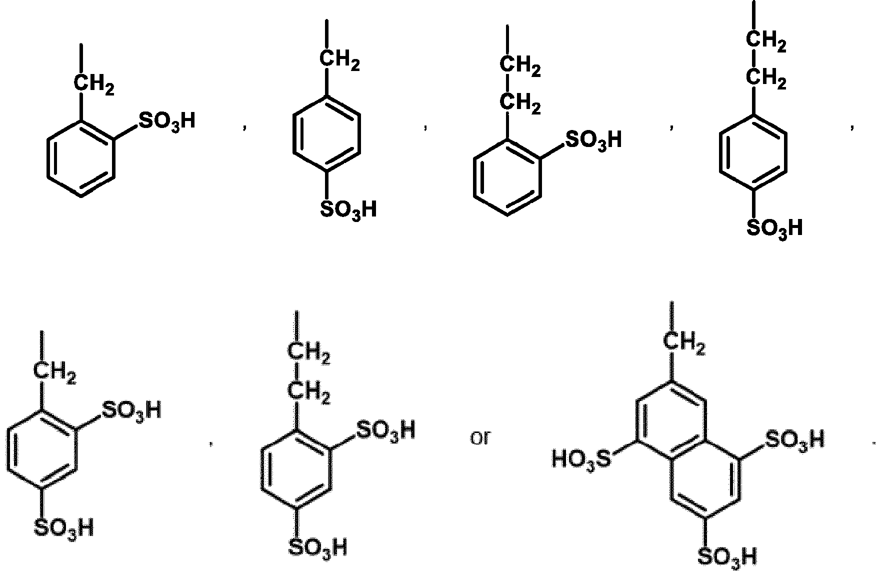

6. The compounds of the general formulae (I) - (IV) according to any one of claims 1-5, wherein X = O.

7. The compounds of the general formulae (I) - (III) according to any one of claims 1-5, wherein X = CR₁₁R₁₂, wherein R₁₁ and R₁₂ independently of one another mean hydrogen or a hydrocarbon group having 1-20 C atoms, in particular an alkyl group having 1-6 C atoms.

8. The compounds of the general formulae (I) and (II) according to any one of claims 1-5, wherein X = SiR₁₃R₁₄, wherein R₁₃ and R₁₄ independently of one another mean hydrogen or a hydrocarbon group having 1-20 C atoms, in particular an alkyl group having 1-6 C atoms.

9. The compounds of the general formula (I) according to any one of claims 1-8, **characterized in that** A has one of the following partially isomeric structures (M), (N), (O), (P), (Q) 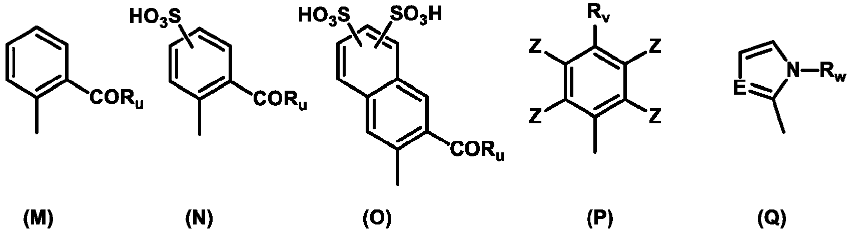 wherein E represents a group selected from O, S, N or ⁺NR₁₉,
Rᵤ = halogen, OR₂₀, NHR₂₁, NR₂₂R₂₃,
Z is selected from hydrogen or halogen,
Rᵥ = Z, S(CH₂)ₓCOOH, S(CH₂)ₓSO₃H, NHR₂₄, NR₂₅R₂₆ and
R_{w} = H, alkyl, (CH₂)ₓCOOH, (CH₂)ₓSO₃H with x = 1, 2, 3, 4, 5, 6,
R₁₉ and R₂₀ independently of one another mean hydrogen or a hydrocarbon group having 1-20 C atoms, wherein the hydrocarbon group optionally contains one or more heteroatoms selected from the group consisting of N, O and S or/and one or more substituents preferably selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H, and
R₂₁, R₂₂ and R₂₃ as well as R₂₄, R₂₅ and R₂₆ are defined exactly as R₁₆, R₁₇ and R₁₈ and positions not further specified are defined as R₁.

10. The compounds of the general formula (II) according to any one of claims 1-8, wherein Y is hydrogen, CN, CF₃, NHR₁₆, NR₁₇R₁₈, (CF₂)ₓCOOH with x = 1, 2, 3, 4, 5, 6, or the isomeric structure (T) 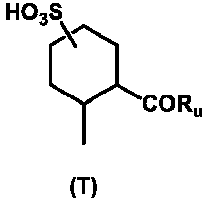 wherein Rᵤ = halogen, OR₂₀, NHR₂₁, NR₂₂R₂₃ and positions not further specified are defined as R₁.

11. Use of a compound of the general formulae (I) - (IV) according to any one of claims 1-10 for qualitative or/and quantitative determination of an analyte in a sample.

12. Use according to claim 11, wherein the compound of the general formulae (I) - (IV) is coupled to the analyte to be determined or/and to a component of a detection reagent or/and to a carrier.

13. Use according to claim 12, wherein coupling is by means of a covalent bond.

14. Conjugate of a compound of the general formulae (I) - (IV) according to any one of claims 1-10 and a binding partner.

15. Conjugate according to claim 14, wherein the binding partner is selected from lipids, peptides, polypeptides, nucleic acids, nucleosides, nucleotides, nucleic acid analogs, and haptens.

## Revendications

1. Composés des formules générales (I) - (IV) 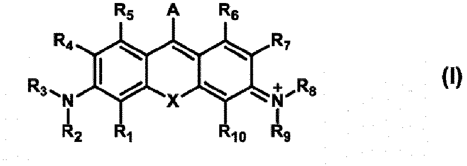 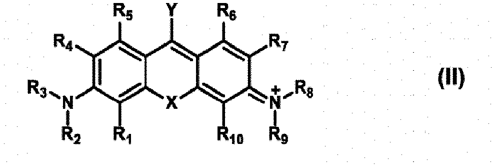 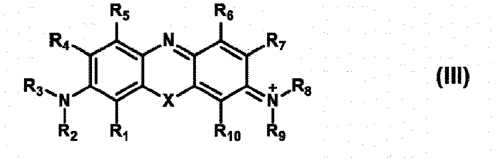 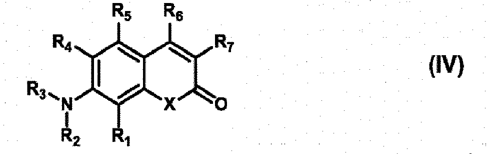 dans lesquels R₁, R₄, R₅, R₆, R₇ et R₁₀ représentent, indépendamment les uns des autres, l'hydrogène, un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H ou un groupe hydrocarboné ayant 1 à 20 atomes de carbone, le groupe hydrocarboné comprenant éventuellement un ou plusieurs hétéroatomes, choisis dans le groupe constitué par N, O et S ou/et un ou plusieurs substituants, choisis de préférence dans le groupe constitué par un halogène, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
dans lequel R₂, R₃, R₈ et R₉ représentent, indépendamment les uns des autres, l'hydrogène ou un groupe hydrocarboné ayant 1 à 20 atomes de carbone, le groupe hydrocarboné comprenant éventuellement un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
dans lesquels chacun des radicaux dans les composés des formules générales (I) - (IV) peut former un système cyclique avec un ou plusieurs radicaux adjacents,
X dans la formule générale (I) représente un groupe choisi parmi O, S, SO₂, CR₁₁R₁₂ ou SiR₁₃R₁₄,
X dans la formule générale (II) représente un groupe choisi parmi O, S, CR₁₁R₁₂, SiR₁₃R₁₄ ou NR₁₅,
X dans la formule générale (III) représente un groupe choisi parmi O, S ou CR₁₁R₁₂, X dans la formule générale (IV) représente O ou NR₁₅,
et dans lesquels R₁₁, R₁₂, R₁₃, R₁₄ et R₁₅ représentent, indépendamment les uns des autres, l'hydrogène, CN, COOH, COO(alkyle), COO(aryle), PO₃H₂, SO₃H ou un groupe hydrocarboné ayant 1 à 20 atomes de carbone, le groupe hydrocarboné comprenant éventuellement un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
ou R₁₁ et R₁₂ ou R₁₃ et R₁₄ forment ensemble avec l'atome auquel ils sont liés un cycle de 3 à 7 chaînons, le cycle comprenant une ou plusieurs doubles liaisons ou/et un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H et un groupe hydrocarboné ayant 1 à 20 atomes de carbone ou/et peut être fusionné avec un ou plusieurs cycles de 3 à 7 chaînons, le groupe hydrocarboné pouvant éventuellement comprendre un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
A, dans la formule générale (I), représente un radical aryle ou hétéroaryle comprenant éventuellement un ou plusieurs substituants choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), CONH(alkyle), CON(alkyle)₂, CONH(aryle), CON(aryle)₂, PO₃H₂, SO₃H et un groupe hydrocarboné, le groupe hydrocarboné comprenant à son tour éventuellement un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle) PO₃H₂ et SO₃H,
Y, dans la formule générale (II), représente l'hydrogène, CN, COOH, COO(alkyle), COO(aryle), NHR₁₆, NR₁₇R₁₈, PO₃H₂, SO₃H ou un groupe hydrocarboné aliphatique ayant 1 à 20 atomes de carbone, le groupe hydrocarboné comprenant éventuellement un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle) PO₃H₂ et SO₃H,
et R₁₆, R₁₇ et R₁₈ représentent, indépendamment les uns des autres, l'hydrogène, CN, COOH, COO(alkyle), COO(aryle), PO₃H₂, SO₃H ou un groupe hydrocarboné ayant 1 à 20 atomes de carbone, le groupe hydrocarboné comprenant éventuellement un ou plusieurs hétéroatomes, choisis dans le groupe constitué par N, O et S ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, et SO₃H,
ou R₁₇ et R₁₈ forment ensemble avec l'atome auquel ils sont liés un cycle de 3 à 7 chaînons, le cycle comprenant une ou plusieurs doubles liaisons ou/et un ou plusieurs hétéroatomes choisis dans le groupe constitué par N, O et S ou/et un ou plusieurs substituants choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H et un groupe hydrocarboné ayant 1 à 20 atomes de carbone ou/et peut être fusionné avec un ou plusieurs cycles de 3 à 7 chaînons, dans lequel le groupe hydrocarboné peut éventuellement comprendre un ou plusieurs hétéroatomes choisis dans le groupe constitué par N, O et S ou/et un ou plusieurs substituants choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, et SO₃H,
à condition qu'au moins un des radicaux R₂, R₃, R₈ ou R₉ a la structure B, dans laquelle B est l'une des structures isomères (H), (I) ou (J) suivantes : 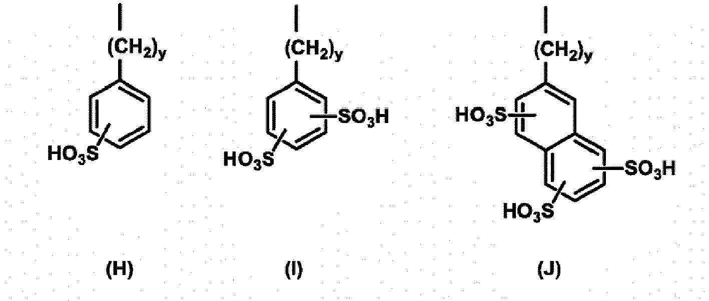 avec y = 0, 1, 2, 3, 4, 5, 6 et dans laquelle des positions non spécifiées dans les systèmes aryle indiqués sont définies comme R₁, ou
dans laquelle B est l'une des structures (K) ou (L) avec x = 1, 2, 3, 4, 5, 6, 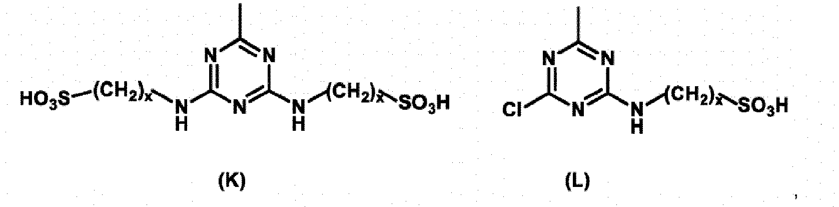 ou dans laquelle B est -CH₂SO₃H ou -CH₂CH₂SO₃H,
dans laquelle la structure B ne forme pas un système cyclique avec un ou plusieurs radicaux adjacents,
dans laquelle, dans les composés de formule générale (I) pour X = O le radical B n'est pas -CH₂SO₃H ou -CH₂CH₂SO₃H à moins qu'au moins un ou plusieurs des radicaux R₁, R₄, R₅, R₆, R₇, R₁₀ ou/et A contiennent simultanément au moins un autre groupe SO₃H.

2. Composés des formules générales (I) - (IV) selon la revendication 1, **caractérisés en ce que** R₁ et R₂, R₃ et R₄, R₇ et R₈ ou/et R₉ et R₁₀ forment ensemble avec les atomes auxquels ils sont liés un cycle à 5 ou 6 chaînons, dans lequel le cycle contient une ou plusieurs doubles liaisons ou/et un ou plusieurs substituants, choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H et un groupe hydrocarboné ayant 1 à 20 atomes de carbone, dans lequel le groupe hydrocarboné peut éventuellement comprendre un ou plusieurs hétéroatomes choisis dans le groupe constitué par N, O et S ou/et un ou plusieurs substituants choisis de préférence dans le groupe constitué par un halogène, CN, OH, O(alkyle), O(aryle), SH, S(alkyl), S(aryl), NH₂, NH(alkyle), NH(aryle), N(alkyle)₂, N(aryle)₂, NO₂, CHO, COOH, COO(alkyle), COO(aryle), PO₃H₂ et SO₃H.

3. Composés des formules générales (I) - (IV) selon la revendication 2, **caractérisés en ce qu'**au moins un cycle à 5 ou 6 chaînons est substitué par un ou plusieurs groupes hydrocarbonés ayant 1 à 20 atomes de carbone, dans lequel le ou les groupes hydrocarbonés comprend/comprennent éventuellement un ou plusieurs substituants choisis dans le groupe constitué par COOH, COO(alkyle), COO(aryle) et SO₃H.

4. Composés des formules générales (I) - (IV) selon la revendication 3, **caractérisés en**
**ce que** les cycles ont une des structures suivantes (C), (D), (E), (F) ou (G) 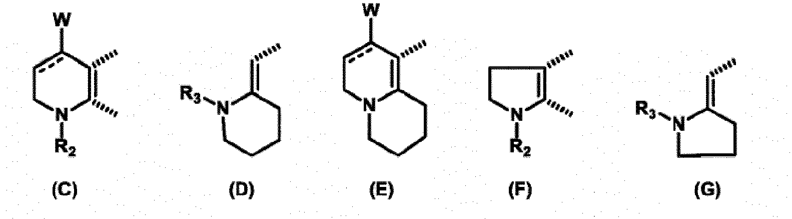 avec W = H, CH₃ ou CH₂SO₃H et où des positions non spécifiées dans les systèmes cycliques indiqués sont définies comme R₁ et où les lignes pointillées (- - -) indiquent des doubles liaisons éventuelles.

5. Composés des formules générales (I) - (IV) selon l'une des revendications 1 à 4, **caractérisés en ce que** le groupe B est un radical choisi parmi

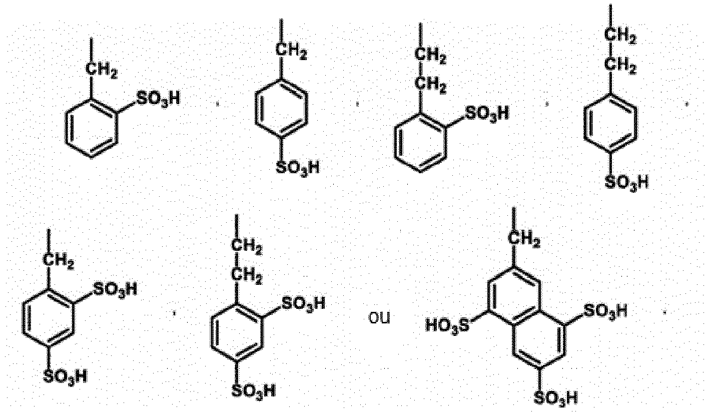

6. Composés des formules générales (I) - (IV) selon l'une des revendications 1 à 5, dans lesquelles X = O.

7. Composés des formules générales (I) - (III) selon l'une des revendications 1 à 5, dans lesquelles X = CR₁₁R₁₂, où R₁₁ et R₁₂ représentent, indépendamment l'un de l'autre, l'hydrogène ou un groupe hydrocarboné ayant 1 à 20 atomes de carbone, en particulier un groupe alkyle ayant 1 à 6 atomes de carbone.

8. Composés des formules générales (I) et (II) selon l'une des revendications 1 à 5, dans lesquelles X = SiR₁₃R₁₄, où R₁₃ et R₁₄ représentent indépendamment l'un de l'autre l'hydrogène ou un groupe hydrocarboné ayant 1 à 20 atomes de carbone, en particulier un groupe alkyle ayant 1 à 6 atomes de carbone.

9. Composés des formule générale (I) selon l'une des revendications 1 à 8, **caractérisés en ce que** A est l'une des structures suivantes partiellement isomères (M), (N), (O), (P), (Q) 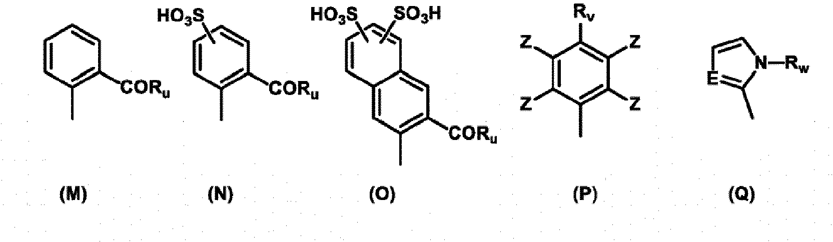 dans lesquels E est un groupe choisi parmi O, S, N ou ⁺NR₁₉,
Rᵤ = un halogène, OR₂₀, NHR₂₁, NR₂₂R₂₃,
Z est choisi parmi l'hydrogène ou un halogène,
R_{V} = Z, S(CH₂)ₓCOOH, S(CH₂)ₓSO₃H, NHR₂₄, NR₂₅R₂₆, et
R_{w} = H, alkyle, (CH₂)ₓCOOH, (CH₂)ₓSO₃H avec x = 1, 2, 3, 4, 5, 6,
R₁₉ et R₂₀ représentent, indépendamment l'un de l'autre, l'hydrogène ou un groupe hydrocarboné ayant 1 à 20 atomes de carbone, le groupe hydrocarboné comprenant éventuellement un ou plusieurs hétéroatomes choisis dans le groupe constitué par N, O et S ou/et un ou plusieurs substituants choisis de préférence dans le groupe constitué par un halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H, et R₂₁, R₂₂ et R₂₃ ainsi que R₂₄, R₂₅ et R₂₆ sont définies exactement comme R₁₆, R₁₇ et R₁₈ et des positions non spécifiées sont définies comme R₁.

10. Composés des formule générale (II) selon l'une quelconque des revendications 1 à 8, **caractérisés en ce que** Y est l'hydrogène, CN, CF₃, NHR₁₆, NR₁₇R₁₈, (CF₂)ₓCOOH avec x = 1, 2, 3, 4, 5, 6, ou la structure isomère (T) 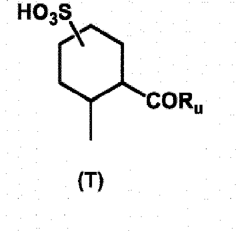 où Rᵤ = un halogène, OR₂₀, NHR₂₁, NR₂₂R₂₃ et des positions non spécifiées sont définies comme R₁.

11. Utilisation d'un composé des formule générale (I) - (IV) selon l'une des revendications 1 à 10 pour la détermination qualitative ou/et quantitative d'un analyte dans un échantillon.

12. Utilisation selon la revendication 11, dans laquelle le composé des formules générales (I) - (IV) est couplé à l'analyte à détecter ou/et à un composant d'un réactif de détection ou/et à un support.

13. Utilisation selon la revendication 12, dans laquelle l'accouplement est effectué par l'intermédiaire d'une liaison covalente.

14. Conjugué d'un composé de formule générale (I) - (IV) selon l'une quelconque des revendications 1 à 10 et d'un partenaire de liaison.

15. Conjugué selon la revendication 14, dans lequel le partenaire de liaison est choisi parmi les lipides, les peptides, les polypeptides, les acides nucléiques, les nucléosides, les nucléotides, les analogues d'acide nucléique et les haptènes.
